(19) Europäisches Patentamt / European Patent Office / Office européen des brevets

(11) EP 4 008 406 A1

(12) EUROPEAN PATENT APPLICATION

(43) Date of publication:
08.06.2022 Bulletin 2022/23

(21) Application number: 20212308.9

(22) Date of filing: 07.12.2020

(51) International Patent Classification (IPC):
*A61Q 5/12* (2006.01) *A61Q 19/08* (2006.01)
*A61Q 19/00* (2006.01) *A61K 8/81* (2006.01)
*A61K 8/37* (2006.01) *A61K 8/41* (2006.01)
*A61K 8/45* (2006.01) *A61K 8/92* (2006.01)
*A61K 8/31* (2006.01) *A61K 8/34* (2006.01)

(52) Cooperative Patent Classification (CPC):
A61Q 5/12; A61K 8/31; A61K 8/345; A61K 8/37; A61K 8/416; A61K 8/45; A61K 8/8158; A61K 8/922; A61K 8/925; A61Q 19/00; A61Q 19/08

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(71) Applicant: Clariant International Ltd
4132 Muttenz (CH)

(72) Inventors:
- AGACKANLI, Sanem.
34752 Istanbul (TR)
- PEDULLA, Cassio.
4132 Muttenz (CH)
- RODRIGUES, Luciana.
04795-900 São Paulo (BR)

(74) Representative: Kampen, Daniela
Clariant Produkte (Deutschland) GmbH
Patent & License Management Chemicals
Industriepark Höchst / G 860
65926 Frankfurt am Main (DE)

# (54) COSMETIC COMPOSITION BASED ON ACROYL TAURATE TYPE POLYMERS

(57) The present invention relates to a cosmetic composition comprising (A) at least one polymer comprising sulfonic groups, (B) at least one emollient, (C) at least one ingredient selected from the group consisting of at least one conditioning agent, at least one solubilizing agent, at least one emulsifier, and a combination of two or more thereof, and (D) water. Furthermore, the present invention relates to the use of such cosmetic composition for hair and/or skin care.

EP 4 008 406 A1

## Description

The present invention relates to a cosmetic composition comprising (A) at least one polymer comprising sulfonic groups, (B) at least one emollient, (C) at least one ingredient selected from the group consisting of at least one conditioning agent, at least one solubilizing agent, at least one emulsifier, and a combination of two or more thereof, and (D) water. Furthermore, the present invention relates to the use of such cosmetic composition for hair and/or skin care.

Cosmetic products, such as hair and/or skin care products, play an important role in hygiene, beauty and well-being. For example, skin care products may be used for moisturizing and nourishing skin, protecting skin against oxidative and other environmental stress, promoting skin elasticity, as anti-wrinkle agent, etc. Hair care products may, for example, be used as hair conditioner, to achieve good combability, detangling, frizz control, to straighten hair, moisturize and nourish hair, protect hair against oxidative and other environmental stress, etc.

There is a general desire for cosmetic products, such as hair and/or skin care products. In particular, there is a desire for cosmetic products which meet the above needs of customers. For example, there is a desire for hair conditioners which effectively facilitate detangling and combing. Furthermore, there is a desire for cosmetic products which are convenient and easy to use, for example cosmetic products which can be applied to both hair and skin.

Surprisingly, it was found that cosmetic compositions comprising (A) a polymer, (B) an emollient, (C) at least one ingredient selected from a conditioning agent, a solubilizing agent and/or an emulsifier, and (D) water are particularly useful for hair and/or skin care. Advantageously, the compositions can be applied to both hair and skin. For example, the compositions can be used to moisturize and nourish hair as well as skin. Furthermore, the compositions are useful for conditioning hair, in particular they effectively facilitate combing and detangling. Advantageously, the compositions are storage stable.

A first aspect relates to a cosmetic composition comprising:

(A) at least one polymer which comprises at least 5 mol-% (referring to the polymer) of repeating units (a) according to Formula (1):

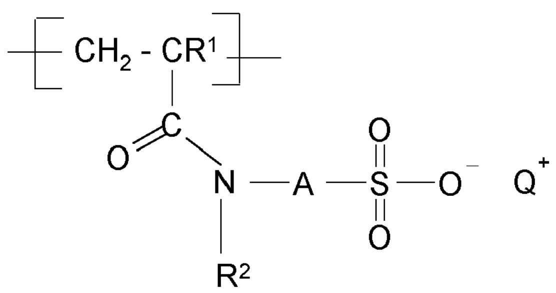

(1)

wherein:

$R^1$ and $R^2$ are independently selected from H, methyl or ethyl; A is a linear or branched $C_1$-$C_{12}$-alkylene group; and $Q^+$ is a cosmetically acceptable cation as component A;

(B) at least one emollient as component B;

(C) at least one ingredient selected from the group consisting of at least one conditioning agent, at least one solubilizing agent, at least one emulsifier, and a combination of two or more thereof as component C; and

(D) water.

Further embodiments of the invention are laid out in the following.

General definitions

In this application, including in all embodiments of all aspects of the present invention, the following definitions apply unless specifically stated otherwise. Unless otherwise stated, all percentages are by weight (w/w) of the respective component or the total composition, respectively. "wt.-%" means percentage by weight; "vol.-%" means percentage by volume; "mol-%" means percentage by mole. Unless otherwise stated, all ratios are weight ratios (weight per weight). Preferably, references to ’parts’ e.g. a mixture of 1 part X and 3 parts Y, is a ratio by weight. "QS" or "QSP" means sufficient quantity for 100 % or for 100 g. +/- indicates the standard deviation. All ranges are inclusive and combinable. Unless otherwise stated, all measurements are understood to be made at 23 °C and at ambient conditions, where "ambient conditions" means at approximately 1 atmosphere (atm) of pressure and at about 50 % relative humidity. Herein "mol" means mole. Herein "g" following a number means "gram" or "grams". Herein, "q.s." and "Q.S." means *quantum*

*sates,* thus, as much as needed. In the context of contents, this refers to the mass to obtain 100 wt.-% as total. For instance, the water is filled up to obtain a sum of 100 wt.-% of all ingredients. In the context of the pH, "q.s." means that the amount of acids and bases needed to obtain the desired pH is used.

The following acronyms are used herein: ACDMT = acryloyldimethyltaurate; AM = acrylamide; AN = acrylonitrile; tBAM = tert.-butyl acrylamide; IBSA = isobutene sulfonic acid; IBDSA = 2-methylidene-1,3-propylenedisulfonic acid.

Unless otherwise stated, "viscosity" herein is measured at 20-25 °C viscosity in centipoise (cP) or mPa·s using a Brookfield viscometer model LV, RVT DV-II or LVT DV-II with 10 - 90 % torque at 20 rpm.

"Molecular weight" or "M.Wt." "Mw", "$M_w$" or "MW" and grammatical equivalents mean the weight average molecular weight, unless otherwise stated. Also relevant for the determination of the molecular weight distribution is the number average molecular weight "Mn", "$M_n$" and grammatical equivalents, and the polydispersity "D" or "PD".

The weight average molecular weight can be measured by gel permeation chromatography (GPC), also referred to as size exclusion chromatography (SEC). The molecular weight of polymers and its measurement is described in the textbook "Principles of Polymerization" by Georg Odian, third edition, Wiley-Interscience, New York, in chapter 1-4, page 19 to 24, ISBN 0-471-61020-8. The process to determine the weight average molecular weight is described in detail in chapter 3 of Makromolekulare Chemie: Eine Einführung" by Bernd Tieke, Wiley-VCH, 2. vollständig uberarbeitete und erweiterte Auflage (3. Nachdruck 2010) ISBN-13: 978-3-527-31379-2, page 259-261.

Determination of molecular weight and distribution of ACDMT samples by GPC can be determined under the following conditions:

Column: PSS Suprema 30,000 Å 10 μm, 300 mm x 8 mm
Detector: RID
Oven temperature: 23 °C
Flow: 1 ml/min
Injection volume: 20 μl
Eluent: 0.07 mol/l disodium hydrogen phosphate in water
Calibration method: Conventional poly(styrene sulfonate) sodium salt calibration
Sample preparation: Weigh approx. 10 mg sample in 10 ml 0.07 mol/l disodium hydrogen phosphate in water and shake for 15 min.

"Water-soluble" refers to any material that is sufficiently soluble in water to form a clear solution to the naked eye at a concentration of 0.1 wt.-% of the material in water at 25 °C. The term "water-insoluble" refers to any material that is not "water-soluble".

"Substantially free from" or "substantially free of" means less than 1 wt.-%, or less than 0.8 wt.-%, or less than 0.5 wt.-%, or less than 0.3 wt.-%, or about 0 wt.-%, by total weight of the composition.

"Monomer" means a discrete, non-polymerized chemical moiety capable of undergoing polymerisation in the presence of an initiator or any suitable reaction that creates a macromolecule e.g. such as polycondensation, polyaddition, radical, anionic or cationic polymerization. "Unit" and "repeating unit" means a monomer-derived moiety that has already been polymerized and/or forms part of a polymer, i.e. is part of a polymer.

"Polymer" means a chemical entity formed from the polymerization of two or more, preferably at least three, more preferably at least five, even more preferably at least ten, in particular more than 20 monomers, thus, including of two or more, preferably at least three, more preferably at least five, even more preferably at least ten, in particular more than 20 repeating units. The term "polymer" shall include all materials made by the polymerization of monomers as well as natural polymers. Polymers made from only one type of monomer (i.e. containing only one type of repeating unit) may be called homopolymers. Polymers made from two or more different types of monomers (i.e. containing two or more different types of repeating units) may be called copolymers. The distribution of the different repeating units can be random (random copolymer) or alternating or block-wise (block copolymer). The term "polymer" used herein includes any type of polymer including homopolymers and copolymers as defined by the composition of repeating units comprised therein.

"Fuming sulfuric acid" herein means a solution of sulfur trioxide in sulfuric acid. Fuming sulfuric acid is also known as oleum and is identified by the CAS number 8014-95-7, and can be described by the Formula $H_2SO_4.xSO_3$ where x is the molar free sulfur trioxide content.

The "biobased content" is reported in ASTM D6866-12, Method B (see section 3.3.9 of ASTM D6866-12). The terms "biobased carbon content", "biobased content", "biogenic carbon content", "bio-based content", "biomass-derived carbon" may be understood interchangeably and each refer to wt.-%. Herein, the term "bio-based carbon content" is used. ASTM D6866-12, Method B lab results report the percentage of bio-based carbon content relative to total carbon, and not to total mass of the sample or molecular weight. A comment on bio-based carbon content calculation: Presently ASTM D6866-12, Method B (see section 9 of ASTM D6866-12) requires the percent modern carbon value (pMC) reported to be multiplied by a correction factor of 0.95 to account for excess carbon-14 in the atmosphere due to nuclear weapons

testing. However, a revision is pending for ASTM D6866-12, Method B to update the correction factor to 0.98 due to ongoing decrease in excess atmospheric $^{14}CO_2$. For the purposes of accuracy, the new correction factor of 0.98 is often reported in the field e.g. by suppliers. Generally, results below ~20 % bio-based carbon will not be affected. However, results close to 100% will be ~2-3 % bio-based carbon higher using the 0.98 factor vs 0.95. Results between ~20-90 % will increase by 0-3 %. Hence the term "bio-based carbon content" as used herein is defined by the equation:

$$\text{Bio-based carbon content} = \text{pMC} * 0.95\ (\%)$$

A review on measurement methods of bio-based carbon content for biomass-based chemicals and plastics is given by Massao Kunioka in Radioisotopes, 62, 901-925 (2013).

The analytical procedure for determination of bio-based carbon content may also comprise: the provided sample material may optionally not undergo any pretreatment procedure and is converted to graphite as is using the following procedure: Depending on the estimated amount of carbon content, typically a few milligrams of sample material is combusted in an elemental analyzer (EA). The resulting gas mixture is cleaned and $CO_2$ is automatically separated by the EA using the purge and trap technology. The remaining $CO_2$ is transferred into a custom-made graphitization system, converted into carbon (graphite) catalytically using $H_2$ and an iron-powder catalyst. The $^{14}C$ determination of the graphite is performed at the Klaus-Tschira-Archaeometrie-Center using an accelerator mass-spectrometer (AMS) of the type MICADAS (developed at the ETH Zurich, Switzerland).

"Hair" may be understood in the broadest sense as mammalian keratin fibres including scalp hair, facial hair and body hair. It includes such hair still being attached to a living subject and also hair that has been removed therefrom such as hair swatches and hair on a doll/mannequin. In at least one preferred embodiment, "hair" means human hair.

"Cosmetically acceptable" may be understood in the broadest sense in that the compositions or components described are suitable for use in contact with human keratinous tissue without undue toxicity, incompatibility, instability, allergic response, and the like. All compositions described herein which have the purpose of being directly applied to keratinous tissue are cosmetically acceptable.

For brevity and eligibility, cosmetic composition may also be referred to simply as "composition" in some text passages herein.

Component A

The cosmetic composition of the present invention comprises at least one polymer which comprises at least 5 mol-% of repeating units (a) according to Formula (1):

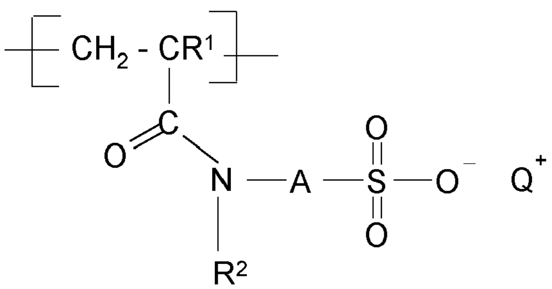

(1)

wherein:

$R^1$ and $R^2$ are independently selected from H, methyl or ethyl; A is a linear or branched $C_1$-$C_{12}$-alkylene group; and $Q^+$ is a cosmetically acceptable cation.

It will be understood that the at least one polymer is typically a polymer that is cosmetically acceptable.

The polymer may be crosslinked or non-crosslinked. In at least one preferred embodiment, the polymer is a crosslinked or non-crosslinked homopolymer. In at least one preferred embodiment, the polymer is a crosslinked or non-crosslinked copolymer.

In at least one preferred embodiment, the polymer has a weight average molecular weight of at least 700 g/mol, preferably from 700 g/mol to 10 million g/mol.

In at least one embodiment, the polymer comprises or consists of:

(a) from 5 to 99.99 mol-% repeating units (a) according to Formula (1);

(b) from 0.01 to 10 mol-% crosslinking or branching units (b), wherein the crosslinking or branching units (b) result

from the incorporation of a monomer comprising at least two olefinically unsaturated double bonds;
(c) optionally from 0 to 89 mol-% of neutral structural units (c);
(d) optionally from 0 to 20 mol-% of anionic structural units (d), wherein the anionic structural units (d) result from the incorporation of a monomer comprising at least one carboxylate anion.

Repeating units (a)

In at least one preferred embodiment, the polymer comprises at least 9 mol-% of repeating units (a) according to Formula (1). In at least one preferred embodiment, the polymer comprises at least 9.49 mol-% of repeating units (a) according to Formula (1). In at least one preferred embodiment, the polymer comprises from 5 to 100 mol-%, or from 90 to 99.9 mol-%, or from 95 to 99.5 mol-%, or from 27.5 to 97.4 mol-%, or from 40 to 99 mol-%, or from 55 to 98 mol-%, or from 9 to 99.9 mol-%, or from 9.49 to 99.8 mol-%, or from 9.49 to 98 mol-%, or from 10 to 99.7 mol-%, or from 20 to 99.5 mol-%, or from 30 to 99 mol-%, or from 40 to 98 mol-%, or from 40 to 95 mol-%, or from 50 to 90 mol-%, or from 60 to 80 mol-%, or from 45 to 75 mol-%, of repeating units (a) according to Formula (1). In at least one preferred embodiment, the polymer consists of repeating units (a) according to Formula (1).

Optionally, repeating units (a) according to Formula (1) may partly or completely comprise a bio-based carbon content. In at least one embodiment, at least 10 wt.-%, preferably at least 20 wt.-% of the repeating units (a) according to Formula (1) comprise from 28 wt.-% to 100 wt.-% bio-based carbon content, relative to the total mass of carbon in the repeating unit (a) according to Formula (1), measured according to standard ASTM D6866-12, Method B.

In at least one embodiment, at least 30 wt.-%, or at least 40 wt.-%, or at least 50 wt.-%, or at least 60 wt.-%, or at least 70 wt.-%, or at least 80 wt.-%, or at least 90 wt.-%, or at least 95 wt.-%, or at least 98 wt.-%, or at least 99 wt.-% of the repeating units (a) according to Formula (1) comprise from 28 wt.-% to 100 wt.-% bio-based carbon content, relative to the total mass of carbon in the repeating unit (a) according to Formula (1), measured according to standard ASTM D6866-12, Method B. In at least one embodiment, the repeating unit (a) according to Formula (1) comprises at least 35 wt.-%, preferably at least 40 wt.-%, more preferably at least 54 wt.-%, even more preferably at least 57 wt.-%, most preferably about 100 wt.-% bio-based carbon content, relative to the total mass of carbon in the repeating unit (a) according to Formula (1), measured according to standard ASTM D6866-12, Method B.

As noted above, $Q^+$ may be any cosmetically acceptable cation. In at least one preferred embodiment, $Q^+$ is $H^+$, $NH_4^+$, an organic ammonium ion $[NHR^5R^6R^7]^+$ wherein $R^5$, $R^6$, and $R^7$ independently of one another may be hydrogen, a linear or branched alkyl group having 1 to 22 carbon atoms, a linear or branched, singularly or multiply unsaturated alkenyl group having 2 to 22 carbon atoms, a $C_6$-$C_{22}$ alkylamidopropyl group, a linear mono-hydroxyalkyl group having 2 to 12 carbon atoms or a linear or branched dihydroxyalkyl group having 3 to 12 carbon atoms, and where at least one of the radicals $R^5$, $R^6$, and $R^7$ is not hydrogen, or $Q^+$ is $Li^+$, $Na^+$, $K^+$, ½ $Ca^{++}$, ½ $Mg^{++}$, ½ $Zn^{++}$, 1/3 $Al^{+++}$, or combinations thereof.

In at least one preferred embodiment, residue A in Formula (1) is selected from the group consisting of methylene, ethylene, n-propylene, $-CH(CH_3)-CH_2-$, $-C(CH_3)_2-CH_2-$, $-C(CH_3)_2-(CH_2)_n-$, and $-CHCH_3-(CH_2)_n-$, wherein n is 1, 2, 3 or an integer between 4 and 12.

In at least one preferred embodiment, the polymer comprises repeating units (a) according to Formula (1) wherein $R^1$ and $R^2$ are independently selected from H, methyl or ethyl; A is a linear or branched $C_1$-$C_{12}$-alkylene group; and $Q^+$ is $H^+$, $NH_4^+$, $Li^+$, $Na^+$, $K^+$, ½ $Ca^{++}$, ½ $Mg^{++}$, ½ $Zn^{++}$, 1/3 $Al^{+++}$, or combinations thereof, preferably wherein $Q^+$ is $Na^+$ or $NH_4^+$. $NH_4^+$ is preferred because it is more soluble in the favored solvent used in the polymer synthesis. $Na^+$ is preferred because of reduced likelihood of unpreferred gases being produced during synthesis and also due to economic advantages.

In at least one preferred embodiment, $Q^+$ is $NH_4^+$. In at least one preferred embodiment, $Q^+$ is $Na^+$. In at least one preferred embodiment, $Q^+$ is selected from the group monoalkylammonium, dialkylammonium, trialkylammonium and/or tetraalkylammonium salts, in which the alkyl substituents of the amines may independently of one another be ($C_1$ to $C_{22}$)-alkyl radicals or ($C_2$ to $C_{10}$)-hydroxyalkyl radicals.

In at least one preferred embodiment, the polymer comprises at least one repeating unit (a) according to Formula (1), for example one repeating unit (a) according to Formula (1). In at least one preferred embodiment, the polymer comprises two or more different repeating units (a) according to Formula (1), for example repeating units according to Formula (1) having different $Q^+$ counterions.

In at least one preferred embodiment, the repeating units (a) according to Formula (1) have a degree of neutralization of between 0 mol-% and 100 mol-%. In at least one preferred embodiment, the repeating units (a) according to Formula (1) have a degree of neutralization of from 50 to 100 mol-%, preferably from 80 to 100 mol-%, more preferably from 90 to 100 mol-%, even more preferably from 95 to 100 mol-%. Particular preference is given to a degree of neutralization of more than 80 mol-%, more preferably more than 90 mol-%, even more preferably more than 95 mol-%. The degree of neutralization is important in view of the molecular weight of the polymer and the yield of polymer produced.

In at least one preferred embodiment, the repeating units (a) according to Formula (1) result from the incorporation of a monomer selected from the group consisting of acryloyldimethyltaurates, acryloyl-1,1-dimethyl-2-methyltaurates (ACDMT), acryloyltaurates, acryloyl-N-methyltaurates, and combinations thereof. Preferably the repeating units (a) according to Formula (1) result from the incorporation of acryloyldimethyltaurate.

Preferably, the repeating units according to Formula (1) are formed by polymerization of a compound according Formula (3)

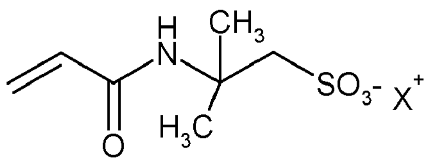

(3)

wherein X preferably is a proton.

Preferably, the compound according to Formula (3) is ACDMT.

The polymer may be crosslinked or non-crosslinked. The polymer may be branched or unbranched. In at least one preferred embodiment, the polymer is crosslinked and/or branched. In at least one preferred embodiment, the polymer comprises, in addition to repeating units (a) according to Formula (1), crosslinking or branching units (b), wherein the crosslinking or branching units (b) result from the incorporation of a monomer comprising at least two olefinically unsaturated double bonds. In at least one preferred embodiment, the polymer comprises from 0.01 to 10 mol-%, or from 0.01 to 5 mol-%, or from 0.01 to 4 mol-%, or from 0.01 to 3 mol-%, of crosslinking or branching units (b), wherein the crosslinking or branching units (b) result from the incorporation of a monomer comprising at least two olefinically unsaturated double bonds.

In at least one preferred embodiment, the polymer comprises (or consists of):

(a) from 90 to 99.9 mol-%, preferably from 95 to 99.5 mol-% of repeating units (a) according to Formula (1); and
(b) from 0.01 to 10 mol-%, preferably from 0.01 to 5 mol-% of crosslinking or branching units (b).

In at least one preferred embodiment, the polymer comprises from 96 to 99.7 mol-%, preferably from 97 to 99.5 mol-% units (a) and from 0.3 to 4 mol-%, preferably from 0.5 to 3 mol-% units (b). In at least one preferred embodiment, the polymer comprises units (a) and (b), such that the sum thereof is at least 99 mol-%, by total weight of the polymer.

In at least one preferred embodiment, the polymer comprises or consists of repeating units (a) resulting from the incorporation of a monomer selected from the group consisting of acryloyldimethyltaurates, acryloyl-1,1-dimethyl-2-methyltaurates (ACDMT), acryloyltaurates, acryloyl-N-methyltaurates, and combinations thereof, in particular wherein the polymer comprises or consists of repeating units (a) resulting from the incorporation of acryloyldimethyltaurate.

Crosslinking or branching units (b)

In at least one preferred embodiment, the polymer comprises crosslinking or branching units (b), wherein the crosslinking or branching units result from the incorporation of a monomer comprising at least two olefinically unsaturated double bonds. In at least one preferred embodiment, the polymer comprises from 0.01 to 5 mol-%, preferably from 0.01 to 4 mol-%, more preferably from 0.01 to 3 mol-%, even more preferably from 0.01 to 2 mol-% of crosslinking or branching units.

In at least one preferred embodiment, the crosslinking or branching units comprise least one oxygen, nitrogen, sulfur or phosphorus atom. In at least one preferred embodiment, the crosslinking or branching units result from monomers having a molecular weight of less than 500 g/mol. In at least one preferred embodiment, the units (b) result from bifunctional or trifunctional crosslinking agents.

In at least one preferred embodiment, the polymer comprises two or more different crosslinking or branching units.

In at least one preferred embodiment, the crosslinking or branching units result from the incorporation of a monomer according to Formula (2):

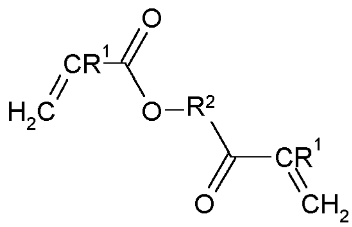

(2)

wherein

$R^1$ is selected from H, methyl or ethyl; and

$R^2$ is a linear or branched alkyl group having 1 to 6 carbon atoms, or is a linear or branched, mono- or polyunsaturated alkylene group having 2 to 6 carbon atoms, or a group $-(CH_2-CH_2-O)_n-$;

n is an integer from 1 to 100.

Monomers according to Formula (2) have the advantage that polymers based thereon can be more brush-like. However, brush-like polymers show different properties, versus linear ones. For example, depending on the different comonomer units the solubility can be in- or decreased.

In at least one preferred embodiment, the crosslinking or branching units are according to Formula (4)

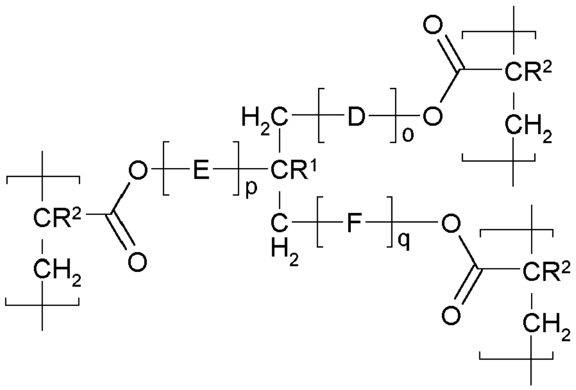

(4)

wherein

$R^1$ is selected from H, methyl or ethyl; and

$R^2$ is a linear or branched alkyl group having 1 to 6 carbon atoms, hydrogen, or a linear or branched, mono- or polyunsaturated alkylene group having 2 to 6 carbon atoms;

D, E, and F are independently methyleneoxy ($-CH_2O-$), ethyleneoxy ($-CH_2-CH_2-O-$), propyleneoxy ($-CH(CH_3)-CH_2-O-$), a linear or branched alkylene group having 1 to 6 carbon atoms, a linear or branched, singularly or multiply unsaturated alkenylene group having 2 to 6 carbon atoms, a linear mono-hydroxyalkylene group having 2 to 6 carbon atoms or a linear or branched dihydroxyalkylene group having 3 to 6 carbon atoms; and

o, p, and q each independently are an integer from 1 to 50.

Crosslinking or branching units according to Formula (4) have the advantage that polymers comprising those can be highly branched.

In at least one preferred embodiment, the crosslinking or branching units result from the incorporation of a monomer selected from the group consisting of methylenebisacrylamide; methylenebismethacrylamide; esters of unsaturated monocarboxylic and polycarboxylic acids with polyols, preferably di-acrylates and tri-acrylates and -methacrylates (e.g. glycerol propoxylate triacrylate [GPTA]), more preferably butanediol and ethylene glycol diacrylate and polyethylene glycol diacrylate and -methacrylate, trimethylolpropane triacrylate (TMPTA) and trimethylolpropane trimethacrylate (TMPTMA); allyl compounds, preferably allyl (meth)acrylate, triallyl cyanurate, diallyl maleate, polyallyl esters, tetraallyloxyethane, triallylamine, tetraallylethylenediamine; allyl esters of phosphoric acid; and/or vinylphosphonic acid derivatives. The choice of crosslinking or branching units is important in view of the flexibility of the crosslinks between the main chains of the polymer, which affects the final performance of the polymer.

In at least one preferred embodiment, the crosslinking or branching units result from the incorporation of a

crosslinker selected from the group consisting of trimethylolpropane triacrylate (TMPTA) and/or glycerol propoxylate triacrylate (GPTA). Particularly preferred crosslinkers are glycerol propoxylate triacrylate (GPTA), trimethylolpropane triacrylate (TMPTA), pentaerythritol diacrylate mono stearate (PEAS), hexanediol diacrylate (HDDA), polyethylene glycol diacrylate (PEG-DA) or hexanediol dimethacrylate (HDDMA).

In at least one preferred embodiment, the crosslinking or branching units (b) result from the incorporation of a crosslinker selected from the group consisting of trimethylolpropane triacrylate (TMPTA) and/or glycerol propoxylate triacrylate (GPTA).

In at least one preferred embodiment, the crosslinking or branching units (b) comprise at least 35 wt.-%, preferably at least 40 wt.-%, more preferably at least 54 wt.-%, even more preferably at least 57 wt.-%, most preferably about 100 wt.-% bio-based carbon content, relative to the total mass of carbon in the crosslinking or branching units (b), measured according to standard ASTM D6866-12, Method B.

Neutral structural units (c)

In at least one preferred embodiment, the polymer comprises at least one neutral structural unit (c). In at least one preferred embodiment, the polymer comprises one or more neutral structural units (c). In at least one preferred embodiment, the polymer comprises from 0.01 to 88.52 mol-%, or from 0.05 to 72.4 mol-%, or from 0.99 to 59.99 mol-%, or from 1.99 to 44.99 mol-%, of neutral structural units.

In at least one preferred embodiment, the polymer comprises at least one neutral structural unit (c) resulting from the incorporation of a monomer selected from the group consisting of acrylic or methacrylic acid amides, polyglycol acrylic or methacrylic acid esters, polyglycol acrylic or methacrylic acid amides, ethoxylated fatty alcohol acrylates, ethoxylated fatty alcohol methacrylates, propoxylated fatty alcohol acrylates, and open-chain or cyclic N-vinylamides or N-methylvinyl amides.

In at least one preferred embodiment, the polymer comprises at least one neutral structural unit (c) resulting from the incorporation of a monomer selected from the group consisting of open-chain N-vinyl amides, preferably N-vinylformamide, N-vinylmethylformamide, N-vinylmethylacetamide or N-vinylacetamide; cyclic N-vinyl amides (N-vinyl lactams) with a ring size of 3 to 9, preferably N-vinylpyrrolidone (NVP) or N-vinylcaprolactam; amides of acrylic and methacrylic acid; alkoxylated acrylamides and alkoxylated methacrylamides.

In at least one preferred embodiment, the polymer comprises at least one neutral structural unit (c) resulting from the incorporation of a monomer selected from the group consisting of N-vinylformamide, N-vinylacetamide, N-methyl-N-vinylformamide, N-methyl-N-vinylacetamide, N-vinyl-2-pyrrolidone, N-vinylcaprolactam, vinylacetate, N,N-dimethylacrylamide, N-isopropylacrylamide, acrylamide, methylacrylate, behenylpolyethoxy-(25)-methacrylate, laurylpolyethoxy-(7)-methacrylate, cetylpolyethoxy-(10)-methacrylate, stearylpolyethoxy-(8)-methacrylate, methoxypolyethoxy-(12)-methacrylate, and combinations thereof.

In at least one preferred embodiment, the polymer comprises at least one neutral structural unit (c) resulting from the incorporation of a monomer selected from the group consisting of hydroxyethyl methacrylate, hydroxymethyl methacrylamide, hydroxyethyl methacrylamide, hydroxypropyl methacrylamide, N,N-dimethylaminomethyl methacrylate and N,N-diethylaminomethyl methacrylate.

In at least one preferred embodiment, the polymer comprises at least one neutral structural unit (c) resulting from the incorporation of a monomer selected from the group consisting of methylvinylether, ethylvinylether, methylallylether, ethylmethallylether, methylmethallylether, ethylallylether, tert-butylacrylamide, N,N-diethylacrylamide, N,N-dimethylacrylamide, N,N-dimethylmethacrylamide, N,N-dipropylacrylamide, N-isopropylacrylamide, N-propylacrylamide, acrylamide, methacrylamide, methylacrylate, methylmethacrylate, tert-butylacrylate, tert-butylmethacrylate, n-butylacrylate, n-butylmethacrylate, laurylacrylate, laurylmethacrylate, behenylacrylate, behenylmethacrylate, cetylacrylate, cetylmethacrylate, stearylacrylate, stearylmethacrylate, tridecylacrylate, and tridecylmethacrylate.

In at least one preferred embodiment, the polymer comprises at least one neutral structural unit (c) resulting from the incorporation of a monomer selected from the group consisting of polyethoxy-(5)-methacrylate, polyethoxy-(5)-acrylate, polyethoxy-(10)-methacrylate, polyethoxy-(10)-acrylate, behenylpolyethoxy-(7)-methacrylate, behenylpolyethoxy-(7)-acrylate, behenylpolyethoxy-(8)-methacrylate, behenylpolyethoxy-(8)-acrylate, behenylpolyethoxy-(12)-methacrylate, behenylpolyethoxy-(12)-acrylate, behenylpolyethoxy-(16)-methacrylate, behenylpolyethoxy-(16)-acrylate, behenylpolyethoxy-(25)-methacrylate, behenylpolyethoxy-(25)-acrylate, laurylpolyethoxy-(7)-methacrylate, laurylpolyethoxy-(7)-acrylate, laurylpolyethoxy-(8)-methacrylate, laurylpolyethoxy-(8)-acrylate, laurylpolyethoxy-(12)-methacrylate, laurylpolyethoxy-(12)-acrylate, laurylpolyethoxy-(16)-methacrylate, laurylpolyethoxy-(16)-acrylate, laurylpolyethoxy-(22)-methacrylate, laurylpolyethoxy-(22)-acrylate, laurylpolyethoxy-(23)-methacrylate, laurylpolyethoxy-(23)-acrylate, cetylpolyethoxy-(2)-methacrylate, cetylpolyethoxy-(2)-acrylate, cetylpolyethoxy-(7)-methacrylate, cetylpolyethoxy-(7)-acrylate, cetylpolyethoxy-(10)-methacrylate, cetylpolyethoxy-(10)-acrylate, cetylpolyethoxy-(12)-methacrylate, cetylpolyethoxy-(12)-acrylate cetylpolyethoxy-(16)-methacrylate, cetylpolyethoxy-(16)-acrylate cetylpolyethoxy-(20)-methacrylate, cetylpolyethoxy-(20)-acrylate, cetylpolyethoxy-(25)-methacrylate, cetylpolyethoxy-(25)-acrylate, cetylpolyethoxy-(25)-methacrylate, cetylpolyethoxy-(25)-acrylate, stearylpolyethoxy-(7)-methacrylate, stearylpolyethoxy-(7)-acrylate, stearylpolyethoxy-(8)-methacrylate, stearylpolyethoxy-(8)-acrylate, stearylpolyethoxy-(12)-methacrylate, stearylpolyethoxy-(12)-acrylate, stearylpolyethoxy-(16)-methacrylate, stearylpolyethoxy-(16)-acrylate, stearylpolyethoxy-(22)-methacrylate, stearylpolyethoxy-(22)-acrylate, stearylpolyethoxy-(23)-methacrylate, stearylpolyethoxy-(23)-acrylate, stearylpolyethoxy-(25)-methacrylate, stearylpolyethoxy-(25)-acrylate, tridecylpolyethoxy-(7)-methacrylate, tridecylpolyethoxy-(7)-acrylate, tridecylpolythoxy-(10)-methacrylate, tridecylpolyethoxy-(10)-acrylate, tridecylpolyethoxy-(12)-methacrylate, tridecylpolyethoxy-(12)-acrylate, tridecylpolyethoxy-(16)-methacrylate, tridecylpolyethoxy-(16)-acrylate, tridecylpolyethoxy-(22)-methacrylate, tridecylpolyethoxy-(22)-acrylate, tridecylpolyethoxy-(23)-methacrylate, tridecylpolyethoxy-(23)-acrylate, tridecylpolyethoxy-(25)-methacrylate, tridecylpolyethoxy-(25)-acrylate, methoxypolyethoxy-(7)-methacrylate, methoxypolyethoxy-(7)-acrylate, methoxypolyethoxy-(12)-methacrylate, methoxypolyethoxy-(12)-acrylate, methoxypolyethoxy-(16)-methacrylate, methoxypolyethoxy-(16)-acrylate, methoxypolyethoxy-(25)-methacrylate, and methoxypolyethoxy-(25)-acrylate.

The neutral structural units (c) may, for example, also be 2-vinylpyridine, 4-vinylpyridine, glycidyl methacrylate, styrene, acetoxystyrene, acrylonitrile, vinyl chloride, vinylidene chloride, or tetrafluoroethylene.

In at least one preferred embodiment, the neutral structural units (c) comprise at least 35 wt.-%, preferably at least 40 wt.-%, more preferably at least 54 wt.-%, even more preferably at least 57 wt.-%, most preferably about 100 wt.-% bio-based carbon content, relative to the total mass of carbon in the neutral structural units (c), measured according to standard ASTM D6866-12, Method B.

Anionic structural units (d)

In at least one preferred embodiment, the polymer comprises at least one anionic structural unit (d) which is different from repeating unit (a). In at least one preferred embodiment, the polymer comprises one or more anionic structural units (d) which are different from repeating units (a). In at least one preferred embodiment, the polymer further comprises one or more anionic structural units (d), wherein the anionic structural units (d) result from the incorporation of a monomer comprising at least one carboxylate anion. In at least one preferred embodiment, the polymer comprises from 1.98 to 20 mol-%, preferably from 2.5 to 18 mol-% of anionic structural units (d) which are different from repeating units (a).

In at least one preferred embodiment, the anionic structural units (d) result from the incorporation of a monomer according to Formula (A):

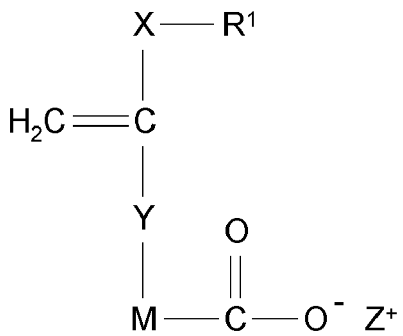

(A)

wherein

$R^1$ and $R^3$ are H, methyl or ethyl, or $C(O)O^-Z^+$;

X, Y are selected from a covalent bond, O, $CH_2$, C(O)O, OC(O), $C(O)NR^3$ or $NR^3C(O)$;

M is selected from a covalent bond, $-[C(O)O-CH_2-CH_2]_n-$, a linear or branched alkylene group with 1 to 6 carbon atoms, a linear or branched, mono- or polyunsaturated alkenylene group with 2 to 6 carbon atoms, a linear mono-hydroxyalkylene group with 2 to 6 carbon atoms or a linear or branched dihydroxyalkylene group with 3 to 6 carbon atoms;

n is an integer from 1 to 5; and

$Z^+$ is $H^+$, $NH_4^+$, an organic ammonium ion $[HNR^5R^6R^7]^+$

wherein $R^5$, $R^6$ and $R^7$ are independently hydrogen, a linear or branched alkyl group with 1 to 22 carbon atoms, a linear or branched, mono- or polyunsaturated alkenyl group with 2 to 22 carbon atoms, a $C_6$ to $C_{22}$ alkylamidopropyl group, a linear mono-hydroxyalkyl group with 2 to 10 carbon atoms or a linear or branched dihydroxyalkyl group with 3 to 10

carbon atoms, and wherein at least one of $R^5$, $R^6$ and $R^7$ is not hydrogen, or $Z^+$ is $Li^+$, $Na^+$, $K^+$, ½ $Ca^{++}$, ½ $Mg^{++}$, ½ $Zn^{++}$, 1/3 $Al^{+++}$, or combinations thereof. In at least one preferred embodiment, $Z^+$ is $H^+$, $NH_4^+$, $Li^+$, $Na^+$, $K^+$, ½ $Ca^{++}$, ½ $Mg^{++}$, ½ $Zn^{++}$, or 1/3 $Al^{+++}$, more preferably $H^+$, $NH_4^+$, $Li^+$, $Na^+$ or $K^+$.

In at least one preferred embodiment, the anionic structural units (d) result from the incorporation of a monomer according to Formula (A) wherein X is a covalent bond or $CH_2$. In at least one preferred embodiment, the anionic structural units (d) result from the incorporation of a monomer according to Formula (A) wherein Y is a covalent bond, $CH_2$, C(O)O, or C(O)$NR^3$. In at least one preferred embodiment, the anionic structural units (d) result from the incorporation of a monomer according to Formula (A) wherein M is a covalent bond, -[C(O)O-$CH_2$-$CH_2$]n-, or a linear or branched alkylene group with 1 to 6 carbon atoms. In at least one preferred embodiment, the anionic structural units (d) result results from the incorporation of a monomer according to Formula (A) wherein $R^1$ is H, methyl or ethyl; X is a covalent bond or $CH_2$; Y is a covalent bond, $CH_2$, C(O)O, or C(O)$NR^3$; $R^3$ is H, methyl or ethyl; M is a covalent bond, -[C(O)O-$CH_2$-$CH_2$]n-, or a linear or branched alkylene group with 1 to 6 carbon atoms; $Z^+$ is $H^+$, $NH_4^+$, $Li^+$, $Na^+$, $K^+$, ½ $Ca^{++}$, ½ $Mg^{++}$, ½ $Zn^{++}$, or 1/3 $Al^{+++}$, or combinations thereof.

In at least one preferred embodiment, the polymer comprises at least one anionic structural unit (d) resulting from the incorporation of a monomer selected from the group consisting of acrylic acid or acrylate, methacrylic acid or methacrylate, itaconic acid or itaconate, carboxyethylacrylic acid or carboxyethylacrylate, carboxyethylacrylic acid oligomers or carboxyethylacrylate oligomers, 2-propylacrylic acid or 2-propylacrylate, 2-ethylacrylic acid or 2-ethylacrylate, and their respective alkali or alkaline earth metal salts.

In at least one preferred embodiment, the polymer comprises at least one anionic structural unit (d) resulting from the incorporation of a monomer selected from the group consisting of acrylic acid or acrylate, methacrylic acid or methacrylate, itaconic acid or itaconate, carboxyethylacrylic acid or carboxyethylacrylate, carboxyethylacrylic acid oligomers or carboxyethylacrylate oligomers, and their respective alkali or alkaline earth metal salts. These anionic structural units are preferred because they can easily be synthesised from bio-based sources.

In at least one preferred embodiment, the polymer comprises at least one anionic structural unit (d) resulting from the incorporation of a monomer selected from the group consisting of acrylic acid, ammonium acrylate, sodium acrylate, potassium acrylate, lithium acrylate, zinc acrylate, calcium acrylate, magnesium acrylate, zirconium acrylate, methacrylic acid, ammonium methacrylate, sodium methacrylate, potassium methacrylate, lithium methacrylate, calcium methacrylate, magnesium methacrylate, zirconium methacrylate, zinc methacrylate, 2-carboxyethylacrylate, ammonium 2-carboxyethylacrylate, sodium 2-carboxyethylacrylate, potassium 2-carboxyethylacrylate, lithium 2-carboxyethylacrylate, zinc 2-carboxyethylacrylate, calcium 2-carboxyethylacrylate, magnesium 2-carboxyethylacrylate, zirconium 2-carboxyethylacrylate, 2-carboxyethylacrylate-oligomers, ammonium 2-carboxyethylacrylate-oligomers, sodium 2-carboxyethylacrylate-oligomers, potassium 2-carboxyethylacrylate-oligomers, lithium 2 carboxyethylacrylate-oligomers, zinc 2-carboxyethylacrylate-oligomers, calcium 2-carboxyethylacrylate-oligomers, magnesium 2-carboxyethylacrylate-oligomers, zirconium 2-carboxyethylacrylate-oligomers, itaconic acid, sodium itaconate, potassium itaconate, lithium itaconate, calcium itaconate, magnesium itaconate, zirconium itaconate, and zinc itaconate.

The anionic structural units (d) may, for example, result from the incorporation of 2-ethylacrylic acid, ammonium 2-ethylacrylate, sodium 2-ethylacrylate, potassium 2-ethylacrylate, lithium 2-ethylacrylate, calcium 2-ethylacrylate, magnesium 2-ethylacrylate, zirconium 2-ethylacrylate, zinc 2-ethylacrylate, 2-propylacryl acid, ammonium 2-propylacrylate, sodium 2-propylacrylate, potassium 2-propylacrylate, lithium 2-propylacrylate, calcium 2-propylacrylate, magnesium 2-propylacrylate, magnesium 2-propylacrylate, zirconium 2-propylacrylate, or zinc 2-propylacrylate.

The anionic structural units (d) may, for example, result from the incorporation of maleic acid, fumaric acid, crotonic acid, or senecic acid. The anionic structural units (d) may, for example, also be mono[2-(methacryloyloxy)ethyl]succinate or acrylamido- or methacrylamidoglycolic acid.

In at least one preferred embodiment, the anionic structural units (d) comprise at least 35 wt.-%, preferably at least 40 wt.-%, more preferably at least 54 wt.-%, even more preferably at least 57 wt.-%, most preferably about 100 wt.-% bio-based carbon content, relative to the total mass of carbon in the anionic structural units (d), measured according to standard ASTM D6866-12, Method B.

Optionally, the polymer comprises one or more further repeating units (e). For example, an optional further repeating unit (e) may result from the incorporation of styrenesulfonic acid.

In at least one preferred embodiment, the polymer comprises (or consists of):

(a) from 40 to 99 mol-% or from 40 to 98 mol-%, preferably from 55 to 98 mol-% of repeating units according to Formula (1);

(b) from 0.01 to 5 mol-%, preferably from 0.01 to 3 mol-% of crosslinking or branching units (b), wherein the crosslinking or branching units (b) result from the incorporation of a monomer comprising at least two olefinically unsaturated double bonds; and

(c) from 0.99 to 59.99 mol-%, preferably from 1.99 to 44.99 mol-% of neutral structural units.

In at least one preferred embodiment, the polymer comprises from 45 to 97 mol-%, preferably from 65 to 96 mol-% units (a), from 0.25 to 4 mol-%, preferably from 0.3 to 3 mol-% units (b), from 2 to 54,7 mol-%%, preferably from 2.5 to 34.5 mol-% units (c). In at least one preferred embodiment, the polymer comprises units (a), (b) and (c) such that the sum thereof is at least 99 mol-%, by total weight of the polymer.

In at least one preferred embodiment, the polymer comprises from 70 to 98 mol-%, preferably from 73 to 96 mol-% units (a), from 0.6 to 2.5 mol-%, preferably from 0.75 to 2 mol-% units (b), from 1.4 to 54,7 mol-%, preferably from 2.5 to 34.5 mol-% units (c). In at least one preferred embodiment, the polymer comprises units (a), (b) and (c) such that the sum thereof is at least 99 mol-%, by total weight of the polymer.

In at least one preferred embodiment, the polymer further comprises one or more neutral structural units and one or more anionic structural units (d) which are different from repeating units (a), preferably wherein the anionic structural units (d) result from the incorporation of a monomer comprising at least one carboxylate anion.

In at least one preferred embodiment, the polymer comprises:

from 0.01 to 88.52 mol-%, preferably from 0.05 to 72.4 mol-% of neutral structural units; and
from 1.98 to 20 mol-%, preferably from 2.5 to 18 mol-% of anionic structural units (d) different from repeating units (a).

In at least one preferred embodiment, the polymer comprises (or consists of):

(a) from 9.49 to 98 mol-%, preferably from 27.5 to 97.4 mol-% repeating units according to Formula (1);
(b) from 0.01 to 5 mol-%, preferably from 0.01 to 4 mol-% crosslinking or branching units (b), wherein the crosslinking or branching units (b) result from the incorporation of a monomer comprising at least two olefinically unsaturated double bonds;
(c) from 0.01 to 88.52 mol-%, preferably from 0.05 to 72.4 mol-% of neutral structural units; and
(d) from 1.98 to 20 mol-%, preferably from 2.5 to 18 mol-% of anionic structural units (d), wherein the anionic structural units (d) result from the incorporation of a monomer comprising at least one carboxylate anion.

In at least one preferred embodiment, the polymer comprises from 37 to 96.4 mol-%, preferably from 43 to 95.3 mol-% units (a), from 0.1 to 3 mol-%, preferably from 0.2 to 2 mol-% units (b), from 0.1 to 59.3 mol-%, preferably from 0.5 to 52.8 mol-% units (c), and from 3.5 to 16 mol-%, preferably from 4 to 14 mol-% units (d). In at least one preferred embodiment, the polymer comprises units (a), (b), (c) and (d) such that the sum thereof is at least 99 mol-%, by total weight of the polymer.

In at least one preferred embodiment, the polymer comprises from 70 to 94.5 mol-%, units (a), from 0.35 to 1.5 mol-%, units (b), from 0.65 to 25.65 mol-% units (c), and from 4.5 to 12 mol-% units (d). In at least one preferred embodiment, the polymer comprises units (a), (b), (c) and (d) such that the sum thereof is at least 99 mol-%, by total weight of the polymer.

In at least one preferred embodiment, the polymer is non-crosslinked. In this case, it may optionally consist of repeating units (A) of Formula (1).

Component B

The present invention also comprises at least one emollient. It will be understood that an emollient in the context of the present invention is typically a cosmetically acceptable emollient.

As used herein, the term "emollient" may be understood in the broadest sense as generally understood in the field of cosmetics. An emollient may be an ingredient which is suitable to soften skin and/or hair and/or to make skin and/or hair supple. An emollient may reduce skin roughness and make the skin appear softer and smoother. Optionally, an emollient may concomitantly act as occlusive and/or moisturizer.

In at least one preferred embodiment, the at least one emollient is selected from waxes, oils, and butters. In at least one preferred embodiment, the at least one emollient is of synthetic origin or natural, origin such as plant or animal origin. In at least one preferred embodiment, the at least one emollient is selected from oils (e.g., volatile or nonvolatile oils), butters (e.g., butters which are pasty, creamy or solid at room temperature) and waxes (e.g., waxes which are pasty, creamy or solid at room temperature), of synthetic origin or natural origin, such as plant or animal origin.

In at least one preferred embodiment, the at least one emollient is selected from the group consisting of:

(B-i) an emollient of synthetic origin selected from the group consisting of petrolatum (also: vaseline oil), other hydrocarbon oils (e.g., paraffin oil), esters of fatty acids with alcohols, esters of fatty alcohols with alkanoic acids, alkyl benzoates, silicone oils, other mineral oils, and combinations thereof;
(B-ii) an emollient of plant origin selected from the group consisting of plant oils, plant butters, plant waxes, and combinations thereof, which is optionally hydrogenated;

(B-iii) an emollient of animal origin selected from the group consisting of lanolin, perhydrosqualene, beeswax (cera alba), beef tallow, other animal oils, animal butters, animal waxes and combinations thereof, which is optionally hydrogenated; and

combinations of two or more thereof.

An emollient of synthetic origin may be any synthetic oil that is cosmetically acceptable. For instance, an emollient of synthetic origin may be selected from petrolatum (also: vaseline oil), paraffin oils, silicone oils, phenylsilicones, silicone resins and silicone gums, esters of fatty acids with alcohols having a low C number (i.e., preferably $C_1$-$C_6$-alcohols), for example with isopropanol, propylene glycol or glycerol, or esters of fatty alcohols with alkanoic acids having a low C number (i.e., preferably $C_1$-$C_6$-acids) or with fatty acids, alkyl benzoates, and synthetic hydrocarbon oils. Further examples of emollients may be stearyl alcohol, cetearylisononanoate, dioctyl ether, 2-hydroxyethyl stearate, isocetylstearate, or zinc stearate.

Emollients may also be benzoic acid esters of linear or branched $C_8$-$C_{22}$-alkanols, for example isostearyl benzoate, C12-C15-alkyl benzoate or ethylhexyl benzoate. A further class of emollients may be dialkyl ethers having a total of from 12 to 36 carbon atoms, in particular having from 12 to 24 carbon atoms, such as di-n-octyl ether, di-n-nonyl ether, di-n-decyl ether, di-n-undecyl ether, di-n-dodecyl ether, n-hexyl-n-octyl ether, n-octyl-n-decyl ether, n-decyl-n-undecyl ether, n-undecyl-n-dodecyl ether, n-hexyl-n-undecyl ether, di-3-ethyldecyl ether, tert-butyl n-octyl ether, isopentyl n-octyl ether, 2-methylpentyl n-octyl ether, di-tert-butyl ether or diisopentyl ether. Further examples are branched saturated or unsaturated fatty alcohols having from 6 to 30 carbon atoms, for example isostearyl alcohol or Guerbet alcohols. A further class of emollients may be dicarboxylic acid esters of linear or branched $C_2$-$C_{10}$-alkanols, such as di-n-butyl adipate, di-(2-ethylhexyl)adipate or di-(2-ethylhexyl)succinate, or diol esters such as ethylene glycol dioleate, ethylene glycol diisotridecanoate, propylene glycol di-(2-ethylhexanoate), propylene glycol diisostearate, propylene glycol dipelargonate, butanediol diisostearate or neopentyl glycol dicaprylate, or diisotridecyl acetate. Emollients may, for example, be symmetrical, non-symmetrical or cyclic esters of carbonic acid with fatty alcohols, glycerol carbonate or dicaprylyl carbonate. A further class of emollients may be esters of dimers of unsaturated $C_{12}$-$C_{22}$-fatty acids (dimer fatty acids) with monovalent linear, branched or cyclic $C_2$-$C_{18}$-alkanols or with polyvalent linear or branched $C_2$-$C_6$-alkanols. A further class of emollients may be hydrocarbon oils, for example those with linear or branched, saturated or unsaturated $C_7$-$C_{40}$-carbon chains, for example dodecane, isododecane, cholesterol, lanolin, synthetic hydrocarbons such as polyolefins, in particular polyisobutene, hydrogenated polyisobutene, polydecane, as well as hexadecane, isohexadecane, paraffin oils, isoparaffin oils, squalane, squalene, and alicyclic hydrocarbons, for example 1,3-di-(2-ethyl-hexyl)-cyclohexane, ozokerite and ceresin. An emollient may also be a synthetic triglyceride oil. A further example of an emollient may be isopropyl isostearate.

An emollient of synthetic origin may also be selected from the group consisting of purcellin oil, isoparaffins, linear or branched fatty alcohols or fatty acid esters, esters of linear ($C_6$-$C_{18}$) fatty acids with linear ($C_6$-$C_{20}$) fatty alcohols, esters of branched ($C_6$-$C_{18}$) fatty acids with linear ($C_6$-$C_{20}$) fatty alcohols, esters of linear ($C_6$-$C_{18}$) fatty acids with branched alcohols, for example 2-ethylhexanol, esters of linear or branched fatty acids with polyhydric alcohols (such as dimerdiol or trimerdiol) and/or guerbet alcohols, triglycerides based on ($C_6$-$C_{18}$) fatty acids, and esters such as dioctyl adipate, diisopropyl dimer dilinoleate, propylene glycol dicaprylate/dicaprate. Emollients may also be waxes, such as paraffin wax or microwaxes, alone or optionally in combination with hydrophilic waxes, such as cetylstearyl alcohol. Emollients may also be fluorinated or perfluorinated oils or fluorinated silicone oils.

An emollient of plant origin may be any hydrophobic substance of plant origin. In at least one preferred embodiment, the emollient of plant origin comprises or consists of one or more glycerides (also: acylglycerols) selected from the group consisting of monoglycerides (esters of one fatty acid bound to a glycerol moiety), diglycerides (esters of two fatty acids bound to a glycerol moiety), and triglycerides (esters of three fatty acids bound to a glycerol moiety). In at least one preferred embodiment, the emollient of plant origin comprises or consists of one or more triglycerides. A glyceride may optionally also contain one or more other groups, for example selected from the group consisting of choline, phosphocholine, amino acid moieties, phoshoamino acid moieties. Additionally or alternatively, an emollient of plant origin may optionally comprise one or more other ingredients in addition to glycerides, such as tocopherol, carotinoids, phytosteroids, vitamins, oleyl alcohol, terpenes, caryophyllene, other (preferably fat-soluble) secondary plant substances, salts, residual water, etc.

An emollient of plant origin may comprise or consist of oils of plant origin (also: vegetable oils or plant oils) such as liquid triglycerides, for example sunflower oil, corn oil, soybean oil, rice oil, jojoba oil, *Orbignya oleifera* (seed) oil or butter (babassu oil or babassu butter), pumpkin oil, grapeseed oil, sesame oil, walnut oil, *Prunus armeniaca* (kernel) oil (apricot oil), *Macadamia ternifolia* (seed) oil (macadamia oil), groundnut oil, sweet almond oil, lady's-smock oil, *Ricinus communis* (castor) (seed) oil, triglycerides of caprylic/capric acids, olive (fruit) oil (*Olea europaea* (fruit) oil), orange oil, cuckoo flower oil, wheatgerm oil, hydrogenated olive oil, hydrogenated ethylhexyl olivate, peanut oil, *Persea gratissima* oil (avocado oil), *Prunus persica* (kernel) oil (peach (kernel) oil), *Citrus aurantifolia* (seed) oil (lime (seed) oil), rape(seed) oil, argan oil, coconut oil, one or more other plant oils, crambisol (*Crambe abyssinica*) (seed) oil (abyssinian oil), Argania

spinosa (kernel) oil, hydrogenated vegetable oil, *Limnanthes alba* (meadowfoam) (seed) oil, olive squalene, *Camellia sinensis* and/or *Camellia japonica* oil (tsubaki oil), lin seed, *Copernica cerifera* (carnauba) wax, *Olea europaea* (olive) oil unsaponifiables, hydrogenated vegetable oil, hydrogenated olive oil, *Butyrospermum parkii* (shea) butter, *Mangifera indica* (seed) butter (mango butter), *Theobroma cacao* seed) butter (cocoa butter), and combinations of two or more thereof.

An emollient of plant origin may optionally be hydrogenated. A glyceride of plant origin may optionally be hydrated. An emollient of plant origin may also be an extract of one or more oils.

An emollient of plant origin may have any fatty acid composition. Typical fatty acids are linear or branched, saturated or unsaturated, optionally hydroxylated $C_8$-$C_{30}$-fatty acids. In at least one preferred embodiment, an emollient of plant origin may comprise at least 3 wt.-% of an oleic acid moiety, referred to the whole mass of all fatty acid moieties of the emollient of plant origin. In at least one preferred embodiment, an emollient of plant origin may comprise at least 3 wt.-% of a palmitic acid moiety, referred to the whole mass of all fatty acid moieties of the emollient of plant origin.

In at least one preferred embodiment, an emollient of plant origin may comprise:

(a) from 3 to 90 wt.-%, preferably from 5 to 85 wt.-%, more preferably from 10 to 80 wt.-%, in particular from 15 to 75 wt.-%, of an oleic acid moiety, referred to the whole mass of all fatty acid moieties of the emollient of plant origin;
(b) from 3 to 70 wt.-%, preferably from 5 to 65 wt.-%, more preferably from 10 to 60 wt.-%, in particular from 15 to 55 wt.-%, of a palmitic acid moiety, referred to the whole mass of all fatty acid moieties of the emollient of plant origin;
(c) from 0 to 80 wt.-%, preferably from 0.1 to 65 wt.-%, more preferably from 0.5 to 50 wt.-%, in particular from 2 to 45 wt.-%, of a linoleic acid moiety, referred to the whole mass of all fatty acid moieties of the emollient of plant origin;
(d) from 0 to 15 wt.-%, preferably from 0 to 13 wt.-%, more preferably from 0 to 10 wt.-%, in particular from 0 to 5 wt.-%, of a stearic acid moiety, referred to the whole mass of all fatty acid moieties of the emollient of plant origin;
(e) from 0 to 10 wt.-%, preferably from 0 to 9 wt.-%, more preferably from 0 to 7 wt.-%, in particular from 0 to 5 wt.-%, of a palmitoleic acid moiety, referred to the whole mass of all fatty acid moieties of the emollient of plant origin; and
(f) from 0 to 15 wt.-%, preferably from 0 to 13 wt.-%, more preferably from 0 to 10 wt.-%, in particular from 0 to 5 wt.-%, of a linolenic acid moiety, referred to the whole mass of all fatty acid moieties of the emollient of plant origin.

In at least one preferred embodiment, an emollient of plant origin may comprise tocopherol. In at least one preferred embodiment, an emollient of plant origin may comprise up to 25 wt.-% of non-glyceride components. A plant oil can also be an essential oil.

In at least one preferred embodiment, the emollient comprises or consists of *Ricinus communis* (castor) seed oil, olive oil and hydrogenated olive oil. Such composition may be commercially obtained as Clear GL 15 (Clariant). In at least one preferred embodiment, the emollient comprises or consists of *Ricinus communis* (castor) seed oil, hydrogenated castor seed oil, and *Copernica cerifera* (carnauba) wax. Such composition may be commercially obtained as Vegetable Petrolatum R15 (Clariant). In at least one preferred embodiment, the emollient comprises or consists of hydrogenated ethylhexyl olivate and hydrogenated olive oil. Such composition may be commercially obtained as Sensolive LD (Clariant). In at least one preferred embodiment, the emollient comprises or consists of propanediol dicaprylate/caprate and hydrogenated olive oil. Such composition may be commercially obtained as Sensolive LD ECO (Clariant). In at least one preferred embodiment, the emollient comprises or consists of glyceryl rosinate, triethylene glycol rosinate, *Butyrospermum parkii* (Shea) butter, beeswax (cera alba), glyceryl oleate, polyglyceryl-3 polyricinoleate, and olive oil. Such composition may be commercially obtained as VL (Clariant).

An emollient of animal origin may be any hydrophobic substance of animal origin. In at least one preferred embodiment, the emollient of animal origin comprises or consists of one or more glycerides (also: acylglycerols) selected from the group consisting of monoglycerides (esters of one fatty acid bound to a glycerol moiety), diglycerides (esters of two fatty acids bound to a glycerol moiety), and triglycerides (esters of three fatty acids bound to a glycerol moiety). In at least one preferred embodiment, the emollient of animal origin comprises or consists of one or more triglycerides. A glyceride may optionally also contain one or more other groups, for example selected from the group consisting of choline, phosphocholine, amino acid moieties, phoshoamino acid moieties. Additionally or alternatively, an emollient of animal origin may optionally comprise one or more other ingredients in addition to glycerides, such as steroids, squalenes, vitamins, salts, residual water, etc. An emollient of animal origin may, for example, be selected from lanolin, perhydrosqualene, beeswax and beef tallow.

Component C

The cosmetic composition of the invention comprises at least one further cosmetic ingredient as component C.

According to the present invention, the at least one ingredient of component C is selected from the group consisting of at least one conditioning agent, at least one solubilizing agent, at least one emulsifier, and a combination of two or more thereof. It will be understood that an ingredient of component C in the context of the present invention is

typically a cosmetically acceptable ingredient such as a cosmetically acceptable conditioning agent, or a cosmetically acceptable solubilizing and/or emulsifier.

Conditioning agent

In at least one preferred embodiment, the at least one ingredient of component C is at least one conditioning agent. In at least one preferred embodiment, the at least one conditioning agent is selected from the group consisting of estolide esters, esters of oxalkylated alkylalkylene, oligoester ammonium salts and combinations of two or more thereof.
In at least one preferred embodiment, the at least one conditioning agent is an estolide ester. As used herein, the term "estolide ester" means an oligomeric fatty acid ester, wherein the monomers are joined by ester linkages. Preferred estolide esters have the Formula (5). In at least one preferred embodiment, a conditioning agent of component C comprises or consists of a compound of Formula (5):

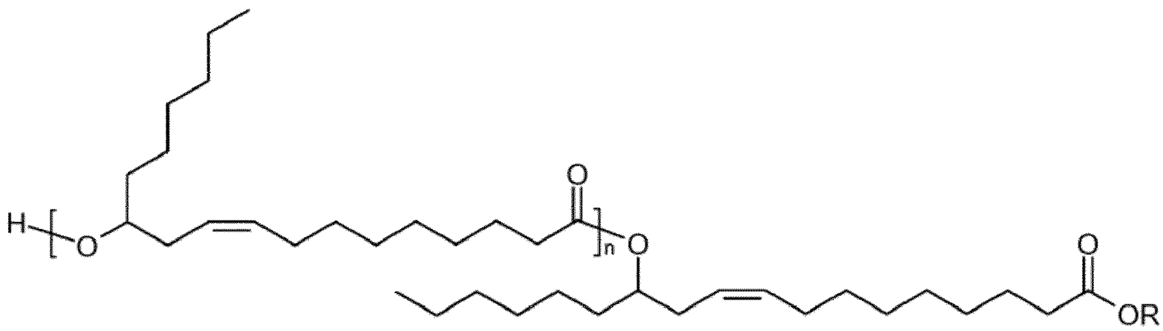

Formula (5)

wherein

R is selected from branched or linear $C_3$-$C_{20}$-alkyl, preferably from branched or linear $C_6$-$C_{18}$-alkyl, more preferably from branched or linear $C_8$-$C_{16}$-alkyl, even more preferably from branched or linear $C_{10}$-$C_{16}$-alkyl, in particular from branched or linear $C_{12}$-$C_{14}$-alkyl; and
n is an integer in the range from 1 to 20, preferably from 1 to 15, more preferably from 1 to 10, even more preferably from 2 to 8, in particular from 3 to 7.

As used herein, the term "estolide ester" means an oligomeric fatty acid ester, wherein the monomers are joined by ester linkages. The term "estolide ester" is a term known in the art - see e.g. WO 2016/174256.
In Formula (5), R is selected from branched or linear $C_3$-$C_{20}$-alkyl, preferably from branched or linear $C_6$-$C_{18}$-alkyl, more preferably from branched or linear $C_8$-$C_{16}$-alkyl, even more preferably from branched or linear $C_{10}$-$C_{16}$-alkyl, in particular from branched or linear $C_{12}$-$C_{14}$-alkyl. In preferred embodiments, R in Formula (5) is selected from linear $C_6$-$C_{18}$-alkyl, more preferably from linear $C_8$-$C_{16}$-alkyl, even more preferably from linear $C_{10}$-$C_{16}$-alkyl, in particular from linear $C_{12}$-$C_{14}$-alkyl. In Formula (5), n is selected from 1 to 20, preferably from 1 to 15, more preferably from 1 to 10, even more preferably from 2 to 8, in particular from 3 to 7. In at least one preferred embodiment, R in Formula (5) is selected from branched or linear $C_{10}$-$C_{16}$-alkyl; and n in Formula (5) is selected from 2 to 8, in particular from 3 to 7. In at least one preferred embodiment, R in Formula (5) is selected from linear $C_{10}$-$C_{16}$-alkyl; and n in Formula (5) is selected from 2 to 8, in particular from 3 to 7. In at least one preferred embodiment, R in Formula (5) is selected from branched or linear $C_{12}$-$C_{14}$-alkyl; and n in Formula (5) is selected from 2 to 8, in particular from 3 to 7. In at least one preferred embodiment, R in Formula (5) is selected from linear $C_{12}$-$C_{14}$-alkyl; and n in Formula (5) is selected from 2 to 8, in particular from 3 to 7.
As used herein, the term "estolide ester" means an oligomeric fatty acid ester, wherein the monomers are joined by ester linkages. The term "estolide ester" is a term known in the art - see e.g. WO 2016/174256.
Since R in Formula (5) is not hydrogen, the compounds of Formula (5) are esters, not acids. Such esters are beneficial over the corresponding acids. Acids are prone to self-aggregation, which can cause stability issues. Esters of Formula (5) provide excellent stability. The compounds of Formula (5) have an unsaturated backbone, which advantageously leads to lower melting points, higher solubility and higher biodegradability.
Estolide esters having the Formula (5) may be prepared by esterification starting from ricinoleic acid or from commercially available self-condensation products, such as Hostagliss L2, L4, and L6 (Clariant). The number of Hostagliss describes the average degree of oligomerization. L2 is a product mixture with an average molecular weight of

dimers; L4 is a product mixture with an average molecular weight of tetramers; and L6 is a product mixture with an average molecular weight of hexamers. Preferably, 2 to 6 equivalents of ricinoleic acid may be esterified with 0.5 to 2 equivalents of one or several branched or linear $C_3$-$C_{20}$-alkyl alcohols. The esterification may be acid-catalyzed. Suitable acid catalysts are e.g. hypophosphoric acid, methane sulfonic acid, p-toluene sulfonic acid, phosphoric acid, or sulfuric acid.

Ricinoleic acid is a natural occurring fatty acid and therefore a renewable material. Estolide esters having the Formula (5) may be derived from R-ricinoleic acid, S-ricinoleic acid, or a mixture thereof. In at least one preferred embodiment, compounds of Formula (5) contain more than 70 wt.-% of the R-ricinoleic acid stereoisomer. In another preferred embodiment, compounds of Formula (5) contain more than 70 wt.-% of the S-ricinoleic acid stereoisomer.

Estolide esters having the Formula (5) and their preparation are further described in WO 2016/174256. An estolide ester of Formula (5) may be commercially obtained as in the product Genadvance Hydra.

In at least one preferred embodiment, component C is a conditioning agent comprising or consisting of polyricinoleate. In at least one preferred embodiment, component C comprises or consists of lauryl/myristyl polyricinoleate.

In at least one preferred embodiment, component C is a conditioning agent comprising or consisting of polyricinoleate and optionally glycerol (also: glycerin). In at least one preferred embodiment, component C comprises or consists of lauryl/myristyl polyricinoleate and optionally glycerol (also: glycerin).

In at least one preferred embodiment, the at least one component C is at least one ester of an oxalkylated alkylalkylene diamine and/or a cosmetically acceptable salt thereof (e.g., a quaternized salt thereof). A preferred ester of an oxalkylated alkylalkylene diamine has the Formula (6). In at least one preferred embodiment, the at least one component C comprises or consists of a compound of Formula (6) or a cosmetically acceptable optionally quaternized salt thereof:

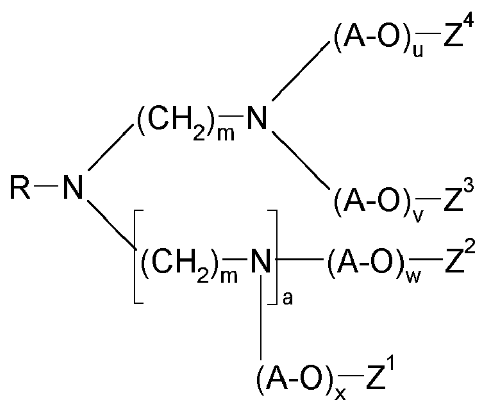

Formula (6)

wherein

| | |
|---|---|
| R | is $C_8$-$C_{24}$-alkyl or $C_8$-$C_{24}$-alkenyl, in particular $C_{10}$-$C_{20}$-alkyl or $C_{10}$-$C_{20}$-alkenyl; |
| A | is each independently a group -$C_2H_4$- or -$C_3H_6$-, in particular a group -$C_2H_4$-; |
| $Z^1$ | is a group -C(O)-R', wherein R' is $C_5$-$C_{35}$-alkyl or $C_5$-$C_{35}$-alkenyl, in particular $C_8$-$C_{24}$-alkyl or $C_8$-$C_{24}$-alkenyl; |
| $Z^2$ | is a group -C(O)-R", wherein R" is $C_5$-$C3_5$-alkyl or $C_5$-$C_{35}$-alkenyl, in particular $C_8$-$C_{24}$-alkyl or $C_8$-$C_{24}$-alkenyl; |
| $Z^3$ | is a group -C(O)-R''', wherein R''' is $C_5$-$C3_5$-alkyl or $C_5$-$C_{35}$-alkenyl, in particular $C_8$-$C_{24}$-alkyl or $C_8$-$C_{24}$-alkenyl; |
| $Z^4$ | is a group -C(O)-R"", wherein R"" is $C_5$-$C_{35}$-alkyl or $C_5$-$C_{35}$-alkenyl, in particular $C_8$-$C_{24}$-alkyl or $C_8$-$C2_4$-alkenyl; |
| a | is 0 or 1, in particular 0; |
| m | is 2 or 3, in particular 3; |
| u, v, w and x | are each independently numbers from 1 to 9, in particular 2 to 9. |

In more preferred embodiments, a conditioning agent of component C comprises an ester of an oxalkylated alkylalkylene diamine having the Formula (6) and/or a cosmetically acceptable salt thereof (e.g., quaternized salts thereof),
wherein

R is $C_8$-$C_{24}$-alkyl or $C_8$-$C_{24}$-alkenyl, in particular $C_{10}$-$C_{20}$-alkyl or $C_{10}$-$C_{20}$-alkenyl;
A is each a group -$C_2H_4$-;
$Z^1$ is -C(O)-R’, wherein R’ is $C_8$-$C_{18}$-alkyl or $C_8$-$C_{18}$-alkenyl;
$Z^2$ is -C(O)-R", wherein R" is $C_8$-$C_{18}$-alkyl or $C_8$-$C_{18}$-alkenyl;
$Z^3$ is -C(O)-R’’’, wherein R’" is $C_8$-$C_{18}$-alkyl or $C_8$-$C_{18}$-alkenyl;
$Z^4$ is -C(O)-R"", wherein R"" is $C_8$-$C_{18}$-alkyl or $C_8$-$C_{18}$-alkenyl;
a is 0;
m is 2 or 3, in particular 3;
u, v and w are each independently numbers from 3 to 9, in particular 5 to 9, where the salt is formed by quaternizing one or two of the nitrogen atoms of the compound of Formula (6).

In even more preferred embodiments, a conditioning agent of component C comprises an ester of an oxalkylated alkylalkylene diamine having the Formula (6) and/or a cosmetically acceptable salt thereof (e.g., quaternized salts thereof),
wherein

R is $C_8$-$C_{18}$-alkyl or $C_8$-$C_{18}$-alkenyl;
A is each a group -$C_2H_4$-;
$Z^1$, $Z^2$, $Z^3$ and $Z^4$ are the same and are -C(O)-R’,
wherein R’ is $C_8$-$C_{18}$-alkyl or $C_8$-$C_{18}$-alkenyl;
a is 0;
m is 3;
u, v and w are each independently numbers from 5 to 8, in particular 7 to 8, where the salt is formed by quaternizing one or two of the nitrogen atoms of the compound of Formula (6).

The cosmetically acceptable salts thereof preferably are quaternized salts of the compounds of Formula (6). The quaternized salts of the compounds of Formula (6) can be formed by quaternizing one or two or more of the nitrogen atoms of the compound of Formula (6), e.g. by using an alkylating agent. Preferred alkylating agents are $C_1$-$C_4$-alkylating agents. Particularly preferred alkylating agents are methylating agents. Examples of alkylating agents are dimethyl sulfate, diethyl sulfate, dimethyl carbonate, diethyl carbonate, methyl chloride, ethyl chloride, methyl bromide, ethyl bromide, methyl iodide or ethyl iodide. Examples of methylating agents are dimethyl sulfate, dimethyl carbonate, methyl chloride, methyl bromide or methyl iodide. A particularly preferred alkylating agent / methylating agent is dimethyl sulfate.

Esters of oxalkylated alkylalkylene diamines having the Formula (6) and quaternized salts thereof as well as their preparation are further described in WO 2015/110269 and WO 2019/175124. A compound of Formula (5) may be commercially obtained as in the product Genadvance Repair.

In at least one preferred embodiment, a conditioning agent of component C is at least one oligoester ammonium salt. A preferred oligoester ammonium salt is obtainable by the following steps. In at least one preferred embodiment, a conditioning agent of component C comprises or consist of an oligoester ammonium salt (OAS) obtainable by the following steps:

(i) heating a mixture of the following components of Formulae (I), (II), (III) and (IV) under continuous removal of reaction water:

0.5 to 3.0 molar equivalents, preferably 0.75 to 3.0 molar equivalents, of a diethanolamine compound represented by the Formula (I)

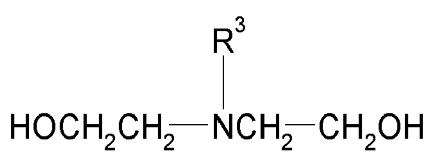
(I)

wherein $R^3$ is linear or branched $C_1$-$C_6$-alkyl, preferably linear or branched $C_1$-$C_4$-alkyl, more preferably methyl or ethyl;

0.5 to 1.5 molar equivalents of a dicarboxylic acid of Formula (II)

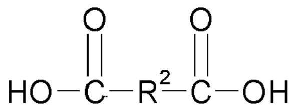

(II)

wherein $R^2$ is linear or branched $C_1$-$C_{10}$-alkylene or linear or branched $C_2$-$C_{10}$-alkenylene, preferably linear or branched $C_2$-$C_8$-alkylene, more preferably linear or branched $C_4$-alkylene;

0.5 to 1.5 molar equivalents of an organic triol (III) represented by the Formula (III-1) or (III-2)

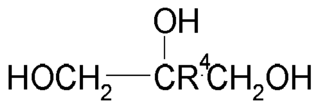

or

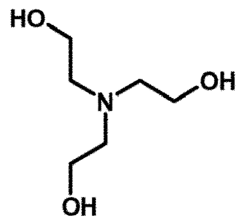

(III-1) (III-2)

wherein $R^4$ is hydrogen or linear or branched $C_1$-$C_4$-alkyl or hydroxyl-$C_1$-$C_4$-alkyl, preferably hydrogen, methyl or ethyl, more preferably hydrogen;

1.0 molar equivalent of a monocarboxylic acid represented by Formula (IV)

$R^1$-COOH (IV)

wherein $R^1$ is linear or branched $C_{11}$-$C_{25}$-alkyl or linear or branched $C_{11}$-$C_{25}$-alkenyl, or wherein $R^1$ is linear or branched $C_{12}$-$C_{24}$-alkyl or linear or branched $C_{12}$-$C_{24}$-alkenyl, preferably linear or branched $C_{11}$-$C_{23}$-alkyl or linear or branched $C_{11}$-$C_{23}$-alkenyl, more preferably linear or branched $C_{19}$-$C_{23}$-alkyl; or wherein $R^1$ is linear or branched $C_{12}$-$C_{24}$-alkyl or linear or branched $C_{12}$-$C_{24}$-alkenyl;

(ii) reacting the oligoester product of step (i) with a quaternization agent (V), in particular dimethyl sulfate, diethyl sulfate or alkyl halides; and

(iii) optionally purifying the oligoester ammonium salt (OAS).

Preferred OAS are obtainable by the above steps using N-methyl diethanolamine as the diethanolamine compound (I).

Preferred OAS are obtainable by the above steps using a dicarboxylic acid (II) which is selected from the group consisting of adipic acid, sebacic acid, glutaric acid, succinic acid, itaconic acid, maleic acid, and combinations thereof. Particularly preferred OAS are obtainable by the above steps using adipic acid as the dicarboxylic acid (II). Also particularly preferred OAS are obtainable by the above steps using sebacic acid as the dicarboxylic acid (II).

Preferred OAS are obtainable by the above steps using an organic triol (III) which is selected from the group consisting of glycerol, triethanolamine, and combinations thereof. Particularly preferred OAS are obtainable by the above steps using glycerol as the organic triol (III). Also particularly preferred OAS are obtainable by the above steps using triethanolamine as the organic triol (III).

Preferred OAS are obtainable by the above steps using a carboxylic acid (IV) selected from the group consisting of behenic acid, oleic acid, coconut fatty acid, linoleic acid, and combinations thereof. Particularly preferred OAS are obtainable by the above steps using behenic acid as the carboxylic acid (IV).

In a particularly preferred embodiment, a conditioning agent of component C comprises an oligoester ammonium salt (OAS) obtainable by the above steps using N-methyl diethanolamine as the diethanolamine compound (I), adipic acid as the dicarboxylic acid (II), glycerol as the organic triol (III), and behenic acid as the carboxylic acid (IV).

In a particularly preferred embodiment, a conditioning agent of component C comprises an oligoester ammonium salt (OAS) obtainable by the above steps using N-methyl diethanolamine as the diethanolamine compound (I), adipic acid as the dicarboxylic acid (II), triethanolamine as the organic triol (III), and behenic acid as the carboxylic acid (IV).

In a particularly preferred embodiment, a conditioning agent of component C comprises an oligoester ammonium salt (OAS) obtainable by the above steps using N-methyl diethanolamine as the diethanolamine compound (I), sebacic acid as the dicarboxylic acid (II), glycerol as the organic triol (III), and behenic acid as the carboxylic acid (IV).

In a particularly preferred embodiment, a conditioning agent of component C comprises an oligoester ammonium salt (OAS) obtainable by the above steps using N-methyl diethanolamine as the diethanolamine compound (I), sebacic acid as the dicarboxylic acid (II), triethanolamine as the organic triol (III), and behenic acid as the carboxylic acid (IV).

Preferred OAS are obtainable by the above steps using a quaternization agent (V) selected from the group consisting of dimethyl sulfate, diethyl sulfate, methyl chloride, ethyl chloride, butyl chloride, and combinations thereof. Particularly preferred OAS are obtainable by the above steps using dimethyl sulfate as the quaternization agent (V).

Preferred OAS are obtainable by the above steps, wherein the molar ratio of the components of Formulae (I), (II), (III) and (IV) is chosen such that the molar equivalents of hydroxyl functions are in excess of the molar equivalents of acid functions.

Preferred OAS are obtainable by the above steps, wherein in step (i) the mixture of the components of Formulae (I), (II), (III) and (IV) is heated to a temperature from 80 to 220°C, preferably from 150 to 210°C, more preferably from 160 to 200°C, and in step (ii) the oligoester product of step (i) is reacted with a quaternization agent (V) which is selected from the group consisting of dimethyl sulfate, diethyl sulfate, methyl chloride, ethyl chloride, butyl chloride, and combinations thereof.

Preferred OAS have a molecular mass Mn (number average) of from 500 to 4000 g/mol, preferably from 600 to 3000 g/mol, more preferably from 650 to 2000 g/mol, more preferably from 800 to 1900 g/mol, even more preferably from 665 to 1800 g/mol, even more preferably from 900 to 1800 g/mol.

Oligoester ammonium salts (OAS) and their preparation are further described in WO 2017/097816, WO 2017/097817 and WO 2017/097819. An oligoester ammonium salt (OAS) may be commercially obtained as in the product Genadvance Life.

Solubilizing agent and/or emulsifier

In at least one preferred embodiment, the at least one ingredient of component C is at least one solubilizing agent and/or emulsifier.

As used herein, the term "solubilizing agent" may be understood in the broadest sense as generally understood in the field of cosmetics. A solubilizing agent may be suitable to support or facilitate incorporating the solubilizate (the component that undergoes solubilization) into or onto micelles.

As used herein, the term "emulsifier" may be understood in the broadest sense as generally understood in the field of cosmetics. An emulsifier may be understood as an ingredient that may stabilize an emulsion. This may be achieved by increasing kinetic stability. Preferably, an emulsifier has an amphiphilic structure, i.e., has a polar or hydrophilic (i.e. water-soluble) part and a non-polar (i.e. hydrophobic or lipophilic) part. An emulsifier may stabilize an oil-in-water and/or a water-in-oil emulsion. An emulsifier may be a surfactant ("surface active agent"). An emulsifier may also serve as a co-emulsifier. Thus, optionally, a combination of two or more emulsifiers or of one or more emulsifiers and one or more solubilizing agents may be used.

An emulsifier can optionally also act as a solubilizing agent and vice versa. Thus, optionally in some circumstances, an emulsifier may concomitantly also be a solubilizing agent. Optionally in some circumstances, a solubilizing agent may also be an emulsifier.

In at least one preferred embodiment, the composition of the invention comprises one or more solubilizing agents and/or emulsifiers. For example, the composition of the invention may comprise two or more solubilizing agents and/or emulsifiers, or three or more solubilizing agents and/or emulsifiers. For example, the composition of the invention may comprise one to three solubilizing agents and/or emulsifiers, or one or two solubilizing agents and/or emulsifiers, or two or three solubilizing agents and/or emulsifiers.

Preferably, the solubilizing agent and/or emulsifier is selected from N-methyl-N-acylglucamines, anhydro methyl glucamides, glyceryl fatty acid esters, polyglyceryl fatty acid esters, polyglyceryl polyesters, sorbitan esters, alkyl glycosides, alkyl polyglycosides, stearoyl lactylate, fatty acid sugar esters (in particular fatty acid sucrose esters), sugar polyesters (in particular sucrose polyesters), fatty alcohols, fatty alcohol ethoxylates, fatty acid ethoxylates, and combinations thereof.

More preferably, the solubilizing agent and/or emulsifier is selected from N-methyl-N-acylglucamines, anhydro methyl glucamides, glyceryl fatty acid esters, polyglyceryl polyesters, sorbitan esters, alkyl glycosides, stearoyl lactylate, sugar polyesters (in particular sucrose polyesters), fatty alcohols, and combinations thereof.

In a particular embodiment, the solubilizing agent and/or emulsifier is selected from, sugar polyesters (in particular sucrose polyesters), fatty alcohols, and combinations thereof.

In at least one preferred embodiment, the solubilizing agent and/or emulsifier is selected from N-methyl-N-acylglucamines, preferably N-methyl-N-acylglucamines of Formula (X):

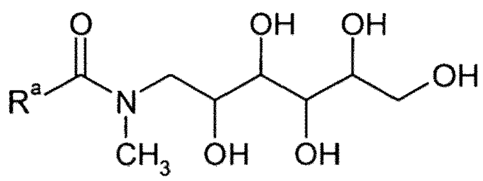

(X)

wherein $R^a$ is selected from saturated or unsaturated hydrocarbon chains having 5 to 23 carbon atoms. Preferably, $R^a$ in Formula (X) is selected from saturated or unsaturated hydrocarbon chains having 7 to 17 carbon atoms. In preferred embodiments, $R^a$ in Formula (X) is selected from saturated hydrocarbon chains having 7 to 17 carbon atoms. In preferred embodiments, $R^a$ in Formula (X) is selected from unsaturated hydrocarbon chains having 7 to 17 carbon atoms. Also preferably, the $R^a$-C=O residue in Formula (X) is derived from caprylic acid, capric acid, lauric acid, myristic acid, palmitic acid, stearic acid, oleic acid, linoleic acid, linolenic acid, or mixtures thereof. Also preferably, the $R^a$-C=O residue in Formula (X) is derived from coconut oil. Also preferably, the $R^a$-C=O residue in Formula (X) is derived from 9-decenoic acid, 9-dodecenoic acid, or mixtures thereof. Particularly preferred N-methyl-N-acylglucamines of Formula (X) are capryloyl/caproyl methyl glucamide, lauroyl/myristoyl methyl glucamide, cocoyl methyl glucamide, oleyl methyl glucamide, or mixtures thereof. Such N-methyl-N-acylglucamines are commercially available from Clariant (GlucoTain® Clear, GlucoTain® Plus, GlucoTain® Flex, GlucoTain® Care, GlucoTain® Sense). Also particularly preferred N-methyl-N-acylglucamines of Formula (X) are N-9-decenoyl-N-methylglucamine, N-9-dodecenoyl-N-methylglucamine, or mixtures thereof.

In at least one preferred embodiment, the solubilizing agent and/or emulsifier is selected from anhydro methyl glucamides, preferably anhydro methyl glucamides of Formula (XI),

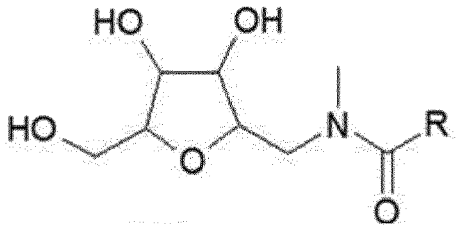

(XI)

wherein R is selected from saturated or unsaturated hydrocarbon chains having 5 to 23 carbon atoms. Preferably, R in Formula (XI) is selected from saturated or unsaturated hydrocarbon chains having 7 to 17 carbon atoms. More preferably, R in Formula (XI) is selected from saturated or unsaturated hydrocarbon chains having 7 to 13 carbon atoms. Even more preferably, R in Formula (XI) is $-(CH_2)_6CH_3$, $-(CH_2)_8CH_3$, $-(CH_2)_{10}CH_3$, $-(CH_2)_{12}CH_3$, or mixtures thereof. Also even more preferably, the R-C=O residue in Formula (XI) is derived from coconut oil. Also even more preferably, the R-C=O residue in Formula (XI) is derived from 9-decenoic acid, 9-dodecenoic acid, or mixtures thereof. Particularly preferably, R in Formula (XI) is $-(CH_2)_6CH_3$, $-(CH_2)_8CH_3$, or mixtures thereof. Capryloyl/caproyl anhydro methyl glucamide is commercially available from Clariant (Velsan® Flex).

In at least one preferred embodiment, the solubilizing agent and/or emulsifier is selected from the group consisting of glyceryl fatty acid esters. Preferred glyceryl fatty acid esters are esters of glycerol and one or more C8-C20 fatty acids. Preferably, the glyceryl fatty acid esters are mono- or diesters of glycerol and one or more C8-C20 fatty acids. Particularly preferably, the glyceryl fatty acid esters are monoesters of glycerol and one or more C8-C20 fatty acids. Also particularly preferably, the glyceryl fatty acid esters are diesters of glycerol and one or more C8-C20 fatty acids. Also particularly preferably, the glyceryl fatty acid esters are mixtures of mono- and diesters of glycerol and one or more C8-C20 fatty acids.

The fatty acids may be saturated or unsaturated. Preferred fatty acids are C12-C18 fatty acids. Preferably, the fatty acids are selected from oleic acid, capric acid, caprylic acid, lauric acid, myristic acid, palmitic acid, stearic acid, ricinoleic acid, and mixtures thereof. Particularly preferred fatty acids are oleic acid or stearic acid. Also preferred are fatty acid mixtures derived from coconut oil.

Examples of preferred glyceryl fatty acid esters are glyceryl oleate, glyceryl stearate, glyceryl caprate, glyceryl caprylate, glyceryl laurate, glyceryl myristate, glyceryl palmitate, glyceryl cocoate, glyceryl ricinoleate, or mixtures thereof. Particularly preferred glyceryl fatty acid esters are glyceryl oleate or glyceryl stearate.

In at least one preferred embodiment, the solubilizing agent and/or emulsifier is selected from the group consisting of polyglyceryl fatty acid esters. Preferred polyglyceryl fatty acid esters are esters of polyglycerol having 2 to 20 glyceryl units and one or more C8-C20 fatty acids.

Preferably, the polyglyceryl fatty acid esters are mono-, di-, tri- or tetraesters, more preferably mono-, di- or triesters, even more preferably mono- or diesters of polyglycerol and one or more C8-C20 fatty acids. Particularly preferably, the polyglyceryl fatty acid esters are monoesters of polyglycerol and one or more C8-C20 fatty acids. Also particularly preferably, the polyglyceryl fatty acid esters are diesters of polyglycerol and one or more C8-C20 fatty acids. Also particularly preferably, the polyglyceryl fatty acid esters are mixtures of mono- and diesters of polyglycerol and one or more C8-C20 fatty acids.

Preferably, the polyglycerols have 2 to 4 glyceryl units, more preferably 2 or 3 glyceryl units, particularly preferably 2 glyceryl units.

In preferred embodiments, the polyglyceryl fatty acid esters are mono- or diesters of polyglycerol having 2 to 4, preferably 2 or 3, particularly preferably 2 glyceryl units and one or more C8-C20 fatty acids. In preferred embodiments, the polyglyceryl fatty acid esters are monoesters of polyglycerol having 2 to 4, preferably 2 or 3, particularly preferably 2 glyceryl units and one or more C8-C20 fatty acids. In preferred embodiments, the polyglyceryl fatty acid esters are diesters of polyglycerol having 2 to 4, preferably 2 or 3, particularly preferably 2 glyceryl units and one or more C8-C20 fatty acids. In preferred embodiments, the polyglyceryl fatty acid esters are mixtures of mono- and diesters of polyglycerol having 2 to 4, preferably 2 or 3, particularly preferably 2 glyceryl units and one or more C8-C20 fatty acids.

The fatty acids may be saturated or unsaturated. Preferred fatty acids are C12-C18 fatty acids. Preferably, the fatty acids are selected from stearic acid, isostearic acid, capric acid, caprylic acid, lauric acid, myristic acid, palmitic acid, oleic acid, ricinoleic acid, and mixtures thereof. Particularly preferred fatty acids are stearic acid or isostearic acid. Also preferred are fatty acid mixtures derived from coconut oil. Examples of preferred polyglyceryl fatty acid esters are polyglyceryl-2 stearate, polyglyceryl-3 stearate, polyglyceryl-4 stearate, polyglyceryl-2 sesquistearate, polyglyceryl-2 sesquiisostearate, polyglyceryl-2 caprate, polyglyceryl-3 caprate, polyglyceryl-4 caprate, polyglyceryl-2 caprylate, polyglyceryl-3 caprylate, polyglyceryl-4 caprylate, polyglyceryl-2 laurate, polyglyceryl-3 laurate, polyglyceryl-4 laurate, polyglyceryl-2 myristate, polyglyceryl-3 myristate, polyglyceryl-4 myristate, polyglyceryl-2 palmitate, polyglyceryl-3 palmitate, polyglyceryl-4 palmitate, polyglyceryl-2 oleate, polyglyceryl-3 oleate, polyglyceryl-4 oleate, polyglyceryl-2 cocoate, polyglyceryl-3 cocoate, polyglyceryl-4 cocoate, polyglyceryl-2 ricinoleate, polyglyceryl-3 ricinoleate, polyglyceryl-4 ricinoleate, or mixtures thereof. Particularly preferred polyglyceryl fatty acid esters are polyglyceryl-2 stearate or polyglyceryl-2 sesquiisostearate. Polyglyceryl-2 stearate is, e.g., commercially available from Clariant (Plantasens® Emulsifier DGDS). Polyglyceryl-2 sesquiisostearate is, e.g. commercially available from Clariant (Plantasens® Emulsifier DGI).

In at least one preferred embodiment, the solubilizing agent and/or emulsifier is selected from the group consisting of polyglyceryl polyesters. Preferred polyglyceryl polyesters are polyglyceryl polyricinoleates. An example of a polyglyceryl polyricinoleate is polyglyceryl-3 polyricinoleate.

In at least one preferred embodiment, the solubilizing agent and/or emulsifier is selected from sorbitan esters. Preferred sorbitan esters may be selected from polyethylene glycol (20) sorbitan monolaurate, polyethylene glycol (20) sorbitan monostearate, polyethylene glycol (20) sorbitan monoisostearate, polyethylene glycol (20) sorbitan monopalmitate, and polyethylene glycol (20) sorbitan monooleate. Also preferred sorbitan esters may be selected from sorbitan caprylate, sorbitan stearate, sorbitan isostearate, sorbitan olivate, sorbitan oleate, sorbitan sesquioleate, sorbitan laurate, and sorbitan palmitate. Particularly preferred sorbitan esters are sorbitan caprylate, sorbitan olivate or sorbitan oleate. Sorbitan caprylate is commercially available from Clariant (Velsan® SC).

In at least one preferred embodiment, the solubilizing agent and/or emulsifier is selected from alkyl glycosides and alkyl polyglycosides. Typically, alkyl glycosides or alkyl polyglycosides comprise an alkyl group connected (optionally via a bridging group) to a block of one or more glycosyl groups. Preferred alkyl glycosides or alkyl polyglycosides can be defined by the following Formula (X'):

$$RO\text{-}(G)_n \qquad (X')$$

wherein R is a linear or branched C5-C20 alkyl group; G is a saccharide group; and n is from 1 to 20. Preferably, R is a linear or branched C8-C18 alkyl group, more preferably a linear or branched C12-C18 alkyl group, particularly preferably a linear C16-C18 alkyl group. Preferably, G is a $C_5$- or $C_6$-monosaccharide residue, particularly preferably a glucoside. Also preferably, G is selected from a glucose residue, xylose residue, lactose residue, fructose residue, and mannose residue. Particularly preferably, G is a glucose residue. Preferably, n is from 1 to 10, more preferably from 1 to 5, even more preferably from 1 to 2, particularly preferably 1. In preferred embodiments, the alkyl glycoside or alkyl polyglycoside is an alkyl glucoside or alkyl polyglucoside. An example of an alkyl glucoside is cetearyl glucoside.

In at least one preferred embodiment, the solubilizing agent and/or emulsifier is a stearoyl lactylate, such as sodium stearoyl lactylate.

In at least one preferred embodiment, the solubilizing agent and/or emulsifier is selected from fatty acid sugar esters, preferably fatty acid sucrose esters, in particular esters of sucrose and one or two C8- to C22-fatty acids. Examples of fatty acid sucrose esters are sucrose cocoate, sucrose dilaurate, sucrose distearate, sucrose laurate, sucrose myristate, sucrose oleate, sucrose palmitate, sucrose ricinoleate, or sucrose stearate.

In at least one preferred embodiment, the solubilizing agent and/or emulsifier is selected from sugar polyesters, preferably sucrose polyesters. An example of a sucrose polyester is sucrose polystearate.

In at least one preferred embodiment, the solubilizing agent and/or emulsifier is selected from fatty alcohols, preferably $C_8$- to $C_{22}$-fatty alcohols, more preferably $C_{12}$- to $C_{22}$-fatty alcohols, even more preferably $C_{16}$- to $C_{22}$-fatty alcohols, particularly preferably $C_{16}$- to $C_{18}$-fatty alcohols. Examples of fatty alcohols are cetyl alcohol, stearyl alcohol, cetearyl alcohol, behenyl alcohol, or isobehenyl alcohol. A particularly preferred fatty alcohol is cetearyl alcohol.

In at least one preferred embodiment, the solubilizing agent and/or emulsifier is selected from fatty alcohol ethoxylates. Fatty alcohol ethoxylates are also called ethoxylated fatty alcohols. Preferably, the fatty alcohol ethoxylates are $C_8$- to $C_{22}$-fatty alcohol ethoxylates, more preferably $C_{12}$- to $C_{18}$-fatty alcohol ethoxylates. Preferably, the number of ethyleneoxy ($-CH_2CH_2O-$) units in the fatty alcohol ethoxylates is from 1 to 30, more preferably from 5 to 25, particularly preferably from 5 to 20. Fatty alcohol ethoxylates may, for example, be selected from the group consisting of ethoxylated stearyl alcohols, isostearyl alcohols, cetyl alcohols, isocetyl alcohols, oleyl alcohols, lauryl alcohols, isolauryl alcohols and cetylstearyl alcohols. Examples of fatty alcohol ethoxylates are polyethylene glycol (13) stearyl ether, polyethylene glycol (14) stearyl ether, polyethylene glycol (15) stearyl ether, polyethylene glycol (16) stearyl ether, polyethylene glycol (17) stearyl ether, polyethylene glycol (18) stearyl ether, polyethylene glycol (19) stearyl ether, polyethylene glycol (20) stearyl ether, polyethylene glycol (12) isostearyl ether, polyethylene glycol (13) isostearyl ether, polyethylene glycol (14) isostearyl ether, polyethylene glycol (15) isostearyl ether, polyethylene glycol (16) isostearyl ether, polyethylene glycol (17) isostearyl ether, polyethylene glycol (18) isostearyl ether, polyethylene glycol (19) isostearyl ether, polyethylene glycol (20) isostearyl ether, polyethylene glycol (13) cetyl ether, polyethylene glycol (14) cetyl ether, polyethylene glycol (15) cetyl ether, polyethylene glycol (16) cetyl ether, polyethylene glycol (17) cetyl ether, polyethylene glycol (18) cetyl ether, polyethylene glycol (19) cetyl ether, polyethylene glycol (20) cetyl ether, polyethylene glycol (13) isocetyl ether, polyethylene glycol (14) isocetyl ether, polyethylene glycol (15) isocetyl ether, polyethylene glycol (16) isocetyl ether, polyethylene glycol (17) isocetyl ether, polyethylene glycol (18) isocetyl ether, polyethylene glycol (19) isocetyl ether, polyethylene glycol (20) isocetyl ether, polyethylene glycol (12) oleyl ether, polyethylene glycol (13) oleyl ether, polyethylene glycol (14) oleyl ether, polyethylene glycol (15) oleyl ether, polyethylene glycol (12) lauryl ether, polyethylene glycol (12) isolauryl ether, polyethylene glycol (13) cetylstearyl ether, polyethylene glycol (14) cetylstearyl ether, polyethylene glycol (15) cetylstearyl ether, polyethylene glycol (16) cetylstearyl ether, polyethylene glycol (17) cetylstearyl ether, polyethylene glycol (18) cetylstearyl ether, polyethylene glycol (19) cetylstearyl ether, or ceteareth-20.

In at least one preferred embodiment, the solubilizing agent and/or emulsifier is selected from fatty acid ethoxylates. Fatty acid ethoxylates are also called ethoxylated fatty acids. Preferably, the fatty acid ethoxylates are $C_8$- to $C_{22}$-fatty acid ethoxylates, more preferably $C_{12}$- to $C_{18}$-fatty acid ethoxylates. Preferably, the number of ethyleneoxy ($-CH_2CH_2O-$) units in the fatty acid ethoxylates is from 1 to 30, more preferably from 5 to 25, particularly preferably from 10 to 25. Fatty acid ethoxylates may, for example, be selected from the group consisting of ethoxylated stearates, isostearates and oleates. Examples of fatty acid ethoxylates are polyethylene glycol (20) stearate, polyethylene glycol (21) stearate, polyethylene glycol (22) stearate, polyethylene glycol (23) stearate, polyethylene glycol (24) stearate, polyethylene glycol (25) stearate, polyethylene glycol (12) isostearate, polyethylene glycol (13) isostearate, polyethylene glycol (14) isostearate, polyethylene glycol (15) isostearate, polyethylene glycol (16) isostearate, polyethylene glycol (17) isostearate, polyethylene glycol (18) isostearate, polyethylene glycol (19) isostearate, polyethylene glycol (20) isostearate, polyethylene glycol (21) isostearate, polyethylene glycol (22) isostearate, polyethylene glycol (23) isostearate, polyethylene glycol (24) isostearate, polyethylene glycol (25) isostearate, polyethylene glycol (12) oleate, polyethylene glycol (13) oleate, polyethylene glycol (14) oleate, polyethylene glycol (15) oleate, polyethylene glycol (16) oleate, polyethylene glycol (17) oleate, polyethylene glycol (18) oleate, polyethylene glycol (19) oleate, or polyethylene glycol (20) oleate.

In at least one preferred embodiment, the solubilizing agent and/or emulsifier may be a blend of polyglyceryl-3 polyricinoleate and sorbitan oleate (e.g., Plantasens Emulsifier CP40 from Clariant), or a blend of cetearyl glucoside and sorbitan olivate (e.g., Plantasens Natural Emulsifier HE20 from Clariant) or a blend of glyceryl stearate, cetearyl alcohol and sodium stearoyl lactylate (e.g., Plantasens Natural Emulsifier HP30 from Clariant), or a blend of glyceryl oleate and polyglyceryl-3-polyricinoleate and *Olea europaea* (olive) oil unsaponifiables (e.g., Plantasens Natural Emulsifier CP5 from Clariant), or a blend of sucrose polystearate and cetearyl alcohol and *Olea europaea* (olive) oil unsaponifiables (e.g., Plantasens Natural Emulsifier HP10 from Clariant).

In at least one preferred embodiment, the solubilizing agent and/or emulsifier is selected from the group consisting of glyceryl monostearate, glyceryl monooleate, diglyceryl monostearate, glyceryl isostearate, polyglyceryl-3-oleate, polyglyceryl-3-diisostearate, polyglyceryl-4-isostearate, polyglyceryl-2-sesquiisostearate, polyglyceryl-3 polyricinoleate, polyglyceryl-2-dipolyhydroxystearate, polyglyceryl-4-dipolyhydroxystearate, PEG-30-dipolyhydroxystearate, diisostearoylpolyglyceryl-3-diisostearate, glycol distearate, polyglyceryl-3-dipolyhydroxystearate, sorbitan monoisostearate, sorbitan stearate, sorbitan oleate, sucrose distearate, sucrose polystearate, PEG-7-hydrogenated castor oil, cetyl alcohol, stearyl alcohol, cetearyl alcohol, behenyl alcohol, isobehenyl alcohol, cetearyl glucoside, steareth-2, and combinations of two or more thereof.

Component D

The cosmetic composition of the present invention further comprises water. Water is useful for economic and ecological reasons. As used herein, water may be demineralized water, deionized water, distilled water, mineral water, or tap water, preferably demineralized water.

In at least one preferred embodiment, the cosmetic composition of the present invention comprises from 10 to 99 wt.-%, or from 10 to 98.5 wt.-%, or from 10 to 98 wt.-%, or from 10 to 97.8 wt.-%, or from 10 to 97.5 wt.-%, or from 10 to 97 wt.-%, or from 10 to 95 wt.-%, or from 20 to 94 wt.-%, or from 30 to 93 wt.-%, or from 40 to 92 wt.-%, or from 50 to 91 wt.-%, or from 60 to 90 wt.-%, or from 70 to 90 wt.-% of water, based on the total weight of the composition.

Component E

The cosmetic composition of the present invention may optionally comprise one or more further components.

In at least one preferred embodiment, the cosmetic composition further comprises at least one further cosmetically acceptable component or mixture of cosmetically acceptable components, wherein the cosmetically acceptable component or mixture of cosmetically acceptable components is selected from the group consisting of further emollients, waxes, surfactants, cationic polymers, film formers, superfatting agents, refatting agents, foam stabilizers, stabilizers, active biogenic substances, preservatives, preservation boosting ingredients, anti-fungal substance, anti-dandruff agents, dyes or pigments, particulate substances, opacifiers, abrasives, absorbents, anticaking agents, bulking agents, pearlizing agents, direct dyes, perfumes or fragrances, carriers, solvents or diluents, propellants, functional acids, active ingredients, skin-brightening agents, self-tanning agents, exfoliants, enzymes, anti-acne agents, deodorants and anti-perspirants, viscosity modifiers, thickening and gelling agents, pH adjusting agents, buffering agents, anti-oxidants, chelants, astringents, sunscreens, sun protection agents, UV filters, conditioning agents, humectants, occlusive agents, pediculocides, anti-foaming agents, flavouring agents, electrolytes, oxidizing agents and reducing agents.

These optional further components may be understood in the broadest sense as generally understood in the art. Advantageously, the optional further components are cosmetically acceptable. Non-limiting examples of such optional further components are given in the following:

Solvent

The cosmetic composition of the present invention may optionally comprise one or more solvents. Preferred solvents are alcohols. Also preferred solvents are mixtures of alcohols. More preferred solvents are glycerin, ethanol, propanol, isopropanol, ethylene glycol, propylene glycol, or mixtures thereof. Even more preferred solvents are glycerin, ethanol, propylene glycol, or mixtures thereof. A particularly preferred solvent is ethanol. Advantageously, the solvents are cosmetically acceptable. In at least one preferred embodiment, the solvent is an alcohol (in particular ethanol) or a mixture of two or more alcohols.

Auxiliary

In at least one preferred embodiment, the composition comprises at least one additive common in the field of cosmetics, which are hereinafter called auxiliaries. In at least one preferred embodiment, the composition comprises an auxiliary. In at least one preferred embodiment, the auxiliary is cosmetically acceptable. In at least one preferred embodiment, the auxiliary is selected from the group consisting of waxes, surfactants, cationic polymers, film formers, superfatting agents, refatting agents, foam stabilizers, stabilizers, active biogenic substances, preservatives, preservation boosting ingredients, anti-fungal substance, anti-dandruff agents, dyes or pigments, particulate substances, opacifiers, abrasives, absorbents, anticaking agents, bulking agents, pearlizing agents, direct dyes, perfumes or fragrances, carriers, solvents or diluents, propellants, functional acids, active ingredients, skin-brightening agents, self-tanning agents, exfoliants, enzymes, anti-acne agents, deodorants and anti-perspirants, viscosity modifiers, thickening and gelling agents, pH adjusting agents, buffering agents, anti-oxidants, chelants, astringents, sunscreens, sun protection agents, UV filters, conditioning agents (for example hair conditioning agents or skin conditioning agents), further emollients, humectants, occlusive agents, pediculocides, anti-foam ing agents, flavouring agents, electrolytes, oxidizing agents and reducing agents. In at least one preferred embodiment, the cosmetically acceptable component is an auxiliary or mixture of auxiliaries.

In at least one preferred embodiment, the wax is selected from the group consisting of carnauba wax, beeswax, candelilla wax, synthetic wax, polyethylene, paraffin wax, microcrystalline wax, hydrogenated vegetable oil, hydrogenated castor oil, rice bran wax, cetyl dimethicone, bis-PEG-18 methyl ether dimethyl silane, and combinations thereof. In at least one preferred embodiment, the composition comprises from 0.001 wt% to 30 wt%, preferably from 0.05 wt% to 20 wt%, even more preferably from 0.1 wt% to 10 wt% of at least one wax.

In at least one preferred embodiment, the composition comprises a cationic polymer. Suitable cationic polymers include those known under the INCI designation "polyquaternium", especially polyquaternium-31, polyquaternium-16, polyquaternium-24, Polyquaternium-7, polyquaternium-22, polyquaternium-39, polyquaternium-28, polyquaternium-2, polyquaternium-10, polyquaternium-11, and also polyquaternium-37 & mineral oil & PPG trideceth (Salcare SC95), PVP-dimethylaminoethyl methacrylate copolymer, guar-hydroxypropyltriammonium chlorides, and also calcium alginate and ammonium alginate. It is additionally possible to employ cationic cellulose derivatives; cationic starch; copolymers of diallylammonium salts and acrylamides; quaternized vinylpyrrolidone/vinylimidazole polymers; condensation products of polyglycols and amines; quaternized collagen polypeptides; quaternized wheat polypeptides; polyethyleneimines; cationic silicone polymers, such as amidomethicones, for example; copolymers of adipic acid and dimethylaminohydroxypropyldiethylenetriamine; polyaminopolyamide and cationic chitin derivatives, such as chitosan, for example.

In at least one preferred embodiment, the cationic polymer is selected from the group consisting of polyquaternium-10, guar hydroxypropyltrimonium chloride, polyquaternium-7, polyquaternium-6, and combinations thereof.

In at least one preferred embodiment, the composition comprises from 0.1 wt% to 10 wt%, preferably from 0.5 wt% to 7.5 wt%, even more preferably from 1.0 wt% to 5.0 wt% of at least one cationic polymer.

In at least one preferred embodiment, the composition comprises a film former. Film formers are materials which produce a continuous film on skin, hair, or nails such as synthetic or natural polymers and their derivatives. The compositions according to the invention can contain film formers, which are, depending on the intended use, selected from salts of phenylbenzimidazole sulfonic acid, water-soluble polyurethanes, for example $C_{10}$-polycarbamyl polyglyceryl ester, polyvinyl alcohol, polyvinylpyrrolidone (PVP) copolymers, vinylpyrrolidone/vinyl acetate copolymer or PVP/eicosene copolymers, vinylpyrrolidone /alkene copolymers, for example VP/eicosene copolymer or VP/hexadecene copolymer, PVM/MA copolymer or esters thereof, maleinized polypropylene polymers, water-soluble acrylic acid polymers/copolymers or esters or salts thereof, for example partial-ester copolymers of acrylic/methacrylic acid, polyalkylsilsesquioxanes, polyacrylamide, water-soluble cellulose, for example hydroxymethylcellulose, hydroxyethylcellulose, hydroxypropylcellulose, water-soluble quaterniums, polyquaterniums, carboxyvinyl polymers, such as carbomers and salts thereof, polysaccharides, for example polydextrose and glucan, vinyl acetate/crotonate. In at least one preferred embodiment, the film former is selected from the group consisting of VP/eicosene copolymer, PVP, VP/VA copolymer, styrene/acrylates copolymer, acrylates copolymer, butyl ester of PVM/MA copolymers, hydroxyethylcellulose, polyquaternium-10, polypropylsilsesquioxane, polyurethane-64, and combinations thereof.

In at least one preferred embodiment, the composition comprises from 0.1 wt% to 10 wt%, preferably from 0.5 wt% to 7.5 wt%, even more preferably from 1.0 wt% to 5.0 wt% of at least one film former.

In at least one preferred embodiment, the composition comprises a superfatting agent and/or a refatting agent. For example, lanolin, polyethoxylated lanolin derivatives, lecithin, lecithin derivatives, non-ethoxylated and polyethoxylated or acylated lanolin and lecithin derivatives, polyol fatty acid esters such as glyceryl oleate, mono-, di- and triglycerides and/or fatty acid alkanolamides can preferably be used as superfatting agents or refatting agents. These compounds can also simultaneously serve as foam stabilizers. In at least one preferred embodiment, the superfatting agent and/or refatting agent is selected from the group consisting of, lanolin, glyceryl ricinoleate, PEG-8 glyceryl laurate, glyceryl oleate, cocamide MEA, PEG-75 lanolin, and combinations thereof. In at least one preferred embodiment, the composition comprises from 0.01 wt% to 10 wt%, preferably from 0.1 wt% to 5.0 wt%, even more preferably from 0.5 wt% to 3.0 wt% of at least one superfatting agent and/or a refatting agent.

In at least one preferred embodiment, the composition comprises a stabiliser. For example, metal salts of fatty acids, such as magnesium, aluminum and/or zinc stearate can preferably be used as stabilizers. In at least one preferred embodiment, the stabilizer is selected from the group consisting of aluminum stearate, aluminum isostearates/myristates, magnesium stearate, magnesium cocoate, zinc palmitate, zinc stearate, and combinations thereof. In at least one preferred embodiment, the composition comprises from 0.01 wt% to 10 wt%, preferably from 0.5 wt% to 8.0 wt%, even more preferably from 1.0 wt% to 5.0 wt% of at least one stabilizer.

In at least one preferred embodiment, the composition comprises a biogenic substance. In at least one preferred embodiment, the composition comprises a biogenic substance, wherein such substances are selected from plant extracts (e.g. leaf, root, seed, flower and/or stem extracts from aloe vera, camomile, green tea, hamamelis or licorice), local anesthetics, antibiotics, antiphlogistics, antiallergic agents, hormones, beta-glucans, cholesterol, amino acids, ceramides, corticosteroids, sebostatics, Bisabolol allantoin, Phytantriol, proteins, vitamins selected from niacin, biotin, vitamin B2, vitamin B3, vitamin B6, vitamin B5, vitamin B3 derivatives (salts, acids, esters, amides, alcohols), vitamin C and vitamin C derivatives (salts, acids, esters, amides, alcohols), preferably as sodium salt of the monophosphoric acid ester of ascorbic acid or as magnesium salt of the phosphoric acid ester of ascorbic acid, tocopherol and tocopherol acetate, vitamin E and/or its derivatives, protein derivatives such as gelatin, collagen hydrolysates, polypeptides, egg yolk, lecithin, hydrolyzed silk, hydrolyzed keratin, milk protein, cerebrosides or phospholipids. In at least one preferred embodiment, the biogenic active substance is selected from the group consisting of, aloe vera extract, collagen hydrolysates, bisabolol, vitamin C, vitamin E, allantoin, vitamin B5, tocopherol acetate, retinyl palmitate, and combinations thereof. In at least one preferred embodiment, the composition comprises from 0.001 wt% to 5.0 wt%, preferably from 0.01 wt% to 3.0 wt%,

even more preferably from 0.1 wt% to 2.0 wt% of at least one biogenic active substance.

In at least one preferred embodiment, the composition comprises a preservative, preservation boosting ingredient, anti-fungal agent, and/or anti-dandruff agent. In at least one preferred embodiment, the preservative is selected from the group consisting of benzyl alcohol, piroctone olamine, phenoxyethanol, parabens, pentanediol, benzoic acid/sodium benzoate, sorbic acid/potassium sorbate, and combinations thereof. Other organic acids can also be used to provide antimicrobial protection. In at least one preferred embodiment, the preservation boosting ingredient is selected from the group consisting of anisic acid, lactic acid, sorbitan caprylate, ethylhexylglycerin, caprylyl glycol, octanediol, and mixtures thereof. A suitable preservation boosting ingredient is also disclosed in European patent application 16176830.4 filed on 29th June 2016 by Clariant International Ltd (see in particular claim 1 therein), which is incorporated herein by reference. In at least one preferred embodiment, the composition comprises 0.01 to 5.0 wt%, particularly preferably from 0.05 wt% to 1.0 wt% of at least one preservative. Suitable preservatives include the substances listed in the International Cosmetic Ingredient Dictionary and Handbook, 9th Edition with the function "preservatives". In at least one preferred embodiment, the preservative is selected from the group consisting of phenoxyethanol, benzyl paraben, butyl paraben, ethyl paraben, isobutyl paraben, isopropyl paraben, methyl paraben, propyl paraben, iodopropynyl butylcarbamate, methyldibromoglutaronitrile, DMDM hydantoin and combinations thereof. In at least one preferred embodiment, the composition comprises a preservative selected from the group consisting of cetyltrimethyl ammonium chloride, cetylpyridinium chloride, benzethonium chloride, diisobutylethoxyethyldimethyl benzylammonium chloride, sodium N-lauryl sarcosinate, sodium-N-palmethyl sarcosinate, lauroyl sarcosine, N-myristoylglycine, potassium-N-laurylsarcosine, trimethylammonium chloride, sodium aluminium chlorohydroxylactate, triethylcitrate, tricetylmethylammonium chloride, 2,4,4'-trichloro-2'-hydroxydiphenylether (Triclosan), phenoxyethanol, 1,5-pentandiol, 1,6-hexandiol, 3,4,4'-trichlorocarbanilide (Triclocarban), diaminoalkylamide, L-lysine hexadecylamide, heavy metal citrate salts, salicylate, piroctose, zinc salts, pyrithione and its heavy metal salts, zinc pyrithione, zinc phenol sulfate, farnesol, ketoconazol, oxiconazol, bifonazole, butoconazole, cloconazole, clotrimazole, econazole, enilconazole, fenticonazole, isoconazole, miconazole, sulconazole, tioconazole, fluconazole, itraconazole, terconazole, naftifine, terbinafine, selenium disulfide, piroctone olamine (Octopirox), methylchloroisothiazolinone, methylisothiazolinone, methyldibromo glutaronitrile, silver chloride (AgCl), diazolidinyl urea, imidazolidinyl urea, dehydroacetic acid, undecylenic acid, chlorphenesin, proprionic acid, salicylic acid, chloroxylenol, sodium salts of diethylhexylsulfosuccinate, sodiumbenzoate, phenoxyethanol, (*RS*)-1-(4-chlorophenoxy)-1-imidazol-1-yl-3,3-dimethylbutan-2-one (climbazole), benzyl alcohol, phenoxyisopropanol, parabens such as butyl-, ethyl-, methyl- and propylparaben and their salts, 2-bromo-2-nitropropane-1,3-diol, polyaminopropyl biguanide, phenoxyisopropanol, iodopropynyl butylcarbamate, benzalkonium chloride, benzethonium chloride, pentandiol, 1,2-octanediol, ethylhexylglycerin, sorbic acid, benzoic acid, lactic acid, imidazolidinyl urea, diazolidinyl urea, dimethylol dimethyl hydantoin (DMDMH), chlorhexidine, sodium salts of hydroxymethyl glycinate, hydroxyethylglycine of sorbic acid, and combinations thereof. In at least one preferred embodiment, the preservative is selected from the group consisting of phenoxyethanol, benzyl paraben, butyl paraben, ethyl paraben, isobutyl paraben, isopropyl paraben, methyl paraben, propyl paraben, iodopropynyl butylcarbamate, methyldibromoglutaronitrile, DMDM hydantoin and combinations thereof. In at least one preferred embodiment, the composition is substantially free of parabens.

In at least one preferred embodiment, the composition comprises from 0.1 wt% to 5.0 wt% antimicrobial agents. In at least one preferred embodiment, the antimicrobial agent is chlorhexidine.

In at least one preferred embodiment, the composition comprises an anti-fungal substance. In at least one preferred embodiment, the anti-fungal substance is selected from the group consisting of ketoconazole, oxiconazole, bifonazole, butoconazole, cloconazole, clotrimazole, econazole, enilconazole, fenticonazole, isoconazole, miconazole, sulconazole, tioconazole, fluconazole, itraconazole, terconazole, naftifine, terbinafine, zinc pyrithione, piroctone olamine (Octopirox), (RS)-1-(4-chlorophenoxy)-1-imidazol-1-yl-3,3-dimethylbutan-2-one (climbazole), and combinations thereof. In at least one preferred embodiment, the composition comprises a total amount of anti-fungal substance in the composition of from 0.1 wt% to 1.0 wt%. In at least one preferred embodiment, the composition comprises pyridinethione anti-dandruff particulates, for example 1-hydroxy-2-pyridinethione salts, are highly preferred particulate anti-dandruff agents. The concentration of pyridinethione anti-dandruff particulate may ranges from 0.1% to 4.0%, by weight of the composition, preferably from 0.1% to 3.0%, more preferably from 0.3% to 2.0%. Preferred pyridinethione salts include those formed from heavy metals such as zinc, tin, cadmium, magnesium, aluminum and zirconium, preferably zinc, more preferably the zinc salt of 1-hydroxy-2-pyridinethione (known as "zinc pyridinethione" or "ZPT"), more preferably 1-hydroxy-2-pyridinethione salts in platelet particle form. Salts formed from other cations, such as sodium, may also be suitable. Pyridinethione anti-dandruff agents are described, for example, in US patents US 2,809,971; US 3,236,733; US 3,753,196; US 3,761,418; US 4,345,080; US 4,323,683; US 4,379,753; and US 4,470,982. It is contemplated that when ZPT is used as the anti-dandruff particulate in the compositions herein, the growth or regrowth of hair may be stimulated or regulated, or both, or that hair loss may be reduced or inhibited, or that hair may appear thicker or fuller.

In at least one preferred embodiment, the composition comprises a preservative system comprising a plurality of different compounds selected from the group consisting of preservatives, preservation boosting ingredients, anti-fungal agents and anti-dandruff agents.

In at least one preferred embodiment, the composition comprises a dye or pigment. In at least one preferred embodiment, the composition comprises at least one dye or pigment. Suitable dyes and pigments are disclosed in WO 2013/017262A1 in the table spanning pages 36 to 43. These may be colored pigments which impart color effects to the product mass or to hair or skin, or they may be luster effect pigments which impart luster effects to the product mass or to the hair or skin. The color or luster effects on the hair or skin are preferably temporary, i.e. they last until the next hair or skin wash and can be removed again by washing the hair or skin with customary shampoos, body or face cleansers, body washes etc. or by using a make-up remover, micellar water or cleansing wipes. In at least one preferred embodiment, the composition comprises a total amount of from 0.01 wt% to 25 wt%, preferably from 0.1 wt% to 15 wt%, even more preferably from 0.5 wt% to 10 wt% of at least one pigment. In at least one preferred embodiment, the particle size of the pigment is from 1 micron to 200 micron, preferably from 3 micron to 150 micron, more preferably 10 micron to 100 micron. The pigments are colorants which are virtually insoluble in the application medium, and may be inorganic or organic. Inorganic-organic mixed pigments are also possible. Preference is given to inorganic pigments. The advantage of inorganic pigments is their excellent resistance to light, weather and temperature. The inorganic pigments may be of natural origin. In at least one preferred embodiment, the inorganic pigment is selected from the group consisting of chalk, ochre, umber, green earth, burnt sienna, graphite, and combinations thereof. The pigments may be white pigments, such as, for example, titanium dioxide or zinc oxide, black pigments, such as, for example, iron oxide black, colored pigments, such as, for example, ultramarine or iron oxide red, luster pigments, metal effect pigments, pearlescent pigments, and fluorescent or phosphorescent pigments, where preferably at least one pigment is a colored, nonwhite pigment. In at least one preferred embodiment, the pigment is selected from the group consisting of metal oxides, hydroxides and oxide hydrates, mixed phase pigments, sulfur-containing silicates, metal sulfides, complex metal cyanides, metal sulfates, chromates and molybdates, and the metals themselves (bronze, silver, gold pigments), and combinations thereof. In at least one preferred embodiment, the pigment is selected from the group consisting of titanium dioxide (CI 77891), black iron oxide (CI 77499), yellow iron oxide (CI 77492), red and brown iron oxide (CI 77491), manganese violet (CI 77742), ultramarine (sodium aluminum sulfosilicates, CI 77007, Pigment Blue 29), chromium oxide hydrate (CI 77289), Prussian blue (ferric ferrocyanide, CI 77510), carmine (cochineal), and combinations thereof. In at least one preferred embodiment, the pigment is selected from the group consisting of pearlescent and colored pigments which consist of either single crystals like mica, silica, bismuth oxychloride, boron nitride or titanium dioxide or which have a layer-substrate structure based on mica, aluminium, aluminium oxide, titanium dioxide, silicium dioxide, silicates (e.g. calcium aluminium borosilicate, calcium sodium borosilicate, magnesium aluminium silicate or sodium magnesium fluorosilicate) which are coated with a metal oxide (e.g. iron oxide, chromium oxide, tin oxide) or a metal oxychloride, such as titanium dioxide or bismuth oxychloride, or a metal hydroxide, such as aluminum hydroxide, and optionally further color-imparting substances, such as Prussian blue, ultramarine, carmine or other organic dyes and where the color can be determined by varying the layer thickness. Such pigments are sold, for example, under the trade names RonaFlair, Colorona, Xirona, and Timiron by Merck or under the trade name Flamenco, Timica and Cloisonne by BASF, Germany. The pearlescent effect can be controlled both by means of the particle size and by means of the particle size distribution of the pigment population. Suitable particle size distributions are e.g. in the range 2-50 μm, 5-25 μm, 5-40 μm, 5-60 μm, 5-95 μm, 5-100 μm, 10-60 μm, 10-100 μm, 10-125 μm, 20-100 μm, 20-150 μm, and < 15 μm. A wider particle size distribution e.g. of 20-150 μm, produces glittering effects, whereas a narrower particle size distribution of < 15 μm gives a uniform silky appearance. In at least one preferred embodiment, the pigment is selected from the group consisting of titanium dioxide (CI 77891), black iron oxide (CI 77499), yellow iron oxide (CI 77492), red and brown iron oxide (CI 77491), mica, silica, bismuth oxychloride, and combinations thereof. In at least one preferred embodiment, the pigment is selected from the group consisting of organic pigments such as sepia, gamboge, bone charcoal, Cassel brown, indigo, chlorophyll and other plant pigments. In at least one preferred embodiment, the pigment is selected from the group consisting of synthetic organic pigments such as azo pigments, anthraquinoids, indigoids, dioxazine, quinacridone, phthalocyanine, isoindolinone, perylene and perinone, metal complex, alkali blue and diketopyrrolopyrrole pigments.

In at least one preferred embodiment, the composition comprises a particulate substance. In at least one preferred embodiment, the composition comprises at least one particulate substance. Suitable substances are, for example, substances which are solid at room temperature (25°C) and are in the form of particles. Suitable substances are, for example, substances which serve as opacifiers, abrasives, absorbents, anti-caking agents, bulking agents or performance fillers. In at least one preferred embodiment, the particulate substance is selected from the group consisting of silica, silicates (e.g. sepiolite, montmorillonite, bentonite, kaolin, hectorite), aluminates, clay earths, mica, talc, starch, perlite, charcoal, pulp powder, seed powder, insoluble salts, in particular insoluble inorganic metal salts, metal oxides (e.g. titanium dioxide), minerals and insoluble polymer particles, such as polyamide derivatives (e.g. nylon-12, nylon-6, polyamide-5), silicones (e.g. polymethylsilsesquioxane), polyesters (e.g. polyester-12), polyethylene and polymethyl methacrylates. The particles are present in the composition in undissolved, preferably stably dispersed form, and, following application to the keratin substrate and evaporation of the solvent, can deposit on the substrate in solid form. A stable dispersion can be achieved by providing the composition with a yield point which is large enough to prevent the

solid particles from sinking. An adequate yield point can be established using suitable gel formers in a suitable amount. In at least one preferred embodiment, the particulate substance is selected from the group consisting of silica (silica gel, silicon dioxide) and metal salts, in particular inorganic metal salts, where silica is particularly preferred. Metal salts are, for example, alkali metal or alkaline earth metal halides, such as sodium chloride or potassium chloride; alkali metal or alkaline earth metal sulfates, such as sodium sulfate or magnesium sulfate. In at least one preferred embodiment, the particulate substance is selected from the group consisting of, silica, mica, bentonite, kaolin, talc, polymethylsilsesquioxane, polyethylene, clay, and combinations thereof. In at least one preferred embodiment, the composition comprises from 0.01 wt% to 20 wt%, preferably from 0.05 wt% to 15 wt%, even more preferably from 0.5 wt% to 10 wt% of at least one particulate substance.

In at least one preferred embodiment, the composition comprises pearlizing agents. In at least one preferred embodiment, the composition comprises at least one pearlizing agent. In at least one preferred embodiment, the pearlizing agent is selected from the group consisting of, fatty acid monoalkanolamides, fatty acid dialkanolamides, monoesters or diesters of alkylene glycols, especially ethylene glycol and/or propylene glycol or oligomers thereof, with higher fatty acids, e.g. palmitic acid, stearic acid and behenic acid, monoesters or polyesters of glycerol with carboxylic acids, fatty acids and metal salts thereof, ketosulfones or mixtures of the aforementioned compounds. In at least one preferred embodiment, the pearlizing agent is selected from the group consisting of, ethylene glycol distearates and/or polyethylene glycol distearates with 3 glycol units on average, and combinations thereof. In at least one preferred embodiment, the composition comprises from 0.1 wt% to 15 wt%, preferably from 0.5 wt% to 10 wt%, even more preferably from 1.0 wt% to 5.0 wt% of at least one pearlizing agent.

In at least one preferred embodiment, the composition comprises a direct dye. In at least one preferred embodiment, the composition comprises at least one direct dye. Preferred among the direct dyes are the following compounds, alone or in combination with one another: hydroxyethyl-2-nitro-p-toluidine, 2-hydroxyethylpicramic acid, 4-nitrophenylaminourea, tri(4-amino-3-methylphenyl)carbenium chloride (Basic Violet 2), 1,4-di-amino-9,10-anthracenedione (Disperse Violet 1), 1-(2-hydroxy-ethyl)amino-2-nitro-4-[di(2-hydroxyethyl)amino]benzene (HC Blue No. 2), 4-[ethyl-(2-hydroxyethyl)amino]-1-[(2-hydroxyethyl)amino]-2-nitrobenzene hydrochloride (HC Blue No. 12), 1-amino-4-[di(2-hydroxyethyl)amino]-2-nitrobenzene hydrochloride (HC Red No. 13), 4-amino-1-[(2-hydroxyethyl)amino]-2-nitrobenzene (HC Red No. 3), 4-amino-3-nitrophenol, 4-[(2-hydroxyethyl)amino]-3-nitrophenol, 1-amino-5-chloro-4-[(2,3-dihydroxypropyl)amino]-2-nitrobenzene (HC Red No. 10), 5-chloro-1,4-[di(2,3-dihydroxypropyl)amino]-2-nitrobenzene (HC Red No. 11), 2-chloro-6-ethylamino-4-nitrophenol, 2-amino-6-chloro-4-nitrophenol, 4-[(2-hydroxyethyl)amino]-3-nitro-1-trifluoromethylbenzene (HC Yellow No. 13), 8-amino-2-bromo-5-hydroxy-4-imino-6-{[3-(trimethylammonio)-phenyl]amino}-1(4H)-naphthalenone chloride (C.I. 56059; Basic Blue No. 99), 1-[(4-aminophenyl)azo]-7-(trimethylammonio)-2-naphthol chloride (C.I. 12250; Basic Brown No. 16), 1-[(4-amino-2-nitrophenyl)azo]-7-(trimethylammonio)-2-naphthol chloride (Basic Brown No. 17), 2-hydroxy-1-[(2-methoxyphenyl)azo]-7-(trimethylammonio)naphthalene chloride (C.I. 12245; Basic Red No. 76), 3-methyl-1-phenyl-4-{[3-(trimethylammonio)phenyl]azo}pyrazol-5-one chloride (C.I. 12719; Basic Yellow No. 57) and 2,6-diamino-3-[(pyridin-3-yl)azo]pyridine as well as the salts thereof. Particularly preferred among the aforesaid direct dyes are the following compounds, alone or in combination with one another: hydroxyethyl-2-nitro-p-toluidine, 2-hydroxyethylpicramic acid, 4-nitrophenylaminourea, tri(4-amino-3-methylphenyl)carbenium chloride (Basic Violet 2), 1,4-di-amino-9,10-anthracenedione (Disperse Violet 1), 1-(2-hydroxy-ethyl)amino-2-nitro-4-[di(2-hydro-xyethyl)amino]benzene (HC Blue No. 2), 4-[ethyl-(2-hydroxyethyl)amino]-1-[(2-hydroxyethyl)amino]-2-nitrobenzene hydrochloride (HC Blue No. 12), 1-amino-4-[di(2-hydroxyethyl)amino]-2-nitrobenzene hydrochloride (HC Red No. 13), 4-amino-1-[(2-hydroxyethyl)amino]-2-nitrobenzene (HC Red No. 3), 4-amino-3-nitrophenol, 4-[(2-hydroxyethyl)amino]-3-nitrophenol, 1-amino-5-chloro-4-[(2,3-dihydroxypropyl)amino]-2-nitrobenzene (HC Red No. 10), 5-chloro-1,4-[di(2,3-dihydroxypropyl)- amino]-2-nitrobenzene (HC Red No. 11), 2-chloro-6-ethylamino-4-nitrophenol, 2-amino-6-chloro-4-nitrophenol, 4-[(2-hydroxyethyl)amino]-3-nitro-1-trifluoromethylbenzene (HC Yellow No. 13), 8-amino-2-bromo-5-hydroxy-4-imino-6-{[3-(trimethylammonio)-phenyl]amino}-1(4H)-naphthalenone chloride (C.I. 56059; Basic Blue No. 99), 1-[(4-aminophenyl)azo]-7-(trimethylammonio)-2-naphthol chloride (C.I. 12250; Basic Brown No. 16), 1-[(4-amino-2-nitrophenyl)azo]-7-(trimethylammonio)-2-naphthol chloride (Basic Brown No. 17), 2-hydroxy-1-[(2-methoxyphenyl)azo]-7-(trimethylammonio)naphthalene chloride (C.I. 12245; Basic Red No. 76), 3-methyl-1-phenyl-4-{[3-(trimethylammonio)phenyl]azo}pyrazol-5-one chloride (C.I. 12719; Basic Yellow No. 57) and 2,6-diamino-3-[(pyridin-3-yl)azo]pyridine as well as the salts thereof.

In at least one preferred embodiment, the composition comprises from 0.1 wt% to 15 wt%, preferably from 0.1 wt% to 10 wt%, even more preferably from 0.5 wt% to 8.0 wt% of at least one direct dye.

In at least one preferred embodiment, the composition comprises a perfume or fragrance ingredient. Individual fragrance compounds, e.g. the synthetic products of the type of esters, ethers, aldehydes, ketones, alcohols and hydrocarbons can be used as fragrance or perfume oils. Fragrance compounds of the ester type are e.g. benzyl acetate, phenoxyethyl isobutyrate, p-tert-butylcyclohexyl acetate, linalyl acetate, glyceryl rosinate, dimethylbenzylcarbinyl acetate, phenylethyl acetate, linalyl benzoate, benzyl formate, ethyl-methylphenyl glycinate, allylcyclohexyl propionate, styrallyl propionate and benzyl salicylate. The ethers include for example benzyl ethyl ether, the aldehydes include e.g.

the linear alkanals with 8 to 18 carbon atoms, citral, citronellal, citronellyloxyacetaldehyde, cyclamen aldehyde, hydroxycitronellal, lilial and bourgeonal, the ketones include e.g. the ionones, alpha-isomethylionone and methyl-cedryl ketone, the alcohols include anethole, citronellol, eugenol, geraniol, linalool, phenylethyl alcohol and terpineol, and the hydrocarbons include mainly the terpenes and balsams. Preferably, mixtures of various fragrances are used, which together produce an attractive perfume note. Perfume oils can also contain mixtures of natural odoriferous substances that can be obtained from vegetable or animal sources, e.g. pine oil, citrus oil, jasmine oil, lily oil, rose oil, or ylang-ylang oil. Essential oils of lower volatility, which are used mostly as flavor components, are also suitable as perfume oils, e.g. sage oil, chamomile oil, clove oil, melissa oil, mint oil, cinnamon leaf oil, linden flower oil, juniper berry oil, vetiver oil, olibanum oil, galbanum oil and labdanum oil. In at least one preferred embodiment, the composition comprises from 0.01 wt% to 3.0 wt%, preferably from 0.05 wt% to 2.0 wt%, even more preferably from 0.1 wt% to 1.0 wt% of at least one perfume or fragrance ingredient.

In at least one preferred embodiment, the composition comprises a carrier, solvent or diluent. In at least one preferred embodiment, the composition comprises a solvent, wherein the solvent comprises optionally in addition to water at least one alcohol. In at least one preferred embodiment, the solvent is cosmetically acceptable. Optionally the composition comprises water-miscible or water-soluble solvents such as lower alkyl alcohols. In at least one preferred embodiment, the composition comprises $C_1$-$C_5$ alkyl monohydric alcohols, preferably $C_2$-$C_3$ alkyl alcohols. The alcohols which may be present are in particular lower monohydric or polyhydric alcohols having 1 to 4 carbon atoms customarily used for cosmetic purposes, such as preferably ethanol and isopropanol. Optionally, the composition comprises a water-soluble polyhydric alcohol. In at least one preferred embodiment, the water-soluble polyhydric alcohols are polyhydric alcohols having two or more hydroxyl groups in the molecule. In at least one preferred embodiment, the water-soluble polyhydric alcohol is selected from the group consisting of: dihydric alcohols such as ethylene glycol, propylene glycol, trimethylene glycol, 1,2-butylene glycol, 1,3-butylene glycol, 1,4-butylene glycol, tetramethylene glycol, 2,3-butylene glycol, pentamethylene glycol, 2-butene-1,4-diol, hexylene glycol, octylene glycol; trihydric alcohols such as glycerine, trimethylol propane, 1,2,6-hexanetriol and the like; tetrahydric alcohols such as penthaerythritol; polyhydric alcohol polymers such as diethylene glycol, dipropylene glycol, polyethylene glycol, polypropylene glycol, tetraethylene glycol, diglycerine, polyethylene glycol, triglycerine, tetraglycerine, polyglycerine; dihydric alcohol alkyl ethers such as ethylene glycol monomethyl ether, ethylene glycol monoethyl ether, ethylene glycol monobutyl ether, ethylene glycol monophenyl ether, ethylene glycol monohexyl ether, ethylene glycol mono-2-methylhexyl ether, ethylene glycol isoamyl ether, ethylene glycol benzyl ether, ethylene glycol isopropyl ether, ethylene glycol dimethyl ether, ethylene glycol diethyl ether, ethylene glycol dibutyl ether; dihydric alcohol alkyl ethers such as diethylene glycol monomethyl ether, diethylene glycol monoethyl ether, diethylene glycol monobutyl ether, diethylene glycol dimethyl ether, diethylene glycol diethyl ether, diethylene glycol butyl ether, diethylene glycol methyl ethyl ether, triethylene glycol monomethyl ether, triethylene glycol monoethyl ether, propylene glycol monomethyl ether, propylene glycol monoethyl ether, propylene glycol monobutyl ether, propylene glycol isopropyl ether, dipropylene glycol methyl ether, dipropylene glycol ethyl ether, dipropylene glycol butyl ether; dihydric alcohol ether esters such as ethylene glycol monomethyl ether acetate, ethylene glycol monoethyl ether acetate, ethylene glycol monobutyl ether acetate, ethylene glycol monophenyl ether acetate, ethylene glycol diadipate, ethylene glycol disuccinate, diethylene glycol monoethyl ether acetate, diethylene glycol monobutyl ether acetate, propylene glycol monomethyl ether acetate, propylene glycol monoethyl ether acetate, propylene glycol monopropyl ether acetate, propylene glycol monophenyl ether acetate; and mixtures thereof. In at least one preferred embodiment, the composition comprises a solvent selected from the group consisting of glycols, ethanol, and mixtures thereof. In at least one preferred embodiment, the composition comprises a solvent selected from the group consisting of ethanol, propanol, isopropanol, 1,2-propylene glycol, 1,3-propylene glycol, isobutanol, butanol, butyl glycol, butyl diglycol, glycerol, and mixtures thereof; preferably a solvent selected from the group consisting of ethanol, propanol, isopropanol, 1,2-propylene glycol, 1,3-propylene glycol, glycerol, and mixtures thereof; more preferably a solvent selected from the group consisting of isopropanol, 1,2-propylene glycol, 1,3-propylene glycol, and mixtures thereof. In at least one preferred embodiment, the composition comprises from 0.5 wt% to 80 wt%, preferably from 1.0 wt% to 75 wt%, more preferably from 5.0 wt% to 70 wt% of at least one carrier, solvent and/or diluent.

In at least one preferred embodiment, the composition comprises a propellant. In at least one preferred embodiment, the propellant is selected from compressed gas propellants and liquefied gas propellants. In at least one preferred embodiment, the compressed gas propellants are selected from the group consisting of air, nitrogen ($N_2$), nitrous oxide (N2O), carbon dioxide (CO2), and mixtures thereof; preferably air, nitrogen ($N_2$), and mixtures thereof; most preferably nitrogen (N2). In at least one preferred embodiment, the liquefied gas propellants are selected from the group consisting of dimethylether (DME), 1,1-difluoroethane (HFC-152a), 1,1,1,2-tetrafluoroethane (HFC-134a), pentane, n-butane, iso-butane, propane, trans-1,3,3,3-tetrafluoropropene (HF0-1234ze), and mixtures thereof, preferably dimethylether (DME), 1,1-difluoroethane (HFC-152a), and mixtures thereof.

In at least one preferred embodiment, the propellant is selected from the group consisting of nitrogen, carbon dioxide, pentane, n-butane, iso-butane, propane, and combinations thereof. In at least one preferred embodiment, the composition comprises from 0.5 wt% to 60 wt%, preferably from 1.0 wt% to 50 wt%, even more preferably from 2.0 wt%

to 40 wt% of at least one propellant.

In at least one preferred embodiment, the composition comprises a functional acid or an active ingredient. Functional acids and active ingredients are substances used to impart a clinical functionality to the skin or hair upon application. Functional acids and active ingredients are for example used as exfoliants, skin-brightening agents, self-tanning agents, anti-acne agents and anti-ageing agents. In another preferred embodiment, the compositions according to the invention contain one or more hydroxy acids, especially preferably one or more substances selected from alpha- and beta-hydroxy acids. The compositions according to the invention can contain, as hydroxy acids, preferably lactic acid, glycolic acid, salicylic acid and alkylated salicylic acids or citric acid. Furthermore, the compositions according to the invention can contain other acidic components. Consideration may be given to the following as active ingredient: tartaric acid, mandelic acid, caffeic acid, pyruvic acid, oligo-oxa mono- and dicarboxylic acids, fumaric acid, retinoic acid, sulfonic acids, benzoic acid, kojic acid, fruit acid, malic acid, gluconic acid, pyruvic acid, galacturonic acid, ribonic acid, hyaluronic acid, and all derivatives thereof, polyglycol diacids in free or partial neutralized form, vitamin C (ascorbic acid), vitamin C derivatives (e.g. sodium ascorbyl phosphate, magnesium ascorbyl phosphate and magnesium ascorbyl glucoside), dihydroxyacetone, minoxidil, proteolytic enzymes (e.g. fruit enzymes from papaya, pumpkin and pineapple such as papainase and bromelin ananase), caffeine, niacinamide and its derivatives, diethyl toluamide (DEET), or skin-whitening actives such as arbutin or glycyrrhetic acid and salts thereof, glutathione, cysteine, resveratrol, 4-butylresorcinol, or plant extracts like pancratium maritimum extract or mulberry extract. In at least one preferred embodiment, the functional acid and/or an active ingredient is selected from the group consisting of salicylic acid, kojic acid, hyaluronic acid, ascorbic acid, and all derivatives thereof, dihydroxyacetone, arbutin, and combinations thereof. In at least one preferred embodiment, the composition comprises from 0.05 wt% to 15 wt%, preferably from 0.1 wt% to 10 wt%, even more preferably from 0.5 wt% to 5.0 wt% of at least one functional acid and/or an active ingredient.

In at least one preferred embodiment, the composition comprises a deodorising agent or an antiperspirant. In at least one preferred embodiment, the composition comprises a deodorising agent. In at least one preferred embodiment, the deodorising agent is selected from the group consisting of allantoin, bisabolol, and combinations thereof. In at least one preferred embodiment, the composition comprises from 0.001 wt% to 10 wt%, or from 0.01 wt% to 9.0 wt%, or from 0.05 wt% to 8.0 wt%, or from 0.1 wt% to 5.0 wt% of at least one deodorising agent. The composition may comprise an antiperspirant. As antiperspirant it is possible to use aluminium chloride, aluminum chloride hydroxide, aluminum chloride dihydroxide, aluminum chlorohydrex polyethylene glycol complex, magnesium zirconium complexes or aluminum zirconium chloride hydroxide, for example. In at least one preferred embodiment, the composition comprises from 0.001 wt% to 10 wt%, or from 0.01 wt% to 9.0 wt%, or from 0.05 wt% to 8.0 wt%, or from 0.1 wt% to 5.0 wt% of at least one antiperspirant.

In at least one preferred embodiment, the composition comprises at least one viscosity modifier or thickening and/or gelling agent. The desired viscosity and rheology profile of the compositions can be adjusted by adding further thickeners and gelling agents. The viscosity-modifying substance is preferably a thickening polymer. In at least one preferred embodiment, the thickening polymer selected from the group consisting of: copolymers of at least one first monomer type, which is chosen from acrylic acid and methacrylic acid, and at least one second monomer type, which is chosen from esters of acrylic acid and ethoxylated fatty alcohol, crosslinked polyacrylic acid, crosslinked copolymers of at least one first monomer type, which is chosen from acrylic acid and methacrylic acid, and at least one second monomer type, which is chosen from esters of acrylic acid with $C_{10}$- to $C_{30}$-alcohols; copolymers of at least one first monomer type, which is chosen from acrylic acid and methacrylic acid, and at least one second monomer type, which is chosen from esters of itaconic acid and ethoxylated fatty alcohol; copolymers of at least one first monomer type, which is chosen from acrylic acid and methacrylic acid, at least one second monomer type, which is chosen from esters of itaconic acid and ethoxylated $C_{10}$- to $C_{30}$-alcohol and a third monomer type, chosen from $C_1$-to $C_4$-aminoalkyl acrylates; copolymers of two or more monomers chosen from acrylic acid, methacrylic acid, acrylic esters and methacrylic esters; hydroxyethylcellulose, hydroxypropylcellulose, hydroxypropylguar, glyceryl polyacrylate, glyceryl polymethacrylate, copolymers of at least one $C_2$-, $C_3$- or $C_4$-alkylene and styrene, polyurethanes, hydroxypropyl starch phosphate, polyacrylamide, copolymer of maleic anhydride and methyl vinyl ether crosslinked with decadiene, carob seed flour, gums such as guar gum, karaya gum, xanthan gum or dehydroxanthan gum, carrageenan, hydrolyzed corn starch; copolymers of polyethylene oxide, fatty alcohols and saturated methylenediphenyl diisocyanate (e.g. PEG-150/stearyl alcohol/SMDI copolymer), and mixtures thereof.

In at least one preferred embodiment, the viscosity modifier or thickening and/or gelling agent is selected from the group consisting of carbomers, acrylates copolymers, xanthan gum, hydroxyethylcellulose, laureth-2, and combinations thereof. In at least one preferred embodiment, the composition comprises from 0.01 wt% to 15 wt%, preferably from 0.1 wt% to 10 wt%, even more preferably from 0.5 wt% to 5.0 wt% of at least one viscosity modifier or thickening and/or gelling agent.

In at least one preferred embodiment, the composition comprises an alkalizing agent or pH adjusting agent. In at least one preferred embodiment, ammonia or caustic soda is suitable, but water-soluble, physiologically tolerable salts of organic and inorganic bases can also be considered. Optionally, the pH adjusting agent is selected from ammonium hydrogen carbonate, ammonia, monoethanolamine, ammonium hydroxide, ammonium carbonate. In at least one preferred embodiment, the alkalizing agents is selected from the group consisting of 2-amino-2-methyl-1-propanol, 2-amino-2-methyl-1,3-propanediol, 2-amino-2-ethyl-1,3-propanediol, tris(hydroxyl-methyl)-aminomethane, 2-amino-1-butanole, tris-(2-hydroxypropyl)-amine, 2,2-iminobisethanol, lysine, iminourea (guanidine carbonate), tetrahydro-1,4-oxazine, 2-amino-5-guanidin-valeric acid, 2-aminoethansulfonic acid, diethanolamine, triethanolamine, N-methyl ethanolamine, isopropanolamine, diisopropanolamine, triisopropanolamine, glucamine, sodium hydroxide, potassium hydroxide, lithium hydroxide and magnesium oxide, and mixtures thereof. To establish an acidic pH value, and acid can be included. In at least one preferred embodiment, the composition comprises an acid selected from the group consisting of hydrochloric acid, phosphoric acid, acetic acid, formic acid, sulfuric acid, hydrochloric acid, citric acid, ascorbic acid, and mixtures thereof. Citric acid is most preferred in that it has high consumer acceptance. In at least one preferred embodiment, the acidic pH is adjusted with a buffer, such as a phosphate buffer, a TRIS buffer or a citrate buffer. The buffers may be used alone or in combination with an acid. In at least one preferred embodiment, the alkalizing or pH adjusting agent is selected from the group consisting of triethanolamine, sodium hydroxide, lactic acid, citric acid, and combinations thereof. In at least one preferred embodiment, the composition comprises from 0.001 wt% to 5.0 wt%, preferably from 0.01 wt% to 3.0 wt%, even more preferably from 0.1 wt% to 1.0 wt% of at least one alkalizing or pH adjusting agent.

In at least one preferred embodiment, the composition comprises an anti-oxidant. In at least one preferred embodiment, the anti-oxidant is selected from the group consisting of amino acids, peptides, sugars, imidazoles, carotinoids, carotenes, chlorogenic acid, lipoic acid, thiols, thiol glycosyl esters, thiol N-acetyl esters, thiol methyl esters, thiol ethyl esters, thiol propyl esters, thiol amyl esters, thiol butyl esters, thiol lauryl esters, thiol palmitoyl esters, thiol oleyl esters, thiol linoleyl esters, thiol cholesteryl esters, thiol glyceryl esters, dilaurylthiodipropionate, distearylthiodipropionate, thiodipropionic acid, metal chelators, hydroxy acids, fatty acids, folic acids, vitamin C, tocopherol, vitamin A, stilbenes, derivatives and combinations thereof. In at least one preferred embodiment, the anti-oxidant is selected from the group consisting of glycine, histidine, tyrosine, tryptophan, urocaninic acid, D,L-carnosine, D-carnosine, L-carnosine, beta-carotene, alpha-carotene, lycopene, dihydrolipoic acid, aurothioglucose, propylthiouracil, thioredoxine, glutathione, cysteine, cystine, cystamine, buthioninsulfoximine, homocysteinsulfoximine, buthioninsulfone, penta-, hexa-, heptathioninsulfoximine, hydroxyfatty acids, palmitic acid, phytinic acid, lactoferrin, citric acid, lactic acid, malic acid, humic acid, bile acid, bilirubin, biliverdin, EDTA, EGTA, linoleic acid, linolenic acid, oleic acid, butylhydroxyanisol, trihydroxybutyrophenone, ubichinon, ubichinol, ascorbylpalmitate, Mg-ascorbylphosphate, ascorbylacetate, vitamin E acetate, vitamin A palmitate, carnosine, mannose, ZnO, $ZnSO_4$, selenium methionine, stilbenes, superoxide dismutase, and combinations thereof. In at least one preferred embodiment, the antioxidant is selected from the group consisting of vitamin A, vitamin A derivatives, vitamin E, vitamin E derivatives, and combinations thereof. In at least one preferred embodiment, the composition comprises from 0.001 wt% to 10 wt%, preferably from 0.05 wt% to 5.0 wt%, even more preferably from 0.1 wt% to 3.0 wt%, most preferably from 0.05 wt% to 1.0 wt% of at least one antioxidant.

In at least one preferred embodiment, the composition comprises a chelant. In at least one preferred embodiment, the chelant is selected from the group consisting of EDTA, caprylhydroxamic acid, oxalate derivatives, disodium hydroxyethyliminodiacetate, galacturonic acid and derivatives, glucuronic acid and derivatives, lauroyl ethylenediamine triacetic acid, methyl dihydroxybenzoate, trisodium ethylenediamine disuccinate, phytic acid, itaconic acid, propane tricarboxylic acid, citric acid and derivatives (e.g. diammonium citrate, bismuth citrate and acetyl trihexyl citrate 2,6-dicarboxy pyridine), phosphoric and phosphonic acid derivatives (e.g. diethylenetriamine pentamethylene phosphonic acid, disodium azacycloheptane diphosphonate, glycereth-26 phosphate, disodium pyrophosphate, disodium salicylphosphate, aminotrimethylene phosphonic acid, phosphonobutanetricarboxylic acid, potassium trisphosphonomethylamine oxidebeta-alanine diacetic acid or cyclohexanediamine tetraacetic acid). In at least one preferred embodiment, the chelant is selected from the group consisting EDTA, oxalate derivatives, disodium salicylphosphate, and combinations thereof. In at least one preferred embodiment, the composition comprises from 0.01 wt% to 2.0 wt%, preferably from 0.05 wt% to 1.5 wt%, even more preferably from 0.1 wt% to 1.0 wt%, most preferably from 0.05 wt% to 1.0 wt% of at least one chelant.

In at least one preferred embodiment, the composition comprises an astringent. In at least one preferred embodiment, the astringent is selected from the group consisting of magnesium oxide, aluminium oxide, titanium dioxide, zirconium dioxide, zinc oxide, oxide hydrates, aluminium oxide hydrate (boehmite) and hydroxide, chlorohydrates of calcium, magnesium, aluminium, titanium, zirconium or zinc. In at least one preferred embodiment, the composition comprises from 0.001 wt% to 10 wt%, or from 0.01 wt% to 9.0 wt%, or from 0.05 wt% to 8.0 wt%, or from 0.1 wt% to 5.0 wt% of at least one astringent.

In at least one preferred embodiment, the composition comprises a sun protection agent and/or UV filter. Suitable sun protection agents and UV filters are disclosed in WO2013/017262A1 (published on 7th Feb 2013), from page 32, line 11 to the end of page 33. The photoprotective substances include, in particular, all of the photoprotective substances specified in EP-A 1084696, which is incorporated herein by reference. In another preferred embodiment, the compositions according to the invention contain one or more substances selected from inorganic and organic UV

filters and especially preferably are in the form of sunscreen compositions.

The compositions according to the invention can contain microfine titanium dioxide, mica-titanium oxide, iron oxides, mica-iron oxide, zinc oxide, silicon oxides, ultramarine blue or chromium oxides as pigments/micropigments and as inorganic sunscreen filters or UV filters. The organic sunscreen filters or UV filters are preferably selected from 4-aminobenzoic acid, 3-(4'-trimethylammonium)-benzylidene-bornan-2-one-methylsulfate, camphor benzalkonium methosulfate, 3,3,5-trimethyl-cyclohexylsalicylate, 2-hydroxy-4-methoxybenzophenone, 2-phenylbenzimidazole-5-sulfonic acid and their potassium, sodium and triethanolamine salts, 3,3'-(1,4-phenylenedimethine)-bis(7,7-dimethyl-2-oxobicyclo[2.2.1]-heptane-1-methanesulfonic acid) and salts thereof, 1-(4-tert-butylphenyl)-3-(4-methoxyphenyl)propane-1,3-dione, 3-(4'-sulfo)-benzylidene-bornan-2-one and salts thereof, 2-cyano-3,3-diphenylacrylic acid-(2-ethylhexyl ester), polymers of N-[2(and 4)-(2-oxoborn-3-ylidenemethyl)benzyl]-acrylamide, 4-methoxycinnamic acid-2-ethylhexyl ester, ethoxylated ethyl-4-aminobenzoate, 4-methoxycinnamic acid isoamyl ester, 2,4,6-tris-[p-(2-ethylhexyloxycarbonyl)anilino]-1,3,5-triazine, 2-(2H-benzotriazol-2-yl)-4-methyl-6-(2-methyl-3-(1,3,3,3-tetramethyl-1-(trimethylsilyloxy)-disiloxanyl)-propyl)phenol, 4,4'-[(6-[4-((1,1-dimethylethyl)-aminocarbonyl)phenylamino]-1,3,5-triazin-2,4-yl)diimino]bis-(benzoic acid-2-ethylhexyl ester), benzophenone-3, benzophenone-4 (acid), 3-(4'-methylbenzylidene)-D,L-camphor, 3-benzylidene-camphor, salicylic acid-2-ethylhexyl ester, 4-dimethylaminobenzoic acid-2-ethylhexyl ester, hydroxy-4-methoxybenzophenone-5-sulfonic acid (sulfisobenzonum) and the sodium salt, 4-isopropylbenzylsalicylate, N,N,N-trimethyl-4-(2-oxoborn-3-ylidenemethyl)anilium methyl sulfate, homosalate (INN), oxybenzone (INN), 2-phenylbenzimidazole-5-sulfonic acid and their sodium, potassium, and triethanolamine salts, octylmethoxycinnamic acid, isopentyl-4-methoxycinnamic acid, isoamyl-p-methoxycinnamic acid, 2,4,6-trianilino-(p-carbo-2'-ethylhexyl-1'-oxy)-1,3,5-triazine (octyl triazone) phenol, 2-2(2H-benzotriazol-2-yl)-4-methyl-6-(2-methyl-3-(1,3,3,3-tetramethyl-1-(trimethylsilyl)oxy)-disiloxanyl)propyl (drometrizole trisiloxane) benzoic acid, 4,4-((6-(((1,1-dimethylethyl)amino)carbonyl)phenyl)amino)-1,3,5-triazine-2,4-diyl)diimino)bis,bis(2-ethylhexyl)ester) benzoic acid, 4,4-((6-(((1,1-dimethylethyl)amino)-carbonyl)phenyl)amino)-1,3,5-triazine-2,4-diyl)diimino)bis,bis(2-ethylhexyl)ester), 3-(4'-methylbenzylidene)-D,L-camphor (4-methylbenzylidene camphor), benzylidene-camphor-sulfonic acid, octocrylene, polyacrylamidomethyl-benzylidene-camphor, 2-ethylhexyl salicylate (octyl salicylate), 4-dimethylaminobenzoic acid ethyl-2-hexyl ester (octyl dimethyl PABA), PEG-25 PABA, 2-hydroxy-4-methoxybenzophenone-5-sulfonic acid (benzophenone-5) and the Na salt, 2,2'-methylene-bis-6-(2H-benzotriazol-2-yl)-4-(tetramethylbutyl)-1,1,3,3-phenol, sodium salt of 2-2'-bis-(1,4-phenylene)1H-benzimidazole-4,6-disulfonic acid, (1,3,5)-triazine-2,4-bis((4-(2-ethylhexyloxy)-2-hydroxy)-phenyl)-6-(4-methoxyphenyl), 2-ethylhexyl-2-cyano-3,3-diphenyl-2-propenoate, glyceryl octanoate di-p-methoxycinnamic acid, p-amino-benzoic acid and esters thereof, 4-tert-butyl-4'-methoxydibenzoylmethane, 4-(2-β-glucopyranoxy)propoxy-2-hydroxybenzophenone, octyl salicylate, methyl-2,5-diisopropylcinnamic acid, cinoxate, dihydroxy-dimethoxybenzophenone, disodium salt of 2,2'-dihydroxy-4,4'-dimethoxy-5,5'-disulfobenzophenone, dihydroxybenzophenone, 1,3,4-dimethoxyphenyl-4,4-dimethyl-1,3-pentanedione, 2-ethylhexyl-dimethoxybenzylidene-dioxoimidazolidine propionate, methylene-bis-benzotriazolyl tetramethylbutylphenol, phenyldibenzimidazole tetrasulfonate, bis-ethylhexyloxyphenol-methoxyphenol-triazine, tetrahydroxybenzophenones, terephthalylidene-dicamphor-sulfonic acid, 2,4,6-tris[4,2-ethylhexyloxycarbonyl)anilino]-1,3,5-triazine, methyl-bis(trimethylsiloxy)silyl-isopentyl trimethoxycinnamic acid, amyl-p-dimethylaminobenzoate, amyl-p-dimethylaminobenzoate, 2-ethylhexyl-p-dimethylaminobenzoate, isopropyl-p-methoxycinnamic acid/diisopropylcinnamic acid ester, 2-ethylhexyl-p-methoxycinnamic acid, 2-hydroxy-4-methoxybenzophenone, 2-hydroxy-4-methoxybenzophenone-5-sulfo acid and the trihydrate, and 2-hydroxy-4-methoxybenzophenone-5-sulfonate sodium salt, phenyl-benzimidazole-sulfonic acid, p-aminobenzoic acid butyl ester, methyl-3-[2,4-bis(methylethyl)phenyl]-2-propenoate, 3-(4-hydroxy)-3-methoxyphenyl)-2-propenoic acid, 5-methyl-2-(1-methylethyl)cyclohexanol-2-aminobenzoate, diethylmalonylbenzylidene oxypropene dimethicone, 2,4,6-tris(biphenyl-4-yl)-1,3,5-triazin and tris(2-hydroxyethyl)ammonium 2-hydroxybenzoate. In at least one preferred embodiment, the sun protection agent and/or UV filter is selected from the group consisting of 2-ethylhexyl 4-methoxycinnamate, methyl methoxycinnamate, 2-ethylhexyl salicylate, 2-ethylhexyl-2-cyano-3,3-diphenyl-2-propenoate, 2-hydroxy-4-methoxybenzophenone-5-sulfonic acid, polyethoxylated p-aminobenzoates, and combinations thereof. In at least one preferred embodiment, the composition comprises from 0.001 wt% to 30 wt%, preferably from 0.05 wt% to 20 wt%, even more preferably from 0.1 wt% to 10 wt%, most preferably from 0.05 wt% to 5.0 wt% of at least one sun protection agent and/or UV filter.

In at least one preferred embodiment, the composition comprises a skin conditioning agent. Skin conditioning agents such as emollients, humectants and occlusive agents are ingredients which help to maintain the soft and smooth appearance of the skin or which help to improve the condition of dry or damaged skin. In at least one preferred embodiment, the skin conditioning agent is selected from the group consisting of emollients (description see above), functional acids or active ingredients (description see above), fatty acid N-alkylpolyhydroxyalkyl amides, fatty acids, triglycerides, panthenol, allantoin, bisabolol, glycerol, sorbitol, urea and derivatives thereof, trehalose, erythrulose, pyrrolidone carboxylic acid (PCA) and its salts, polyglucuronic acid, gluconolactone, ubichinon-10 and ubiquinol. In at least one preferred embodiment, the skin conditioning agent is selected from the group consisting of urea, glycerine, pyrrolidone carboxylic acid (PCA) and its salts, panthenol, and combinations thereof. In at least one preferred embodiment, the composition comprises from 0.001 wt% to 5.0 wt%, preferably from 0.05 wt% to 3.0 wt%, even more preferably from 0.1 wt% to 1.0

wt% of at least one skin conditioning agent.

In at least one preferred embodiment, the composition comprises an anti-foaming agent. Antifoams are chemicals which reduce the tendency of finished products to generate foam on shaking or agitation. In at least one preferred embodiment, the anti-foaming agent is selected from the group consisting of alcohols (e.g. ethanol, isopropyl alcohol or propyl alcohol), alkoxylated alcohols (e.g. laureth-5 butyl ether), silicon oils and resins (e.g. dimethicone and its derivatives such as cetyl dimethicone, phenyl dimethicone, PEG/PPG-12/18 dimethicone and hydrogen trifluoropropyl dimethicone, trimethylsiloxysilicate/ dimethicone crosspolymer or polysilicone-10) and hydrophobic silica derivatives (e.g. silica silylate). In at least one preferred embodiment, the anti-foaming agent is selected from the group consisting of ethanol, dimethicone, silica silylate, and combinations thereof. In at least one preferred embodiment, the composition comprises from 0.01 wt% to 5.0 wt%, preferably from 0.1 wt% to 3.0 wt%, even more preferably from 0.5 wt% to 2.0 wt% of at least one anti-foaming agent.

In at least one preferred embodiment, the composition comprises a flavouring agent. In at least one preferred embodiment, the flavouring agent is selected from the group consisting of 1-acetonaphthalene, 1-decen-3-ol, p-methylbenzaldehyde, p-propenylphenyl methyl ether, aspartame, benzaldehyde, bromocinnamal, calcium cyclohexylsulfamate, calcium o-benzolufimide, carvone, cinnamic aldehyde, 3,7-dimethyl-6-octenoic acid, fruit sugar, glucose, glucosyl stevioside, honey, 3-methyl-1-butanol, 4-hydroxy-3-methoxy-1-propenylbenzene, malt sugar, menthol, eucalyptol, thymol, potassium 6-methyl-1,2,2-oxathiazin-4(3H)-one 2,2'-dioxide, isodulcitol, saccharine, stevioside, 1',4,6'-trichlorogalacto-sucrose, sorbitol, saccharose, sodium saccharin, methyl salicylate vanillaldehyde, xylite, xylose and plant extracts. In at least one preferred embodiment, the flavouring agent is selected from the group consisting of benzaldehyde, cinnamic aldehyde, fruit sugar, stevioside and its derivatives, saccharine, saccharose, vanillaldehyde, xylite, and combinations thereof. In at least one preferred embodiment, the composition comprises from 0.001 wt% to 3.0 wt%, preferably from 0.01 wt% to 2.0 wt%, even more preferably from 0.05 wt% to 1.0 wt% of at least one flavouring agent.

In at least one preferred embodiment, the composition comprises an electrolyte. In at least one preferred embodiment, the electrolyte is selected from the group consisting of salts preferably ammonium or metal salts, especially preferably halides, for example $CaCl_2$, $MgCl_2$, LiCl, KCl and NaCl, carbonates, hydrogen carbonates, phosphates, sulfates, nitrates, especially preferably sodium chloride, sodium fluoride, sodium monofluorophosphate, stannous fluoride, and/or organic salts, preferably ammonium or metal salts, especially preferably of glycolic acid, lactic acid, citric acid, tartaric acid, mandelic acid, salicylic acid, ascorbic acid, pyruvic acid, fumaric acid, retinoic acid, sulfonic acids, benzoic acid, kojic acid, fruit acid, malic acid, gluconic acid or galacturonic acid. These also include aluminum salts, preferably aluminum hydrochloride or aluminum-zirconium complex salts. In at least one preferred embodiment the compositions according to the invention therefore contain one or more substances selected from inorganic and organic salts. As electrolyte, the compositions according to the invention can also contain mixtures of various salts. In at least one preferred embodiment, the electrolyte is selected from the group consisting of sodium chloride, magnesium chloride, sodium citrate, sodium acetate, sodium hyaluronate, and combinations thereof. In at least one preferred embodiment, the composition comprises from 0.001 wt% to 5.0 wt%, preferably from 0.05 wt% to 3.0 wt%, even more preferably from 0.1 wt% to 1.0 wt% of at least one electrolyte.

In at least one preferred embodiment, the composition comprises an oxidizing or reducing agent. In at least one preferred embodiment, the oxidizing or reducing agent is selected from the group consisting of ammonium persulfate, calcium peroxide, hydrogen peroxide, hypochlorous acid, sodium hypochlorite, potassium monopersulfate, sodium carbonate peroxide, ammonium thioglycolate, cysteine, glutathione, hydroquinone, mercaptopropionic acid, superoxide dismutase, thioglycerin, thioglycolic acid, thiolactic acid, sodium sulfite, sodium thioglycolate, potassium thioglycolate, cysteine, the compositions according to the invention can also contain mixtures of various agents.

In at least one preferred embodiment, the oxidizing or reducing agent is selected from the group consisting of hydrogen peroxide, sodium hypochlorite, superoxide dismutase, thioglycolic acid, sodium thioglycolate, potassium thioglycolate, cysteine, sodium carbonate peroxide, and combinations thereof. In at least one preferred embodiment, the composition comprises from 0.001 wt% to 10 wt%, preferably from 0.05 wt% to 7.0 wt%, even more preferably from 0.1 wt% to 5.0 wt% of at least one oxidizing or reducing agent.

In at least one preferred embodiment, the composition comprises a hair conditioning agent. In at least one preferred embodiment, the hair conditioning agent is a water insoluble, water dispersible, non-volatile, liquid that forms emulsified, liquid particles. In at least one preferred embodiment, the hair conditioning agent is a silicone (e.g., silicone oil, cationic silicone, silicone gum, high refractive silicone, and silicone resin), an organic conditioning oil (e.g., hydrocarbon oils, polyolefins, and fatty esters), or combinations thereof.

In at least one preferred embodiment, the hair conditioning agent is a silicone, and wherein the composition comprises from 0.01% to 10%, or from 0.1% to 5% silicone hair conditioning agent, by total weight of the composition. Suitable silicone hair conditioning agents, and optional suspending agents for the silicone, are described in US 5,104,646. In at least one preferred embodiment, the composition comprises a silicone gum selected from the group consisting of polydimethylsiloxane, (polydimethylsiloxane) (methylvinylsiloxane) copolymer, poly(dimethylsiloxane) (diphenylsiloxane) (methylvinylsiloxane) copolymer, and mixtures thereof.

In at least one preferred embodiment, the composition comprises a terminal aminosilicone. "Terminal aminosilicone" as defined herein means silicone comprising one or more amino groups at one or both ends of the silicone backbone. In at least one preferred embodiment, the composition is substantially free of any silicone compound comprising pendant amino groups. In at least one preferred embodiment, the composition is substantially free of any silicone compound other than terminal aminosilicones. In at least one preferred embodiment, the amino group at least one terminus of the silicone backbone of the terminal aminosilicone is selected from the group consisting of primary amines, secondary amines and tertiary amines. In at least one preferred embodiment, the composition comprises a terminal aminosilicone conforming to Formula (S):

$$(R^F)_aG_{3-a}\text{-Si-(-OSiG}_2)_n\text{-O-SiG}_{3-a}(R^F)_a \qquad (S)$$

wherein

G is hydrogen, phenyl, hydroxy, or C1-C8 alkyl, preferably methyl; a is an integer having a value from 1 to 3, preferably 1; b is 0, 1 or 2, preferably 1; n is a number from 0 to 1,999; $R^F$ is a monovalent radical conforming to the general Formula $C_qH_{2q}L$, wherein q is an integer having a value from 2 to 8 and L is selected from the following groups: $-N(R^T)CH_2-CH_2-N(R^T)_2$; $-N(R^T)_2$; $-N(R^T)_3A$-; $-N(R^T)CH_2-CH_2-NR^TH_2A^-$; wherein $R^T$ is hydrogen, phenyl, benzyl, or a saturated hydrocarbon radical, preferably an alkyl radical from having from 1 to 20 carbon atoms; $A^-$ is a halide ion.

In at least one preferred embodiment, the terminal aminosilicone corresponding to Formula (S) has a=1, q=3, G=methyl, n is from 1000 to 2500, alternatively from 1500 to 1700; and L is $-N(CH_3)_2$. A suitable terminal aminosilicone corresponding to Formula III has a=O, G=methyl, n is from 100 to 1500, or from 200 to 800, and L is selected from the following groups: $-N(R^T)CH_2-CH_2-N(R^T)_2$; $-N(R^T)_2$; $-N(R^T)_3A$; $-N(R^T)CH_2-CH_2-NR^TH_2A$; wherein $R^T$ is hydrogen, phenyl, benzyl, or a saturated hydrocarbon radical, preferably an alkyl radical from having from 1 to 20 carbon atoms; $A^-$ is a halide ion, alternatively L is $-NH_2$. In at least one preferred embodiment, the terminal aminosilicone is selected from the group consisting of bis-aminomethyl dimethicone, bisaminoethyl dimethicone, bis-aminopropyl dimethicone, bis-aminobutyl dimethicone, and mixtures thereof. In at least one preferred embodiment, the viscosity of the terminal aminosilicone is from 1,000 to 30,000 cPs, or from 5,000 to 20,000 cPs measured at 25°C.

In at least one preferred embodiment, the composition comprises from 0.1% to 20%, or from 0.5% to 10%, or from 1 % to 6% terminal aminosilicone, by total weight of the composition.

In at least one preferred embodiment, the composition comprises a high melting point fatty compound. The high melting point fatty compound has a melting point of 25°C or higher. In at least one preferred embodiment, the high melting point fatty compound is selected from the group consisting of a fatty alcohol, fatty acid, fatty alcohol derivative, fatty acid derivative, and mixtures thereof. Non-limiting examples of the high melting point compounds are found in International Cosmetic Ingredient Dictionary, Fifth Edition, 1993, and CTFA Cosmetic Ingredient Handbook, Second Edition, 1992. The composition may comprise from 0.1% to 40%, or from 1% to 30%, or from 1.5% to 16%, or from 1.5% to 8% of a high melting point fatty compound, by total weight of the composition. This is advantageous in view of providing improved conditioning benefits such as slippery feel during the application to wet hair, softness and moisturized feel on dry hair. In at least one preferred embodiment, the fatty alcohol is selected from the group consisting of: cetyl alcohol, stearyl alcohol, behenyl alcohol, and mixtures thereof. In at least one preferred embodiment, the composition comprises a linear fatty alcohol. The linear fatty alcohol may comprise from 8 to 24 carbon atoms. In at least one preferred embodiment, the linear fatty alcohol is selected from the group consisting of: cetyl alcohol, stearyl alcohol, and mixtures thereof.

In at least one preferred embodiment, the composition of the invention comprises a surfactant. In at least one preferred embodiment, the composition of the invention comprises a combination of surfactants. In at least one preferred embodiment, the composition of the invention comprises from 0.01 wt.-% to 70 wt.-%, preferably from 0.1 wt.-% to 40%, more preferably from 1 wt.-% to 30%, even more preferably from 2 wt.-% to 20 wt.-% of one or more surfactants, based on the total weight of the composition.

In at least one preferred embodiment, the composition comprises a cationic surfactant. In at least one preferred embodiment, the composition comprises from 0.05% to 3.0%, or from 0.075% to 2.0%, or from 0.1% to 1.0%, of a cationic surfactant by total weight of the composition. In at least one preferred embodiment, the cationic surfactant is according to Formula (C):

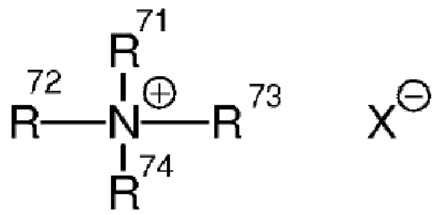

(C)

wherein

at least one of $R^{71}$, $R^{72}$, $R^{73}$ and $R^{74}$ is selected from an aliphatic group of from 8 to 30 carbon atoms, an aromatic, alkoxy, polyoxyalkylene, alkylamido, hydroxyalkyl, aryl, or an alkylaryl group having up to 22 carbon atoms;
the remainder of $R^{71}$, $R^{72}$, $R^{73}$ and $R^{74}$ are independently selected from the group consisting of an aliphatic group of from 1 to 22 carbon atoms, and an aromatic, alkoxy, polyoxyalkylene, alkylamido, hydroxyalkyl, aryl or alkylaryl group having up to 22 carbon atoms;
X is selected from the group consisting of: halogen, acetate, citrate, lactate, glycolate, phosphate, nitrate, sulfonate, sulfate, alkylsulfate, alkyl sulfonate radicals, and combinations thereof.

In at least one preferred embodiment, the cationic surfactant is selected from the group consisting of behenyl trimethyl ammonium chloride, methyl sulfate or ethyl sulfate, and stearyl trimethyl ammonium chloride, methyl sulfate or ethyl sulfate.

In at least one preferred embodiment, the cationic surfactant is a di-long alkyl quaternized ammonium salt selected from the group consisting of: dialkyl (14-18) dimethyl ammonium chloride, ditallow alkyl dimethyl ammonium chloride, dihydrogenated tallow alkyl dimethyl ammonium chloride, distearyl dimethyl ammonium chloride, dicetyl dimethyl ammonium chloride, and mixtures thereof.

In at least one preferred embodiment, the cationic surfactant is a tertiary amido amine having an alkyl group of from 12 to 22 carbons. The tertiary amido amine may be selected from the group consisting of stearamidopropyldimethyl-, stearamidopropyldiethyl-, stearamidoethyldiethyl-, stearamidoethyldimethyl-, palmitamidopropyldimethyl-, palmitamidopropyldiethyl-, palmitamidoethyldiethyl-, palmitamidoethyldimethyl-, behenamidopropyldimethyl-, behenamidopropyldiethyl-, behenamidoethyldiethyl-, behenamidoethyldimethyl-, arachidamidopropy Idimethy 1-, arachidamidopropyldiethyl-, arachidamidoethyldiethyl-, and arachidamidoethyldimethyl-amine, diethylaminoethylstearamide, and mixtures thereof. A tertiary amido amine may be used in combination with an acid. The acid is typically used as a salt-forming anion. In at least one preferred embodiment, the acid is selected from the group consisting of lactic acid, malic acid, hydrochloric acid, 1-glumatic acid, acetic acid, citric acid, and mixtures thereof.

In at least one preferred embodiment, the cationic surfactant is selected from the group consisting of cetyltrimonium chloride (CTAC), stearyltrimonium chloride (STAC), behentrimonium methosulfate, stearoylamidopropyldimethyl amine (SAPDMA), distearyldimethylammonium chloride, and mixtures thereof.

In at least one preferred embodiment, the composition comprises an amphoteric surfactant. In at least one preferred embodiment, the amphoteric surfactant is selected from the group consisting of N-($C_{12}$-$C_{18}$)-alkyl-β-aminopropionates and N-($C_{12}$-$C_{18}$)-alkyl-β-iminodipropionates as alkali metal salts or mono-, di-, or trialkylammonium salts; N-acylaminoalkyl-N,N-dimethylacetobetaine, preferably N-($C_8$-$C_{18}$)-acylaminopropyl-N,N-dimethylacetobetaine, ($C_{12}$-$C_{18}$)-alkyl-dimethyl-sulfopropylbetaine, amphosurfactants based on imidazoline (trade name: Miranol, Steinapon), preferably the sodium salt of 1-(β-carboxymethyloxyethyl)-1-(carboxymethyl)-2-laurylimidazolinium; amine oxide, e.g., ($C_{12}$-$C_{18}$)-alkyl-dimethylamine oxide, fatty acid amidoalkyldimethylamine oxide, and mixtures thereof.

In at least one preferred embodiment, the composition comprises a betaine surfactant. In at least one preferred embodiment, the betaine surfactant is selected from $C_8$- to $C_{18}$-alkylbetaines. In at least one preferred embodiment, the betaine surfactant is selected from the group consisting of cocodimethylcarboxymethylbetaine, lauryldimethylcarboxymethylbetaine, lauryldimethylalphacarboxyethylbetaine, cetyldimethylcarboxymethylbetaine, oleyldimethylgammacarboxypropylbetaine and laurylbis(2-hydroxypropyl)alphacarboxyethylbetaine and combinations thereof. In at least one preferred embodiment, the betaine surfactant is selected from $C_8$- to $C_{18}$-sulfobetaines. In at least one preferred embodiment, the betaine surfactant is selected from the group consisting of cocodimethylsulfopropylbetaine, stearyldimethylsulfopropylbetaine, lauryldimethylsulfoethylbetaine, laurylbis(2-hydroxyethyl)sulfopropylbetaine, and combinations thereof. In at least one preferred embodiment, the betaine surfactant is selected from carboxyl derivatives of imidazole, the $C_8$- to $C_{18}$-alkyldimethylammonium acetates, the $C_8$- to $C_{18}$-alkyldimethylcarbonylmethylammonium salts, and the $C_8$- to $C_{18}$-fatty acid alkylamidobetaines, and mixtures thereof. In at least one preferred embodiment, the $C_8$- to $C_{18}$-fatty acid alkylamidobetaine is selected from coconut fatty acid amidopropylbetaine, N-coconut fatty acid amidoethyl-N-[2-(carboxymethoxy)ethyl]glycerol (CTFA name: Cocoamphocarboxyglycinate), and mixtures thereof.

A particularly preferred amphoteric or betaine surfactant is cocamidopropyl betaine. A further preferred amphoteric or betaine surfactant is sodium cocoamphoacetate.

The total amount of amphoteric or betaine surfactant in the composition of the invention may, for example, range from 0.5 wt.-% to 20 wt.-%, more preferably from 1 wt.-% to 10 wt.-%, based on the total weight of the composition.

In at least one preferred embodiment, the composition comprises an anionic surfactant. In at least one preferred embodiment, the anionic surfactant is selected from the group consisting of ($C_{10}$-$C_{20}$)-alkyl and alkylene carboxylates, alkyl ether carboxylates, fatty alcohol sulfates, fatty alcohol ether sulfates, fatty acid salts, mono-, di- or triphosphoric acid esters, alkylamide sulfates and sulfonates, fatty acid alkylamide polyglycol ether sulfates, alkanesulfonates and hydroxyalkanesulfonates, olefinsulfonates, acyl esters of isethionates, $\alpha$-sulfo fatty acid esters, alkylbenzenesulfonates, alkylphenol glycol ether sulfonates, sulfosuccinates, sulfosuccinic monoesters and diesters, fatty alcohol ether phosphates, protein/fatty acid condensation products, alkyl monoglyceride sulfates and sulfonates, alkylglyceride ether sulfonates, fatty acid methyltaurides, fatty acid sarcosinates, sulforicinoleates, acylglutamates, and mixtures thereof. The anionic surfactants (and their mixtures) can be used in the form of their water-soluble or water-dispersible salts, examples being the sodium, potassium, magnesium, ammonium, mono-, di-, and triethanolammonium, and analogous alkylammonium salts. In at least one preferred embodiment, the anionic surfactant is the salt of an anionic surfactant comprising 12 to 14 carbon atoms. In at least one preferred embodiment, the anionic surfactant is selected from the group consisting of sodium lauryl sulfate, sodium laureth sulfate, sodium tridecyl sulfate, sodium trideceth sulfate, sodium myristyl sulfate, sodium myreth sulfate, and mixtures thereof. Typical anionic surfactants include sodium oleyl succinate, ammonium lauryl sulphosuccinate, sodium lauryl sulphate, sodium lauryl ether sulphate, sodium lauryl ether sulphosuccinate, ammonium lauryl sulphate, ammonium lauryl ether sulphate, sodium dodecyl benzene sulphonate, triethanolamine dodecylbenzene sulphonate, sodium cocoyl isethionate, sodium lauryl isethionate, lauryl ether carboxylic acid and sodium N-lauryl sarcosinate. Preferred anionic surfactants are selected from sodium lauryl sulphate and sodium lauryl ether sulphate(n)EO, where n is from 1 to 3; more preferably sodium lauryl ether sulphate(n)EO, where n is from 1 to 3; most preferably sodium lauryl ether sulphate(n)EO, where n is 1.

In at least one preferred embodiment, the anionic surfactant is a fatty acyl isethionate. Preferably, greater than 20 wt.-% and less than 45 wt.-%, more preferably greater than 25 wt.-% and less than 45 wt.-% of the fatty acyl isethionates are of chain length greater than or equal to $C_6$. Preferably, a mixture of aliphatic fatty acids is used for the preparation of fatty acyl isethionate surfactants. The fatty acyl isethionate surfactants (e.g., resulting from reaction of alkali metal isethionate and aliphatic fatty acid) preferably have more than 20 wt.-%, more preferably more than 25 wt.-%, but no more than 45 wt.-%, preferably no more than 35 wt.-% (on basis of fatty acyl isethionate reaction product) of fatty acyl group with 16 or greater carbon atoms. This provides both excellent lather and mildness of the resulting fatty acyl isethionate product.

In at least one preferred embodiment, the anionic surfactant is an acylglycinate surfactant. In at least one preferred embodiment, the acylglycinate surfactant conforms to Formula (Y):

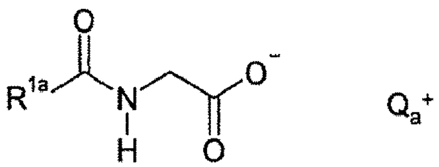

(Y)

wherein

$R^{1a}$ is a linear or branched, saturated alkyl group having 6 to 30, preferably 8 to 22, particularly preferably 8 to 18 carbon atoms or is a linear or branched, mono- or polyunsaturated alkenyl group having 6 to 30, preferably 8 to 22 and particularly preferably 12 to 18 carbon atoms, and $Q_a^+$ is a cation. In at least one preferred embodiment, $Q_a^+$ is selected from the group consisting of $Li^+$, $Na^+$, $K^+$, $Mg^{++}$, $Ca^{++}$, $Al^{+++}$, $NH_4^+$, a monoalkylammmonium ion, a dialkylammonium ion, a trialkylammonium ion and a tetraalkylammonium ion, or combinations thereof. Optionally $R^{1a}$ is ($C_1$-$C_{22}$)-alkyl or ($C_2$-$C_{10}$)-hydroxyalkyl. In at least one preferred embodiment, the acylglycinate surfactant is selected from sodium cocoylglycinate and potassium cocoylglycinate. In at least one preferred embodiment, the acylglycinate surfactant is selected from those conforming to Formula (Y), wherein $R^{1a}$ is $C_{11}$-alkyl, $C_{12}$-alkyl, $C_{13}$-alkyl or $C_{14}$-alkyl, particularly preferably $C_{11}$-alkyl or $C_{13}$-alkyl. In at least one preferred embodiment, the acylglycinate surfactant is selected from those conforming to Formula (Y), wherein $R^{1a}$ is $C_{15}$-alkyl, $C_{16}$-alkyl, $C_{17}$-alkyl or $C_{18}$-alkyl, particularly preferably $C_{15}$-alkyl or $C_{17}$-alkyl.

In at least one preferred embodiment, the anionic surfactant is a glutamate surfactant. In at least one preferred embodiment, the anionic surfactant is a glutamate surfactant corresponding to Formula (Z) or a salt thereof:

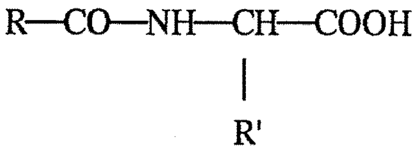

(Z)

wherein

R' is HOOC-$CH_2$-$CH_2$- or $M^{+-}$OOC-$CH_2$-$CH_2$- wherein $M^+$ is a cation; and wherein R is a linear or branched, saturated alkyl group having 6 to 30, preferably 8 to 22, more preferably 8 to 18 carbon atoms or is a linear or branched, mono- or polyunsaturated alkenyl group having 6 to 30, preferably 8 to 22, more preferably 12 to 18 carbon atoms. In at least one preferred embodiment, $M^+$ is a metal cation. In at least one preferred embodiment, $M^+$ is selected from the group consisting of $Li^+$, $Na^+$, $K^+$, $Mg^{++}$, $Ca^{++}$, $Al^{+++}$, $NH_4^+$, a monoalkylammmonium ion, a dialkylammonium ion, a trialkylammonium ion and a tetraalkylammonium ion, or combinations thereof. In at least one preferred embodiment, the glutamate surfactant is selected from sodium cocoyl glutamate and potassium cocoyl glutamate. In at least one preferred embodiment, the glutamate surfactant is selected from those conforming to Formula (Z), wherein R is $C_{11}$-alkyl, $C_{12}$-alkyl, $C_{13}$-alkyl or $C_{14}$-alkyl, particularly preferably $C_{11}$-alkyl or $C_{13}$-alkyl. In at least one preferred embodiment, the glutamate surfactant is selected from those conforming to Formula (Z), wherein R is $C_{15}$-alkyl, $C_{16}$-alkyl, $C_{17}$-alkyl or $C_{18}$-alkyl, particularly preferably $C_{15}$-alkyl or $C_{17}$-alkyl. In at least one preferred embodiment, the composition comprises from 0.01 wt% to 30 wt%, preferably from 1 wt% to 25 wt%, more preferably from 5 wt% to 20 wt%, even more preferably from 12 wt% to 18 wt% glutamate surfactant.

The total amount of anionic surfactant in the composition of the invention may, for example, range from 0.5 wt.-% to 45 wt.-%, more preferably from 1.5 wt.-% to 20 wt.-%, based on the total weight of the composition.

In some embodiments, the composition comprises a hairstyling polymer. The hairstyling polymer may be selected from the group consisting of: amphoteric hairstyling polymers, zwitterionic hairstyling polymers, anionic hairstyling polymers, non-ionic hairstyling polymers, cationic hairstyling polymers, and mixtures thereof. Suitable hairstyling polymers are known to a person skilled in the art. In some embodiments, the composition comprises from 0.01% to 20%, or from 0.01% to 16%, or from 0.01% to 10%, or from 1% to 8%, or from 2% to 6% of hairstyling polymer.

Cosmetic compositions

In at least one preferred embodiment, the cosmetic composition is selected from the group consisting of shampoo, hair and/or skin conditioner, body wash, facial cleanser, face mask, bubble bath, intimate wash, bath oil, cleansing milk, micellar water, make-up remover, cleansing wipes, hair mask, perfume, liquid soap, shaving soap, shaving foam, cleansing foam, day cream, anti-ageing cream, body milk, body lotion, body mousse, face serum, eye cream, sunscreen lotion, sun cream, face cream, after-shave lotion, pre-shaving cream, depilatory cream, skin-whitening gel, self-tanning cream, anti-acne gel, mascara, foundation, primer, concealer, blush, bronzer, blemish balm (bb) cream, eyeliner, night cream, eye brow gel, highlighter, lip stain, hand sanitizer, hair oil, nail varnish remover, hair styling gel, hair styling cream, anti-frizz serum, scalp treatment, hair colorant, split end fluid, deodorant, antiperspirant, baby cream, insect repellent, hand cream, sunscreen gel, foot cream, exfoliator, body scrub, cellulite treatment, bar soap, cuticle cream, lip balm, hair treatment, eye shadow, bath additive, body mist, eau de toilette, lubricating gel, moisturizer, serum, toner, aqua sorbet, cream gel, styling mousse, dry shampoo, lip stick, lip gloss, body oil, shower milk, illuminator, lip crayon, hair spray, combing cream, sunblock, ointment, and lipstick.

In at least one preferred embodiment, the cosmetic composition is for use on skin. In at least one preferred embodiment, the cosmetic composition is for use on hair. In at least one preferred embodiment, the cosmetic composition is for use on scalp.

In a particular embodiment, the cosmetic composition is a hair conditioner. In a particular embodiment, the cosmetic composition is a hair and/or skin moisturizing and/or nourishing composition.

In at least one preferred embodiment, the composition is an emulsion or gel, preferably an oil-in-water (o/w) emulsion, cream gel or hydrogel.

In at least one preferred embodiment, the composition is a hair conditioning and/or hair treatment composition such as leave-on or rinse-off conditioners, masks, lotions, combing creams, detangling creams, anti-frizz liquids, hair serums, color protection creams.

In at least one preferred embodiment, the cosmetic composition of the present invention comprises at least 0.1 wt.-%, or at least 0.2 wt.-%, or at least 0.3 wt.-%, or at least 0.4 wt.-%, or at least 0.5 wt.-%, or at least 0.6 wt.-%, or at least 0.7 wt.-%, or at least 0.8 wt.-%, or at least 0.9 wt.-%, or at least 1.0 wt.-%, or at least 1.1 wt.-%, or at least 1.2 wt.-%, or at least 1.3 wt.-%, or at least 1.4 wt.-%, or at least 1.5 wt.-%, or at least 1.6 wt.-%, or at least 1.5 wt.-%, or at least 1.6 wt.-%, or at least 1.7 wt.-%, or at least 1.8 wt.-%, or at least 1.9 wt.-%, or at least 2.0 wt.-% of the at least one polymer

(component A).

In at least one preferred embodiment, the cosmetic composition of the present invention comprises at least 0.1 wt.-%, or at least 0.2 wt.-%, or at least 0.3 wt.-%, or at least 0.4 wt.-%, or at least 0.5 wt.-%, or at least 0.6 wt.-%, or at least 0.7 wt.-%, or at least 0.8 wt.-%, or at least 0.9 wt.-%, or at least 1.0 wt.-%, or at least 1.1 wt.-%, or at least 1.2 wt.-%, or at least 1.3 wt.-%, or at least 1.4 wt.-%, or at least 1.5 wt.-%, or at least 1.6 wt.-%, or at least 1.5 wt.-%, or at least 1.6 wt.-%, or at least 1.7 wt.-%, or at least 1.8 wt.-%, or at least 1.9 wt.-%, or at least 2.0 wt.-% of the at least one emollient (component B).

In at least one preferred embodiment, the cosmetic composition of the present invention comprises at least 0.1 wt.-%, or at least 0.2 wt.-%, or at least 0.3 wt.-%, or at least 0.4 wt.-%, or at least 0.5 wt.-%, or at least 0.6 wt.-%, or at least 0.7 wt.-%, or at least 0.8 wt.-%, or at least 0.9 wt.-%, or at least 1.0 wt.-%, or at least 1.1 wt.-%, or at least 1.2 wt.-%, or at least 1.3 wt.-%, or at least 1.4 wt.-%, or at least 1.5 wt.-%, or at least 1.6 wt.-%, or at least 1.5 wt.-%, or at least 1.6 wt.-%, or at least 1.7 wt.-%, or at least 1.8 wt.-%, or at least 1.9 wt.-%, or at least 2.0 wt.-% of the at least one ingredient of component C.

In at least one preferred embodiment, the cosmetic composition of the present invention comprises at least 10 wt.-%, or at least 20 wt.-%, or at least 30 wt.-%, or at least 40 wt.-%, or at least 50 wt.-%, or at least 55 wt.-%, or at least 60 wt.-%, or at least 65 wt.-%, or at least 70 wt.-%, or at least 75 wt.-%, or at least 80 wt.-% of water (component D).

In at least one preferred embodiment, the cosmetic composition of the present invention contains components A and B in a weight ratio A:B in a range of from (10:1) to (1:10), or in a range of from (5:1) to (1:5), or in a range of from (2:1) to (1:10), or in a range of from (1:1) to (1:5).

In at least one preferred embodiment, the cosmetic composition of the present invention contains components A and C in a weight ratio A:C in a range of from (10:1) to (1:100), or in a range of from (5:1) to (1:50), or in a range of from (2:1) to (1:50), or in a range of from (1:1) to (1:100), or in a range of from (1:1) to (1:50).

In at least one preferred embodiment, the cosmetic composition of the present invention contains components B and C in a weight ratio B:C in a range of from (10:1) to (1:100), or in a range of from (5:1) to (1:50), or in a range of from (2:1) to (1:50), or in a range of from (1:1) to (1:100), or in a range of from (1:1) to (1:50).

In at least one preferred embodiment, the cosmetic composition of the present invention comprises 0.1 to 25 wt.-%, or 0.2 to 10 wt.-%, or 0.3 to 7 wt.-%, or 0.4 to 5 wt.-%, or 0.5 to 2 wt.-%, of the at least one polymer (component A).

In at least one preferred embodiment, the cosmetic composition of the present invention comprises 0.1 to 25 wt.-%, or 0.2 to 15 wt.-%, or 0.3 to 10 wt.-%, or 0.4 to 7 wt.-%, or 0.5 to 5 wt.-%, of the at least one emollient (component B).

In at least one preferred embodiment, the cosmetic composition of the present invention comprises 0.1 to 25 wt.-%, or 0.2 to 15 wt.-%, or 0.3 to 10 wt.-%, or 0.4 to 7 wt.-%, or 0.5 to 5 wt.-%, of the at least one ingredient of component C.

In at least one preferred embodiment, the cosmetic composition of the present invention comprises from 10 to 99 wt.-%, or from 10 to 98.5 wt.-%, or from 10 to 98 wt.-%, or from 10 to 97.8 wt.-%, or from 10 to 97.5 wt.-%, or from 10 to 97 wt.-%, or from 10 to 95 wt.-%, or from 20 to 94 wt.-%, or from 30 to 93 wt.-%, or from 40 to 92 wt.-%, or from 50 to 91 wt.-%, or from 60 to 90 wt.-%, or from 70 to 90 wt.-% of water (component D).

In at least one preferred embodiment, the cosmetic composition of the present invention further comprises at least one solvent in addition to water.

In at least one preferred embodiment, the cosmetic composition of the present invention further comprises at least 1 wt.-%, or at least 5 wt.-%, or at least 10 wt.-%, or at least 20 wt.-%, or at least 30 wt.-%, or at least 40 wt.-%, or at least 50 wt.-%, or at least 60 wt.-% of at least one solvent in addition to water (component D).

In at least one preferred embodiment, the cosmetic composition of the present invention comprises:

(A) at least 0.1 wt.-% of the at least one polymer which comprises at least 5 mol-% of repeating units (a) according to Formula (1) (component A);
(B) at least 0.5 wt.-% of the at least one emollient (component B);
(C) at least 0.4 wt.-% of the at least one ingredient of component C; and
(D) 10 to 99 wt.-% of water; and
(E) optionally at least one further cosmetically acceptable component.

In at least one preferred embodiment, cosmetic composition contains components A, B and C in the following weight ratio:

| | |
|---|---|
| weight ratio A:B: | from (10:1) to (1:100), |
| weight ratio A:C: | from (10:1) to (1:10), and/or |
| weight ratio C:B: | from (10:1) to (1:100). |

In at least one preferred embodiment, the cosmetic composition of the present invention comprises:

(A) 0.1 to 10 wt.-% of the at least one polymer which comprises at least 5 mol-% of repeating units (a) according to Formula (1) (component A);
(B) 0.5 to 20 wt.-% of the at least one emollient (component B);
(C) 0.4 to 10 wt.-% of the at least one ingredient of component C; and
(D) 10 to 99 wt.-% of water; and
(E) optionally 0 to 50 wt.-% of at least one further cosmetically acceptable component.

In at least one preferred embodiment, the cosmetic composition of the present invention comprises:

(A) 0.2 to 5 wt.-% of the at least one polymer which comprises at least 5 mol-% of repeating units (a) according to Formula (1) (component A);
(B) 1 to 15 wt.-% of the at least one emollient (component B);
(C) 1 to 8 wt.-% of the at least one ingredient of component C; and
(D) 25 to 97.8 wt.-%, preferably 50 to 97 wt.-% of water; and
(E) optionally 0 to 50 wt.-%, preferably 0 to 25 wt.-%, more preferably 0 to 10 wt.-% of at least one further cosmetically acceptable component.

The cosmetic composition of the present invention may have any viscosity. In at least one preferred embodiment, it has a viscosity of at least 1,000 cps, of at least 5,000 cps, of at least 10,000 cps, of at least 15,000 cps, of at least 20,000 cps, of at least 25,000 cps, of at least 30,000 cps, of at least 35,000 cps, of at least 40,000 cps, of at least 45,000 cps, of at least 50,000 cps, of at least 100,000 cps, of at least 200,000 cps, of at least 500,000 cps, or even of at least 1,000,000 cps. In at least one preferred embodiment, it has a viscosity of not more than 10,000,000 cps, or of not more than 5,000,000 cps, or of not more than 2,000,000 cps.

In at least one preferred embodiment, the composition has a viscosity from 100 to 200 000 mPa·s, preferably from 1 000 to 100 000 mPa·s, even more preferably from 2 000 to 50 000 mPa·s and very preferably from 5 000 to 30 000 mPa·s, or from 100 000 to 200 000 mPa·s (measured at 25°C, Brookfield RVT, T-C spindle at 20 revolutions per minute).

In at least one preferred embodiment, the composition has a pH in the range of from 4 to 9, preferably from 5 to 8, more preferably from 5 to 7, also more preferably from 6 to 8, for example from 6 to 7.

The cosmetic composition of the present invention may be used for any purpose. In particular, it may be used for conditioning hair and/or skin. The present invention relates to the use of the cosmetic composition of the present invention for conditioning hair and/or skin.

A further aspect of the present invention relates to the use of a cosmetic composition of the present invention for hair and/or skin care.

It will be understood that the embodiments and definitions of the cosmetic composition as such *mutatis mutandis* apply to the use thereof.

In a preferred embodiment, the present invention relates to the use of a cosmetic composition of the present invention for hair care. In a preferred embodiment, the present invention relates to the use of a cosmetic composition of the present invention for improving hair combability, improving hair luster and/or achieving a pleasant degree of luster of hair, thinning hair, detangling hair, improving frizz control (in particular suppress frizzling of the hair), curly retention control of hair, optimizing volume of hair, hydrating hair, re-enforcing hair, protecting hair against oxidative stress, straightening hair, and/or preventing split ends of hair. In a preferred embodiment, the present invention relates to the use of a cosmetic composition of the present invention for improving hair combability.

The present invention also relates to a method for improving hair combability, wherein said method comprises the step of administering sufficient amount of a cosmetic composition of the present invention to hair of a subject. In at least one preferred embodiment, the subject is a human. Preferably, hair is head hear of a human. Alternatively or additionally, hair may also be hair of an animal.

In a further preferred embodiment, the present invention relates to the use of a cosmetic composition of the present invention for skin care. In a preferred embodiment, the present invention relates to the use of a cosmetic composition of the present invention for moisturizing skin, hydrating skin, nourishing skin, promoting skin elasticity, protecting skin against oxidative and other environmental stress, minimizing the appearance of scars of skin, and/or as anti-wrinkle agent.

In a further preferred embodiment, the present invention relates to the use of a cosmetic composition of the present invention for hair and skin care.

The following Examples are for illustrative purposes. The Examples are intended to provide further embodiments of the present invention and do not limit the scope of the present invention.

Examples

Example 1 - Cosmetic compositions

Preparation

The cosmetic compositions (Formulations) depicted in Table 1 were prepared by mixing its ingredients in a conventional manner.

The odour at 25 °C was acceptable in all samples.

**Table 1**. Cosmetic compositions (Formulations), wherein the contents of ingredients are given in wt.-% of the cosmetic composition

| Cosmetic composition No. | | F1 | F2 | F3 | F4 | F5 | F6 |
|---|---|---|---|---|---|---|---|
| Ingredient | INCI name | | | | | | |
| Water | Water | 85.5 | 85.5 | 85.5 | 85.5 | 85.5 | 85.5 |
| Aristoflex AVC | Ammonium acryloyldimethyltaurate/ VP copolymer | 0 | 0 | 0 | 0 | 0 | 1.7 |
| Aristoflex BLV | Ammonium acryloyldimethyltaurate/ beheneth-25 methacrylate crosspolymer | 0 | 0 | 0 | 0 | 0 | 0 |
| Aristoflex Silk | Sodium polyacryloyldimethyl taurate | 0 | 1.7 | 0 | 1.7 | 0 | 0 |
| Aristoflex Velvet | Polyacrylate crosspolymer-11 | 0 | 0 | 1.7 | 0 | 1.7 | 0 |
| Hostacerin MCP | Ammonium acryloyldimethyltaurate/ beheneth-25 methacrylate crosspolymer | 1.7 | 0 | 0 | 0 | 0 | 0 |
| Plantasens VP 170 | Ricinus communis (castor) seed oil, hydrogenated castor oil, and Copernicia cerifera (carnauba) wax, and cera alba (beeswax) | 0 | 10 | 10 | 0 | 0 | 0 |
| Vaseline | Petrolatum | 10 | 0 | 0 | 10 | 10 | 10 |
| Genadvance Hydra | Lauryl/myristyl polyricinoleate (and) glycerin | 0 | 0 | 0 | 0 | 0 | 0 |
| Genadvance Repair | Quaternium-98 | 2 | 2 | 2 | 2 | 2 | 2 |

| | | | | | | | |
|---|---|---|---|---|---|---|---|
| Nipaguard EHP | Ethylhexylglycerin, phenoxyethanol | 0.8 | 0.8 | 0.8 | 0.8 | 0.8 | 0.8 |
| | pH (at 25°C) | 6.56 | 6.7 | 7 | 7.36 | 7.07 | 7.36 |
| | Viscosity (cps) | 75,000 | | | 40,126 | 26,413 | 383,533 |

| Cosmetic composition No. | | F7 | F8 | F9 | F10 | F11 | F12 |
|---|---|---|---|---|---|---|---|
| Ingredient | INCI name | | | | | | |
| Water | Water | 86.2 | 85.5 | 85.5 | 85.5 | 85.9 | 85.5 |
| Aristoflex AVC | Ammonium acryloyldimethyltaurate/ VP copolymer | 0 | 0 | 0 | 1.7 | 0 | 0 |
| Aristoflex BLV | Ammonium acryloyldimethyltaurate/ beheneth-25 methacrylate crosspolymer | 1 | 0 | 0 | 0 | 0 | 0 |
| Aristoflex Silk | Sodium polyacryloyldimethyl taurate | 0 | 1.7 | 0 | 0 | 0 | 0 |
| Aristoflex Velvet | Polyacrylate crosspolymer-11 | 0 | 0 | 1.7 | 0 | 0 | 0 |
| Hostacerin MCP | Ammonium acryloyldimethyltaurate/ beheneth-25 methacrylate crosspolymer | 0 | 0 | 0 | 0 | 1.7 | 1.7 |
| Plantasens VP 170 | Ricinus communis (castor) seed oil, hydrogenated castor oil, and Copernicia cerifera (carnauba) wax, and cera alba (beeswax) | 0 | 0 | 0 | 0 | 0 | 0 |
| Vaseline | Petrolatum | 10 | 10 | 10 | 10 | 10 | 10 |
| Genadvance Hydra | Lauryl/myristyl polyricinoleate (and) glycerin | 0 | 2 | 2 | 2 | 1.6 | 2 |
| Genadvance Repair | Quaternium-98 | 2 | 0 | 0 | 0 | 0 | 0 |
| Nipaguard EHP | Ethylhexylglycerin, phenoxyethanol | 0.8 | 0.8 | 0.8 | 0.8 | 0.8 | 0.8 |
| | pH (at 25°C) | | 7.85 | 5.57 | 6.76 | 5 | 5 |
| | Viscosity (cps) | | 146,000 | 25,020 | 34,720 | 628,750 | 715,800 |

**Example 2 - Stability**

In order to determine stability properties, the cosmetic compositions were evaluated in stability tests. In this test, the samples were exposed to alternating temperatures for several weeks (weeks 1 to 4):

| | |
|---|---|
| each Monday: | 2±2°C |
| each Tuesday: | 45±2°C; |
| each Wednesday: | 2±2°C; |
| each Thursday: | 45±2°C; |
| each Friday: | 25°C; and |

Saturday and Sunday: room temperature of approximately 20-25°C. Alternating temperatures accelerated aging of the composition. The results are depicted in Table 2.

**Table 2**. Comparison of stability of the Formulations

| Cosmetic Composition No. | Week | pH (at 25°C) | Viscosity (cps) |
|---|---|---|---|
| F1 | 0 | 6.56 | 75,000 |
| | 1 | 6.59 | 72,410 |
| | 2 | 6.55 | 75,133 |
| | 3 | - | - |
| | 4 | 6.67 | 71,530 |
| F4 | 0 | 7.36 | 40,126 |
| | 1 | 7.32 | 36,185 |
| | 2 | 7.15 | 32,720 |
| | 3 | 7.35 | 37,410 |
| | 4 | 7.29 | 34,200 |
| F5 | 0 | 7.07 | 26,413 |
| | 1 | 6.88 | 19,805 |
| | 2 | 6.67 | 18,090 |
| | 3 | 6.92 | 19,210 |
| | 4 | 6.84 | 17,333 |
| F6 | 0 | 7.36 | 383,533 |
| | 1 | 7.24 | 296,700 |
| | 2 | 7.04 | 258,750 |
| | 3 | 7.26 | 303,250 |
| | 4 | 7.14 | 248,333 |
| F8 | 0 | 7.85 | 146,000 |
| | 1 | 7.89 | - |
| | 2 | 7.74 | 151,300 |
| | 3 | 7.95 | 226,300 |
| | 4 | 7.91 | 139,300 |
| F9 | 0 | 5.57 | 25,020 |
| | 1 | 5.58 | 21,450 |
| | 2 | 5.46 | 19,080 |
| | 3 | 5.74 | 18,120 |
| | 4 | 5.86 | 21,420 |
| F10 | 0 | 6.76 | 34,720 |
| | 1 | 6.81 | 44,580 |
| | 2 | 6.68 | 57,880 |
| | 3 | 6.87 | 56,065 |
| | 4 | 6.77 | 72,230 |

(continued)

| Cosmetic Composition No. | Week | pH (at 25°C) | Viscosity (cps) |
|---|---|---|---|
| F11 | 0 | 4.73 | 628,750 |
| | 1 | 4.79 | 751,333 |
| | 2 | 4.82 | 903,000 |
| | 3 | - | - |
| | 4 | | 1,255,500 |
| F12 | 0 | 4.62 | 517,666 |
| | 1 | 4.99 | 796,000 |
| | 2 | 4.93 | 741,666 |
| | 3 | - | - |
| | 4 | 4.75 | 700,300 |

The cosmetic compositions tested were found to be stable. No phase separation was observed.

**Example 3 - Cosmetic compositions**

a.) Hair and skin moisturizing nectar

**Table 3**. Example of cosmetic composition based on natural ingredients

| Phase | Product | INCI name | Content (wt.-%) |
|---|---|---|---|
| P-I | Demineralized Water | aqua | Q.S. |
| P-II | Plantasens Olive LD | Hydrogenated ethylhexyl olivate (and) hydrogenated olive oil unsaponifiables | 5 |
| | Tsubaki Oil | *Camellia japonica* seed oil | 2 |
| | Genadvance Hydra | Lauryl/myristyl polyricinoleate (and) glycerin | 1.5 |
| P-III | Aristoflex Silk | Sodium polyacryloyldimethyl taurate | 0.7 |
| P-IV | Herbex Yeast Beta Glucan | Butylene glycol, yeast beta glucan | 2 |
| | Plantasens Berto Orthomoist | *Orthosiphon stamineus* leaf extract, chlorphenesin (glycerin, aqua) | 1 |
| P-V | Nipaguard EHP | Ethylhexylglycerin, phenoxyethanol | 1 |
| | Fragrance (Elderberry Blitz 285, IFF) | Parfum | 0.4 |
| P-VI | Sodium hydroxide solution (10%) | Aqua, sodium hydroxide | Q.S. |

The hair and skin moisturizing nectar may promote skin elasticity and/or improve hair volume and/or cure frizzy-rebel hair and/or serve as a herbal moisturizer.

Preparation:

The hair and skin moisturizing nectar was prepared as follows:
Phase P-I was filled in a large beaker. Phase P-II was filled in a separate beaker. Phase P-III was added to phase P-II and a dispersion was prepared. The dispersion was added to phase P-I. Further, phase P-IV was added to the dispersion

of phases P-I, P-II and P-III. Further, phase P-V was added to the dispersion of phases P-I, P-II, P-III and P-IV. The dispersion was homogenized. Finally, the pH was adjusted to pH 4.5 to 5.5 with an aqueous 10 wt.% NaOH solution (phase P-VI).

Results:

A snow-white, gel-look cream was obtained. This was found to have a light touch. In a centrifuge (3000 rpm, 30 min), no phase separation occurred. Viscosity (Brookfield DV-II, 4 sp, 20 rpm, 20 °C) was found to be in the range of 15,000 +/- 5,000 mPas.

Stability after storage at 50 °C for 15 days: no phase separation. Challenge Test: recommended preservation was checked by a challenge test according to ISO 11930 method and passed with criteria "A".

b.) Skin and hair nourishing creme

**Table 4.** Example of cosmetic composition based on natural ingredients

| Phase | Product | INCI name | Content (wt.-%) |
|---|---|---|---|
| P-I | Demineralized Water | aqua | Q.S. |
| P-II | Plantasens Sunflower Butter | *Helianthus annuus* (sunflower) seed oil (and) hydrogenated vegetable oil | 7 |
| | Plantasens Natural Emulsifier HP10 | Sucrose polystearate cetearyl alcohol, olea europaea (olive) oil unsaponifiables | 3 |
| | Genadvance Hydra | Lauryl/myristyl polyricinoleate (and) glycerin | 1.5 |
| P-III | Aristoflex BLV | Ammonium acrylodimethyltaurate/ beheneth-25 methacrylate crosspolymer | 0.7 |
| | Aristoflex AVC | Ammonium acryloyldimethyltaurate/ VP copolymer | 0.3 |
| P-IV | Herbex Yeast Beta Glucan | Butylene glycol, yeast beta glucan | 2 |
| | Red Snow | Propanediol, water, *Camellia japonica* flower extract | 2 |
| | Plantasens Berto Orthomoist | *Orthosiphon stamineus* leaf extract, chlorphenesin (glycerin, aqua) | 1 |
| P-V | Nipaguard EHP | Ethylhexylglycerin, phenoxyethanol | 1 |
| | Fragrance (Fizzy Wave 939, IFF) | Parfum | 0.4 |
| P-VI | Sodium hydroxide solution (10%) | Aqua, sodium hydroxide | Q.S. |

The skin and hair nourishing creme may blur signs of aging and/or promote hydrating hair and/or may be used for hair defining.

Preparation:

The skin and hair nourishing creme was prepared as follows:
Phase P-I was heated to 75 °C. Phase P-II was filled in a separate beaker and also heated to 75 °C. Phase P-III was added to phase P-II and a dispersion was prepared. The dispersion was added to phase P-I. Further, phase P-IV was added to the dispersion of phases P-I, P-II and P-III. Further, phase P-V was added to the dispersion of phases P-I, P-II, P-III and P-IV. Further, the pH was adjusted to pH 4.5 to 5.5 with an aqueous 10 wt.% NaOH solution (phase P-VI).

Results:

A snow-white, gel-look thick cream was obtained. In a centrifuge (3000 rpm, 30 min), no phase separation occurred. Stability after storage at 50 °C for 15 days: no phase separation. Challenge Test: recommended preservation was checked by a challenge test according to ISO 11930 method and passed with criteria "A".

**Example 4** - **Combability**

The following compositions were prepared.

**Table 5.** Compositions having a heavy texture Formula

| Product | INCI name | Formulation No. Content (wt.-%) | |
|---|---|---|---|
| | | F14 | F15 |
| Aristoflex AVC | Ammonium acryloyldimethyltaurate/VP copolymer | 0.3 | 0.3 |
| Aristoflex BLV | Ammonium acryloyldimethyltaurate/ beheneth- 25 methacrylate crosspolymer | 0.7 | 0.7 |
| Plantasens sunflower butter | *Helianthus annuus* (Sunflower) seed oil | 7 | 7 |
| Genadvance Hydra | Lauryl/myristyl polyricinoleate (and) glycerin | 1.5 | / (none) |
| Water | Demineralized water | Q.S. | Q.S. |
| Herbex yeast beta glucan | Butylene glycol, water, yeast beta-glucan | 2 | 2 |
| Plantasens Berto Orthomoist | Glycerin (and) water (and) orthosiphon stamineus leaf extract (and) chlorphenesin | 1 | 1 |
| Nipaguard EHP | Ethylhexylglycerin, phenoxyethanol | 0.8 | 0.8 |
| Plantasens Natural Emulsifier HP10 | Sucrose polystearate, cetearyl alcohol, olea Europaea (Olive) oil unsaponifiables | 3 | 3 |
| NaOH 25% (w/v) pH: 5.0-5.5 | Sodium hydroxide | Q.S. | Q.S. |

**Table 6**. Composition having a light texture Formula

| Product | INCI name | F16, Content (wt.-%) |
|---|---|---|
| Aristoflex Silk | Sodium polyacryloyldimethyl taurate | 0.7 |
| Plantasens Flash 100 | Tridecane (and) pentadecane | 5 |
| Genadvance Hydra | Lauryl/myristyl polyricinoleate (and) glycerin | 1.5 |
| Water | Demineralized water | Q.S. |
| Herbex yeast beta glucan | Butylene glycol, water, yeast beta-glucan | 2 |
| Plantasens Berto Orthomoist | Glycerin (and) water (and) orthosiphon stamineus leaf extract (and) chlorphenesin | 1 |
| Nipaguard EHP | Ethylhexylglycerin, phenoxyethanol | 0.8 |
| (Plantasens) Crambisol | Crambe abyssinica seed oil (and) phytosterols (and) oleyl alcohol | 2 |
| NaOH 25% (w/v) | Sodium hydroxide | Q.S. |
| pH: 5.0-5.5 | | |

The compositions were prepared by homogenization of the components similar to the method described above.

Wet combing force measurement

The wet combing force was measured using a Dia-Stron MTT175 instrument. Caucasian hair tresses were washed, and excess water was removed by running the hair tresses gently between two fingers from top to bottom. The compositions according to Formulations F14 to F16 were applied to the hair tresses (distributed homogeneously and left on for 30 seconds). The hair tresses were rinsed with water. Excess water was removed by running the hair tresses gently between two fingers from top to bottom. The hair tresses were pre-combed three times before the combing force measurement. The hair tresses were added to the equipment and the wet combing force was measured.

**Table 7.** Energy to wet comb the tresses (in millijoules, mJ)

| | F14 | F15 | F16 |
|---|---|---|---|
| Energy to comb (combing force) | 12.9 ± 0.5 | 16.0 ± 1.8 | 11.3 |

The compositions are useful for detangling and conditioning hair as well as for improving and facilitating combing.

## Claims

1. A cosmetic composition comprising:

(A) at least one polymer which comprises at least 5 mol-% of repeating units (a) according to Formula (1):

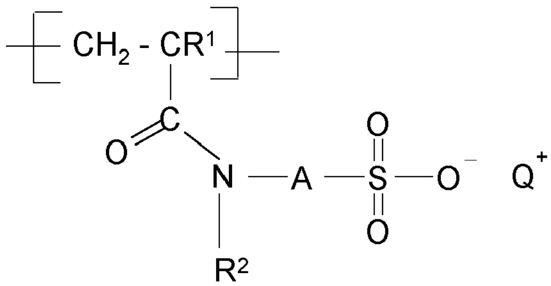

(1)

wherein:
$R^1$ and $R^2$ are independently selected from H, methyl or ethyl; A is a linear or branched $C_1$-$C_{12}$-alkylene group; and $Q^+$ is a cosmetically acceptable cation as component A;
(B) at least one emollient as component B;
(C) at least one ingredient selected from the group consisting of at least one conditioning agent, at least one solubilizing agent, at least one emulsifier, and a combination of two or more thereof as component C; and
(D) water.

2. The cosmetic composition of claim 1, wherein the polymer comprises or consists of:

(a) from 5 to 99.99 mol-% repeating units (a) according to Formula (1);
(b) from 0.01 to 10 mol-% crosslinking or branching units (b), wherein the crosslinking or branching units (b) result from the incorporation of a monomer comprising at least two olefinically unsaturated double bonds;
(c) optionally from 0 to 89 mol-% of neutral structural units (c);
(d) optionally from 0 to 20 mol-% of anionic structural units (d), wherein the anionic structural units (d) result from the incorporation of a monomer comprising at least one carboxylate anion.

3. The cosmetic composition according to any of claims 1 or 2, wherein the polymer comprises or consists of repeating units (a) resulting from the incorporation of a monomer selected from the group consisting of acryloyldimethyltaurates, acryloyl-1,1-dimethyl-2-methyltaurates (ACDMT), acryloyltaurates, acryloyl-N-methyltaurates, and combinations thereof, in particular wherein the polymer comprises or consists of repeating units (a) resulting from the incorporation of acryloyldimethyltaurate.

**4.** The cosmetic composition according to any of claims 2 or 3, wherein the crosslinking or branching units (b) are according to Formula (4)

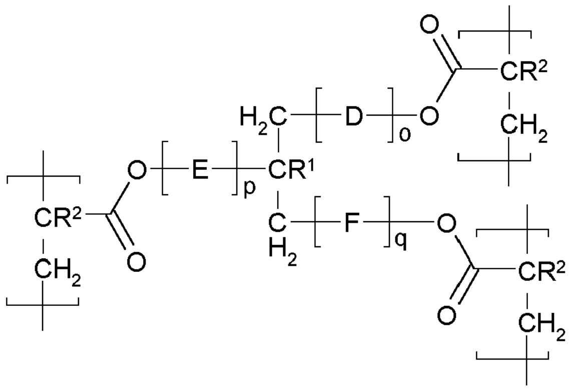 (4)

wherein

$R^1$ is selected from H, methyl or ethyl; and
$R^2$ is a linear or branched alkyl group having 1 to 6 carbon atoms, hydrogen, or a linear or branched, mono- or polyunsaturated alkylene group having 2 to 6 carbon atoms;
D, E, and F are independently methyleneoxy ($-CH_2O-$), ethyleneoxy ($-CH_2-CH_2-O-$), propyleneoxy ($-CH(CH_3)-CH_2-O-$), a linear or branched alkylene group having 1 to 6 carbon atoms, a linear or branched, singularly or multiply unsaturated alkenylene group having 2 to 6 carbon atoms, a linear mono-hydroxyalkylene group having 2 to 6 carbon atoms or a linear or branched dihydroxyalkylene group having 3 to 6 carbon atoms; and
o, p, and q each independently are an integer from 1 to 50, in particular wherein the crosslinking or branching units (b) result from the incorporation of a crosslinker selected from the group consisting of trimethylolpropane triacrylate (TMPTA) and/or glycerol propoxylate triacrylate (GPTA).

**5.** The cosmetic composition according to any of claims 1 to 4, wherein the at least one emollient is selected from the group consisting of:

(B-i) an emollient of synthetic origin selected from the group consisting of petrolatum, other hydrocarbon oils, esters of fatty acids with alcohols, esters of fatty alcohols with alkanoic acids, alkyl benzoates, silicone oils, other mineral oils, and combinations thereof;
(B-ii) an emollient of plant origin selected from the group consisting of plant oils, plant butters, plant waxes, and combinations thereof, which is optionally hydrogenated;
(B-iii) an emollient of animal origin selected from the group consisting of lanolin, perhydrosqualene, beeswax, beef tallow, other animal oils, animal butters, animal waxes, and combinations thereof, which is optionally hydrogenated; and

combinations of two or more thereof.

**6.** The cosmetic composition according to any of claims 1 to 5, wherein the at least one ingredient of component C comprises at least one conditioning agent selected from the group consisting of estolide esters, esters of oxalkylated alkylalkylene, oligoester ammonium salts, and combinations of two or more thereof.

**7.** The cosmetic composition according to any of claims 1 to 6, wherein a conditioning agent of component C comprises or consists of a compound of Formula (5):

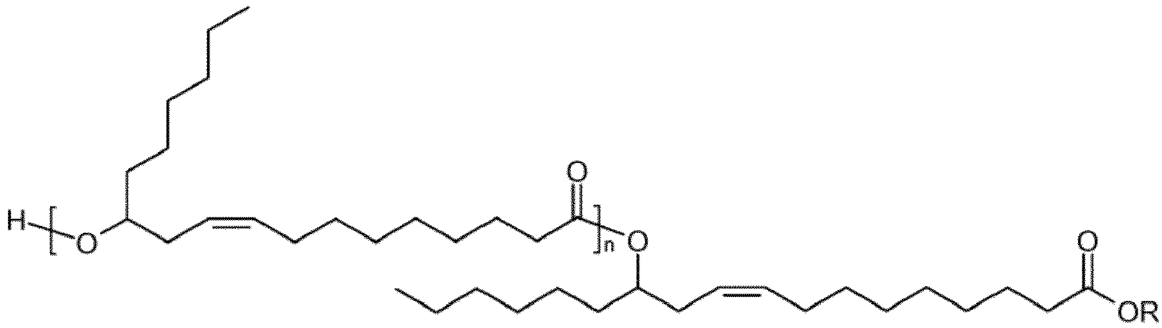

Formula (5)

wherein

R is selected from branched or linear $C_3$-$C_{20}$-alkyl, preferably from branched or linear $C_6$-$C_{18}$-alkyl, more preferably from branched or linear $C_8$-$C_{16}$-alkyl, even more preferably from branched or linear $C_{10}$-$C_{16}$-alkyl, in particular from branched or linear $C_{12}$-$C_{14}$-alkyl; and
n is an integer in the range from 1 to 20, preferably from 1 to 15, more preferably from 1 to 10, even more preferably from 2 to 8, in particular from 3 to 7.

**8.** The cosmetic composition according to any of claims 1 to 7, wherein a conditioning agent of component C comprises or consists of polyricinoleate, preferably lauryl/myristyl polyricinoleate.

**9.** The cosmetic composition according to any of claims 1 to 8, wherein a conditioning agent of component C comprises or consists of a compound of Formula (6) or a cosmetically acceptable optionally quaternized salt thereof:

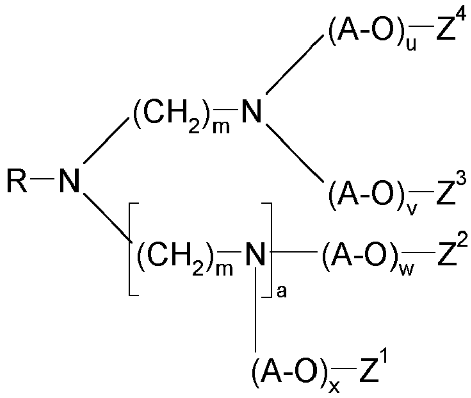

Formula (6)

wherein

R is $C_8$-$C_{24}$-alkyl or $C_8$-$C_{24}$-alkenyl, in particular $C_{10}$-$C_{20}$-alkyl or $C_{10}$-$C_{20}$-alkenyl;
A is each independently a group -$C_2H_4$- or -$C_3H_6$-, in particular a group -C2H4-;
$Z^1$ is a group -C(O)-R', wherein R' is $C_5$-$C_{35}$-alkyl or $C_5$-$C_{35}$-alkenyl, in particular $C_8$-$C_{24}$-alkyl or $C_3$-$C_{24}$-alkenyl;
$Z^2$ is a group -C(O)-R", wherein R" is $C_5$-$C_{35}$-alkyl or C5-$C_{35}$-alkenyl, in particular $C_8$-$C_{24}$-alkyl or $C_3$-$C_{24}$-alkenyl;
$Z^3$ is a group -C(O)-R"', wherein R' is $C_5$-$C_{35}$-alkyl or $C_5$-$C_{35}$-alkenyl, in particular $C_8$-$C_{24}$-alkyl or $C_3$-$C_{24}$-alkenyl;
$Z^4$ is a group -C(O)-R"", wherein R"" is $C_5$-$C_{35}$-alkyl or $C_5$-$C_{35}$-alkenyl, in particular $C_8$-$C_{24}$-alkyl or $C_3$-$C_{24}$-alkenyl;
a is 0 or 1, in particular 0;
m is 2 or 3, in particular 3;
u, v, w and x are each independently numbers from 1 to 9, in particular 2 to 9.

**10.** The cosmetic composition according to any of claims 1 to 9, wherein a conditioning agent of component C comprises or consists of an oligoester ammonium salt obtainable by the following steps:

(i) heating a mixture of the following components of Formulae (I), (II), (III) and (IV) under continuous removal of reaction water:

0.5 to 3.0 molar equivalents, preferably 0.75 to 3.0 molar equivalents, of a diethanolamine compound represented by the Formula (I)

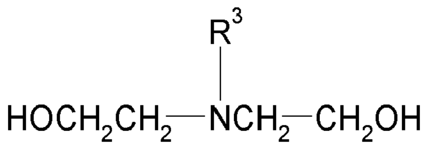

(I)

wherein $R^3$ is linear or branched $C_1$-$C_6$-alkyl, preferably linear or branched $C_1$-$C_4$-alkyl, more preferably methyl or ethyl;
0.5 to 1.5 molar equivalents of a dicarboxylic acid of Formula (II)

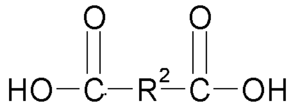

(II)

wherein $R^2$ is linear or branched $C_1$-$C_{10}$-alkylene or linear or branched $C_2$-$C_{10}$-alkenylene, preferably linear or branched $C_2$-$C_8$-alkylene, more preferably linear or branched $C_4$-alkylene;
0.5 to 1.5 molar equivalents of an organic triol (III) represented by the Formula (III-1) or (III-2)

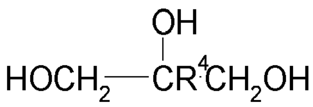

(III-1)

or

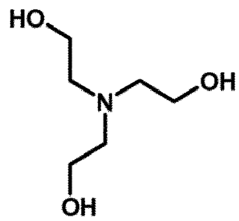

(III-2)

wherein $R^4$ is hydrogen or linear or branched $C_1$-$C_4$-alkyl or hydroxyl-Ci-$C_4$-alkyl, preferably hydrogen, methyl or ethyl, more preferably hydrogen;
1.0 molar equivalent of a monocarboxylic acid represented by Formula (IV)

$R^1$-COOH (IV)

wherein $R^1$ is linear or branched $C_{11}$-$C_{25}$-alkyl or linear or branched $C_{11}$-$C_{25}$-alkenyl, preferably linear or branched $C_{11}$-$C_{23}$-alkyl or linear or branched $C_{11}$-$C_{23}$-alkenyl, more preferably linear or branched $C_{19}$-$C_{23}$-alkyl;

(ii) reacting the oligoester product of step (i) with a quaternization agent (V), in particular dimethyl sulfate, diethyl sulfate or alkyl halides; and
(iii) optionally purifying the oligoester ammonium salt.

**11.** The cosmetic composition according to any of claims 1 to 10, wherein the solubilizing agent and/or emulsifier is selected from N-methyl-N-acylglucamines, anhydro methyl glucamides, glyceryl fatty acid esters, polyglyceryl fatty acid esters, polyglyceryl polyesters, sorbitan esters, alkyl glycosides, alkyl polyglycosides, stearoyl lactylate, fatty acid sugar esters, sugar polyesters, fatty alcohols, fatty alcohol ethoxylates, fatty acid ethoxylates, and combinations thereof.

**12.** The cosmetic composition according to any of claims 1 to 11, wherein said cosmetic composition further comprises at least one further cosmetically acceptable component or mixture of cosmetically acceptable components, wherein the cosmetically acceptable component or mixture of cosmetically acceptable components is selected from the group consisting of further emollients, waxes, emulsifiers, co-emulsifiers, solubilizers, surfactants, cationic polymers, film formers, superfatting agents, refatting agents, foam stabilizers, stabilizers, active biogenic substances, preservatives, preservation boosting ingredients, anti-fungal substance, anti-dandruff agents, dyes or pigments, particulate substances, opacifiers, abrasives, absorbents, anticaking agents, bulking agents, pearlizing agents, direct dyes, perfumes or fragrances, carriers, solvents or diluents, propellants, functional acids, active ingredients, skin-brightening agents, self-tanning agents, exfoliants, enzymes, anti-acne agents, deodorants and anti-perspirants, viscosity modifiers, thickening and gelling agents, pH adjusting agents, buffering agents, anti-oxidants, chelants, astringents, sunscreens, sun protection agents, UV filters, conditioning agents, humectants, occlusive agents, pediculocides, anti-foaming agents, flavouring agents, electrolytes, oxidizing agents and reducing agents.

**13.** The cosmetic composition according to any of claims 1 to 12, wherein said cosmetic composition comprises or consists of:

(A) at least 0.1 wt.-% of the at least one polymer which comprises at least 5 mol-% of repeating units (a) according to Formula (1) (component A);
(B) at least 0.5 wt.-% of the at least one emollient (component B);
(C) at least 0.4 wt.-% of the at least one ingredient of component C; and
(D) 10 to 99 wt.-% of water (component D); and
(E) optionally at least one further cosmetically acceptable component E.

**14.** The cosmetic composition of any of claims 1 to 13, wherein said cosmetic composition is selected from the group consisting of shampoo, hair and/or skin conditioner, body wash, facial cleanser, face mask, bubble bath, intimate wash, bath oil, cleansing milk, micellar water, make-up remover, cleansing wipes, hair mask, perfume, liquid soap, shaving soap, shaving foam, cleansing foam, day cream, anti-ageing cream, body milk, body lotion, body mousse, face serum, eye cream, sunscreen lotion, sun cream, face cream, after-shave lotion, pre-shaving cream, depilatory cream, skin-whitening gel, self-tanning cream, anti-acne gel, mascara, foundation, primer, concealer, blush, bronzer, blemish balm (bb) cream, eyeliner, night cream, eye brow gel, highlighter, lip stain, hand sanitizer, hair oil, nail varnish remover, hair styling gel, hair styling cream, anti-frizz serum, scalp treatment, hair colorant, split end fluid, deodorant, antiperspirant, baby cream, insect repellent, hand cream, sunscreen gel, foot cream, exfoliator, body scrub, cellulite treatment, bar soap, cuticle cream, lip balm, hair treatment, eye shadow, bath additive, body mist, eau de toilette, lubricating gel, moisturizer, serum, toner, aqua sorbet, cream gel, styling mousse, dry shampoo, lip stick, lip gloss, body oil, shower milk, illuminator, lip crayon, hair spray, combing cream, sunblock, ointment, and lipstick.

**15.** Use of a cosmetic composition of any of claims 1 to 14 for hair and/or skin care.

Europäisches Patentamt
European Patent Office
Office européen des brevets

# EUROPEAN SEARCH REPORT

Application Number
EP 20 21 2308

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 109 394 830 A (BEIJING MINGHONG SCIENCE AND TRADE CO LTD) 1 March 2019 (2019-03-01) * claims; tables * | 1-3,5,6, 11-13 | INV. A61Q5/12 A61Q19/08 A61Q19/00 A61K8/81 A61K8/37 A61K8/41 A61K8/45 A61K8/92 A61K8/31 A61K8/34 |
| X<br>Y | WO 2015/151040 A1 (UNIFARCO S P A [IT]) 8 October 2015 (2015-10-08) * examples 2-3 * | 1-6, 11-15<br>1-15 | |
| X<br>Y | DE 10 2010 011245 A1 (BEIERSDORF AG [DE]) 8 September 2011 (2011-09-08) * examples comp 1,3 * * examples 1-5 * | 1-3,5,6, 11-15<br>1-15 | |
| X<br>Y | WO 2018/007476 A1 (OLEON NV [BE]; OLEON SDN BHD [MY]) 11 January 2018 (2018-01-11) * example 2 * | 1-6, 11-15<br>1-15 | |
| X<br>Y | DE 10 2011 013342 A1 (CLARIANT FINANCE BVI LTD) 15 September 2011 (2011-09-15) * paragraph [0144] * * claims; examples 17-35 * | 1-6, 11-15<br>1-15 | TECHNICAL FIELDS SEARCHED (IPC) |
| X<br>Y | WO 2018/108611 A (CLARIANT INTERNATIONAL LTD) 21 June 2018 (2018-06-21) * claims 1-10; examples 1-2, 6-19, 21-28, 30-31, 34-35, 37-40 * | 1-6, 11-15<br>1-15 | A61Q A61K |
| | -/-- | | |

The present search report has been drawn up for all claims

| Place of search | Date of completion of the search | Examiner |
|---|---|---|
| Munich | 9 June 2021 | Krattinger, B |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

4

EPO FORM 1503 03.82 (P04C01)

Europäisches Patentamt
European Patent Office
Office européen des brevets

# EUROPEAN SEARCH REPORT

Application Number
EP 20 21 2308

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Genadvance ET AL: "New customized solutions for hair conditioning", , 1 January 2019 (2019-01-01), XP055808606, Retrieved from the Internet: URL:file:///C:/Users/BK22882/Downloads/Clariant%20Brochure%20Genadvance%20FormulationBooklet%202019%20EN%20(4).pdf [retrieved on 2021-05-28] | 1-8, 11-15 | |
| Y | * the whole document * * page 7 * | 7-10 | |
| Y | WO 2013/009471 A1 (LUBRIGREEN BIOSYNTHETICS LLC [US]; BREDSGUARD JAKOB [US] ET AL.) 17 January 2013 (2013-01-17) * claims; examples * | 6-8 | |
| Y,D | WO 2016/174256 A1 (CLARIANT INT LTD [CH]) 3 November 2016 (2016-11-03) * claims; tables * | 6-8 | TECHNICAL FIELDS SEARCHED (IPC) |
| Y | US 2018/369099 A1 (KITA-TOKARCZYK KATARZYNA [DE] ET AL) 27 December 2018 (2018-12-27) * claims; examples * | 10 | |
| Y | WO 2019/175124 A1 (CLARIANT INT LTD [CH]) 19 September 2019 (2019-09-19) * claims; tables * | 9 | |
| Y | WO 2015/110269 A1 (CLARIANT INT LTD [CH]) 30 July 2015 (2015-07-30) * claims; tables * | 9 | |

The present search report has been drawn up for all claims

| Place of search | Date of completion of the search | Examiner |
|---|---|---|
| Munich | 9 June 2021 | Krattinger, B |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

4

EPO FORM 1503 03.82 (P04C01)

# ANNEX TO THE EUROPEAN SEARCH REPORT ON EUROPEAN PATENT APPLICATION NO. EP 20 21 2308

This annex lists the patent family members relating to the patent documents cited in the above-mentioned European search report. The members are as contained in the European Patent Office EDP file on
The European Patent Office is in no way liable for these particulars which are merely given for the purpose of information.

09-06-2021

| Patent document cited in search report | Publication date | Patent family member(s) | Publication date |
|---|---|---|---|
| CN 109394830 A | 01-03-2019 | NONE | |
| WO 2015151040 A1 | 08-10-2015 | DK 3125865 T3 | 08-07-2019 |
| | | EP 3125865 A1 | 08-02-2017 |
| | | ES 2731250 T3 | 14-11-2019 |
| | | HR P20191059 T1 | 06-09-2019 |
| | | HU E044885 T2 | 28-11-2019 |
| | | PL 3125865 T3 | 31-10-2019 |
| | | PT 3125865 T | 04-07-2019 |
| | | SI 3125865 T1 | 30-08-2019 |
| | | WO 2015151040 A1 | 08-10-2015 |
| DE 102010011245 A1 | 08-09-2011 | NONE | |
| WO 2018007476 A1 | 11-01-2018 | FR 3053587 A1 | 12-01-2018 |
| | | JP 2019520383 A | 18-07-2019 |
| | | KR 20190026807 A | 13-03-2019 |
| | | WO 2018007476 A1 | 11-01-2018 |
| DE 102011013342 A1 | 15-09-2011 | CN 103492438 A | 01-01-2014 |
| | | DE 102011013342 A1 | 15-09-2011 |
| | | EP 2683752 A1 | 15-01-2014 |
| | | JP 5976688 B2 | 24-08-2016 |
| | | JP 2014508835 A | 10-04-2014 |
| | | US 2014127147 A1 | 08-05-2014 |
| | | WO 2012119747 A1 | 13-09-2012 |
| WO 2018108611 A | 21-06-2018 | | |
| WO 2013009471 A1 | 17-01-2013 | EP 2701675 A1 | 05-03-2014 |
| | | US 2013065970 A1 | 14-03-2013 |
| | | US 2015045430 A1 | 12-02-2015 |
| | | WO 2013009471 A1 | 17-01-2013 |
| WO 2016174256 A1 | 03-11-2016 | BR 112017023144 A2 | 10-07-2018 |
| | | CN 107683163 A | 09-02-2018 |
| | | DE 212016000006 U1 | 08-12-2016 |
| | | EP 3288641 A1 | 07-03-2018 |
| | | EP 3406300 A1 | 28-11-2018 |
| | | JP 6688316 B2 | 28-04-2020 |
| | | JP 2018514533 A | 07-06-2018 |
| | | US 2018125767 A1 | 10-05-2018 |
| | | WO 2016174256 A1 | 03-11-2016 |
| US 2018369099 A1 | 27-12-2018 | BR 112018010272 A2 | 27-11-2018 |
| | | BR 112018010358 A2 | 04-12-2018 |
| | | BR 112018010433 A2 | 21-11-2018 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO.

EP 20 21 2308

This annex lists the patent family members relating to the patent documents cited in the above-mentioned European search report. The members are as contained in the European Patent Office EDP file on
The European Patent Office is in no way liable for these particulars which are merely given for the purpose of information.

09-06-2021

| Patent document cited in search report | Publication date | Patent family member(s) | Publication date |
|---|---|---|---|
| | | CN 108391417 A | 10-08-2018 |
| | | CN 108391418 A | 10-08-2018 |
| | | CN 108391419 A | 10-08-2018 |
| | | EP 3386472 A1 | 17-10-2018 |
| | | EP 3386473 A1 | 17-10-2018 |
| | | EP 3386474 A1 | 17-10-2018 |
| | | JP 2018536672 A | 13-12-2018 |
| | | JP 2018536674 A | 13-12-2018 |
| | | JP 2018536675 A | 13-12-2018 |
| | | US 2018353410 A1 | 13-12-2018 |
| | | US 2018360714 A1 | 20-12-2018 |
| | | US 2018369099 A1 | 27-12-2018 |
| | | WO 2017097816 A1 | 15-06-2017 |
| | | WO 2017097817 A1 | 15-06-2017 |
| | | WO 2017097819 A1 | 15-06-2017 |
| WO 2019175124 A1 | 19-09-2019 | BR 112020015014 A2 | 19-01-2021 |
| | | EP 3764978 A1 | 20-01-2021 |
| | | US 2020405607 A1 | 31-12-2020 |
| | | WO 2019175124 A1 | 19-09-2019 |
| WO 2015110269 A1 | 30-07-2015 | BR 112016011249 B1 | 19-01-2021 |
| | | CN 106132486 A | 16-11-2016 |
| | | EP 3065832 A1 | 14-09-2016 |
| | | ES 2690981 T3 | 23-11-2018 |
| | | JP 6537516 B2 | 03-07-2019 |
| | | JP 2017503816 A | 02-02-2017 |
| | | US 2016317410 A1 | 03-11-2016 |
| | | WO 2015110269 A1 | 30-07-2015 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2016174256 A
- WO 2015110269 A
- WO 2019175124 A
- WO 2017097816 A
- WO 2017097817 A
- WO 2017097819 A
- EP 16176830
- US 2809971 A
- US 3236733 A
- US 3753196 A
- US 3761418 A
- US 4345080 A
- US 4323683 A
- US 4379753 A
- US 4470982 A
- WO 2013017262 A1
- EP 1084696 A
- US 5104646 A

### Non-patent literature cited in the description

- **GEORG ODIAN.** Principles of Polymerization. Wiley-Interscience, 19-24
- **BERND TIEKE.** Makromolekulare Chemie: Eine Einführung. Wiley-VCH, 2010, 259-261
- **MASSAO KUNIOKA.** *Radioisotopes,* 2013, vol. 62, 901-925
- International Cosmetic Ingredient Dictionary and Handbook
- International Cosmetic Ingredient Dictionary. 1993
- CTFA Cosmetic Ingredient Handbook. 1992